# EUROPÄISCHE PATENTSCHRIFT

(11) **EP 2 534 147 B1**
(45) Veröffentlichungstag und Bekanntmachung des Hinweises auf die Patenterteilung: **17.06.2015**
(21) Anmeldenummer: 11704433.9
(22) Anmeldetag: 08.02.2011
(51) Int. Cl.: C07D 471/10, C07D 211/94, A01N 43/90

(54) **SPIROHETEROCYCLISCH-SUBSTITUIERTE TETRAMSÄURE-DERIVATE**
SPIROHETEROCYCLIC-SUBSTITUTED TETRAMIC ACID DERIVATIVES
DÉRIVÉS D'ACIDES TÉTRAMIQUES SUBSTITUÉS DE MANIÈRE SPIROHÉTÉROCYCLIQUE

(30) Priorität: 10.02.2010 US 303069 P; 10.02.2010 EP 10153199
(43) Veröffentlichungstag der Anmeldung: 19.12.2012
(73) Patentinhaber: Bayer Intellectual Property GmbH, 40789 Monheim (DE)
(72) Erfinder: FISCHER, Reiner, 40789 Monheim (DE); VOERSTE, Arnd, 50674 Köln (DE); HÄUSER-HAHN, Isolde, 51375 Leverkusen (DE); LEHR, Stefan, 65835 Liederbach (DE); GATZWEILER, Elmar, 61213 Bad Nauheim (DE); GÖRGENS, Ulrich, 40882 Ratingen (DE); HEINEMANN, Ines, 65719 Hofheim (DE)
(86) Internationale Anmeldenummer: PCT/EP2011/051800
(87) Internationale Veröffentlichungsnummer: WO 2011/098443

(56) Entgegenhaltungen:
- WO-A1-2009/049851
- WO-A1-2010/115780
- WO-A2-2010/052161

## Beschreibung

Die vorliegende Erfindung betrifft neue spiroheterocyclisch-substituierte Tetramsäure-Derivate, mehrere Verfahren zu ihrer Herstellung und ihre Verwendung als Schädlingsbekämpfungsmittel und/oder Herbizide und/oder Fungizide. Auch Gegenstand der Erfindung sind selektiv herbizide Mittel, die spiroheterocyclisch-substituierte Tetramsäure-Derivate einerseits und eine die Kulturpflanzenverträglichkeit verbessernde Verbindung andererseits enthalten.

Die vorliegende Erfindung betrifft weiterhin die Steigerung der Wirkung von Pflanzenschutzmitteln enthaltend insbesondere spiroheterocyclisch-substituierte Tetramsäure-Derivate, durch die Zugabe von Ammonium- oder Phosphoniumsalzen und gegebenenfalls Penetrationsförderern, die entsprechenden Mittel, Verfahren zur ihrer Herstellung und ihre Anwendung im Pflanzenschutz als Insektizide und/oder Akarizide und/oder Fungizide und/oder zur Verhinderung von unerwünschtem Pflanzenwuchs.

Von 3-Acyl-pyrrolidin-2,4-dionen sind pharmazeutische Eigenschaften vorbeschrieben (S. Suzuki et al. Chem. Pharm. Bull. 15 1120 (1967)). Weiterhin wurden N-Phenylpyrrolidin-2,4-dione von R. Schmierer und H. Mildenberger (Liebigs Ann. Chem. 1985, 1095) synthetisiert. Eine biologische Wirksamkeit dieser Verbindungen wurde nicht beschrieben.

In EP-A-0 262 399 und GB-A-2 266 888 werden ähnlich strukturierte Verbindungen (3-Aryl-pyrrolidin-2,4-dione) offenbart, von denen jedoch keine herbizide, insektizide oder akarizide Wirkung bekannt geworden ist. Bekannt mit herbizider, insektizider oder akarizider Wirkung sind unsubstituierte, bicyclische 3-Aryl-pyrrolidin-2,4-dion-Derivate (EP-A-355 599, EP-A-415 211 und JP-A-12-053 670) sowie substituierte monocyclische 3-Aryl-pyrrolidin-2,4-dion-Derivate (EP-A-377 893, EP-A-442 077 und WO 10/066 780).

Weiterhin bekannt sind polycyclische 3-Arylpyrrolidin-2,4-dion-Derivate (EP-A-442 073) sowie 1H-Arylpyrrolidin-dion-Derivate (EP-A-456 063, EP-A-521 334, EP-A-596 298, EP-A-613 884, EP-A-613 885, WO 95/01 971, WO 95/26 954, WO 95/20 572, EP-A-0 668 267, WO 96/25 395, WO 96/35 664, WO 97/01 535, WO 97/02 243, WO 97/36 868, WO 97/43275, WO 98/05638, WO 98/06721, WO 98/25928, WO 99/24437, WO 99/43649, WO 99/48869 und WO 99/55673, WO 01/17972, WO 01/23354, WO 01/74770, WO 03/013249, WO 03/062244, WO 2004/007448, WO 2004/024 688, WO 04/065366, WO 04/080962, WO 04/111042, WO 05/044791, WO 05/044796, WO 05/048710, WO 05/049569, WO 05/066125, WO 05/092897, WO 06/000355, WO 06/029799, WO 06/056281, WO 06/056282, WO 06/089633, WO 07/048545, DEA 102 00505 9892, WO 07/073856, WO 07/096058, WO 07/121868, WO 07/140881, WO 08/067873, WO 08/067910, WO 08/067911, WO 08/138551, WO 09/015801, WO 09/039975, WO 09/049851, WO 09/115262, WO 10/052161, WO 10/063670, WO 10/063378, WO 10/102758). Außerdem sind ketalsubstituierte 1-H-Arylpyrrolidin-2,4-dione aus WO 99/16748 und (spiro)-ketalsubstituierte N-Alkoxy-alkoxy-substituierte Aryl-pyrrolidindione aus JP-A-14 205 984 und Ito M. et al.. Bioscience, Biotechnology and Biochemistry 67, 1230-1238, (2003) bekannt. Der Zusatz von Safenern zu Ketoenolen ist ebenfalls prinzipiell aus der WO 03/013249 bekannt. Außerdem sind aus WO 06/024411 herbizide Mittel enthaltend Ketoenole bekannt.

Die herbizide und/oder fungizide und/oder akarizide und/oder insektizide Wirksamkeit und/oder Wirkungsbreite und/oder die Pflanzenverträglichkeit der bekannten Verbindungen, insbesondere gegenüber Kulturpflanzen, ist jedoch nicht immer ausreichend.

Es wurden nun neue Verbindungen der Formel (I-a) 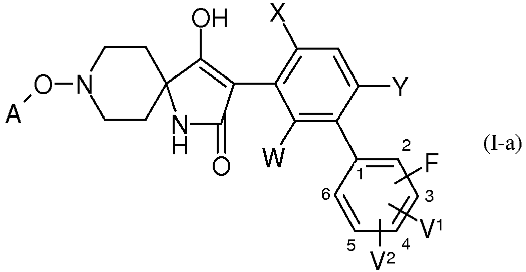 gefunden,
in welcher
A, W, X, Y, V¹ und V² die folgenden Bedeutungen haben

**Tabelle 1**

| **A** | **W** | **X** | **Y** | **F** | **V¹** | **V²** |
|---|---|---|---|---|---|---|
| CH₃ | CH₃ | CH₃ | H | 2 | H | H |
| CH₃ | CH₃ | CH₃ | H | 3 | H | H |
| CH₃ | CH₃ | CH₃ | H | 4 | H | H |
| CH₃ | CH₃ | CH₃ | H | 2 | 4-F | H |
| CH₃ | CH₃ | CH₃ | H | 2 | 4-Cl | H |
| CH₃ | CH₃ | CH₃ | H | 2 | 4-CH₃ | H |
| CH₃ | CH₃ | CH₃ | H | 2 | 4-OCH₃ | H |
| CH₃ | CH₃ | CH₃ | H | 3 | 4-F | H |
| CH₃ | CH₃ | CH₃ | H | 3 | 4-Cl | H |
| CH₃ | CH₃ | CH₃ | H | 3 | 4-CH₃ | H |
| CH₃ | CH₃ | CH₃ | H | 3 | 4-OCH₃ | H |
| CH₃ | CH₃ | CH₃ | H | 4 | 3-Cl | H |
| CH₃ | CH₃ | CH₃ | H | 4 | 3-CH₃ | H |
| CH₃ | CH₃ | CH₃ | H | 4 | 3-OCH₃ | H |
| CH₃ | CH₃ | CH₃ | H | 2 | 4-F | 5-F |
| CH₃ | CH₃ | CH₃ | H | 2 | 4-F | 6-F |
| CH₃ | CH₃ | CH₃ | H | 2 | 4-Cl | 5-F |
| CH₃ | CH₃ | CH₃ | H | 2 | 5-Cl | 4-F |
| CH₃ | CH₃ | CH₃ | H | 3 | 4-F | 5-F |
| CH₃ | CH₃ | CH₃ | CH₃ | 2 | H | H |
| CH₃ | CH₃ | CH₃ | CH₃ | 3 | H | H |
| CH₃ | CH₃ | CH₃ | CH₃ | 4 | H | H |
| CH₃ | CH₃ | CH₃ | CH₃ | 2 | 4-F | H |
| CH₃ | CH₃ | CH₃ | CH₃ | 2 | 4-Cl | H |
| CH₃ | CH₃ | CH₃ | CH₃ | 2 | 4-CH₃ | H |
| CH₃ | CH₃ | CH₃ | CH₃ | 2 | 4-OCH₃ | H |
| CH₃ | CH₃ | CH₃ | CH₃ | 3 | 4-F | H |
| CH₃ | CH₃ | CH₃ | CH₃ | 3 | 4-Cl | H |
| CH₃ | CH₃ | CH₃ | CH₃ | 3 | 4-CH₃ | H |
| CH₃ | CH₃ | CH₃ | CH₃ | 3 | 4-OCH₃ | H |
| CH₃ | CH₃ | CH₃ | CH₃ | 4 | 3-Cl | H |
| CH₃ | CH₃ | CH₃ | CH₃ | 4 | 3-CH₃ | H |
| CH₃ | CH₃ | CH₃ | CH₃ | 4 | 3-OCH₃ | H |
| CH₃ | CH₃ | CH₃ | CH₃ | 2 | 4-F | 5-F |
| CH₃ | CH₃ | CH₃ | CH₃ | 2 | 4-F | 6-F |
| CH₃ | CH₃ | CH₃ | CH₃ | 2 | 4-Cl | 5-F |
| CH₃ | CH₃ | CH₃ | CH₃ | 2 | 5-Cl | 4-F |
| CH₃ | CH₃ | CH₃ | CH₃ | 3 | 4-F | 5-F |

Tabelle 2 W, X, Y, F, V¹ und V² wie in Tabelle 1 angegeben A = C₂H₅

**Tabelle 3 W, X, Y, F, V¹ und V² wie in Tabelle 1 angegeben A = C₃H₇**

| **W** | **X** | **Y** | **F** | **V¹** | **V²** | **A** |
|---|---|---|---|---|---|---|
| Cl | OCH₃ | H | 4 | H | H | C₂H₅ |
| CH₃ | C₂H₅ | H | 4 | H | H | CH₃ |
| CH₃ | C₂H₅ | H | 4 | H | H | C₂H₅ |
| Cl | OCH₃ | H | 4 | H | H | CH₃ |
| CH₃ | CH₃ | H | 4 | 3-F | H | C₂H₅ |
| CH₃ | CH₃ | H | 4 | 3-F | H | CH₃ |

Die Verbindungen der Formel (I-a) können, auch in Abhängigkeit von der Art der Substituenten, als optische Isomere oder Isomerengemische, in unterschiedlicher Zusammensetzung vorliegen, die gegebenenfalls in üblicher Art und Weise getrennt werden können. Sowohl die reinen Isomeren als auch die Isomerengemische, deren Herstellung und Verwendung sowie diese enthaltende Mittel sind Gegenstand der vorliegenden Erfindung. Im folgenden wird der Einfachheit halber jedoch stets von Verbindungen der Formel (I-a) gesprochen, obwohl sowohl die reinen Verbindungen als gegebenenfalls auch Gemische mit unterschiedlichen Anteilen an isomeren Verbindungen gemeint sind.

Weiterhin wurde gefunden, dass man die neuen Verbindungen der Formel (I-a) nach den im Folgenden beschriebenen Verfahren erhält:

### (A) Man erhält Verbindungen der Formel (I-a)

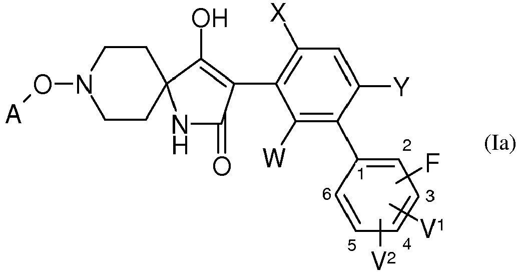

in welcher
A, W, X, Y, V¹ und V² die oben angegebenen Bedeutungen haben,
wenn man

### Verbindungen der Formel (II)

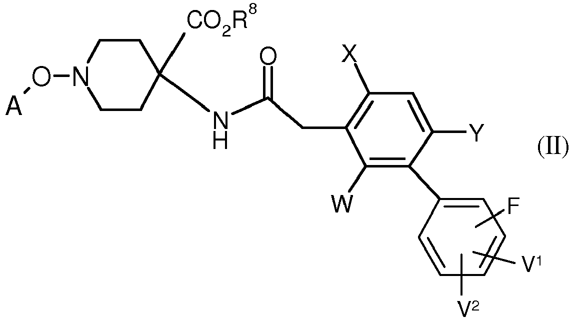

in welcher
A, W, X, Y, V¹ und V² die oben angegebenen Bedeutungen haben,
und
R⁸ für Alkyl (bevorzugt C₁-C₆-Alkyl) steht,
in Gegenwart eines Verdünnungsmittels und in Gegenwart einer Base intramolekular kondensiert.

Außerdem wurde gefunden
(I) dass man Verbindungen der oben gezeigten Formel (I-a), in welcher A, W, X, Y , V¹ und V² die oben angegebene Bedeutung haben, erhält, wenn man Verbindungen der Formel (I-a'), in welcher A, W, X und Y die oben genannte Bedeutung haben und Z' bevorzugt für Brom oder Iod steht 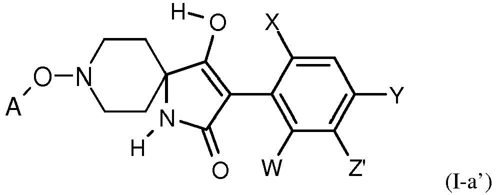 mit kupplungsfähigen (Het)-arylderivaten, z.B. Phenylboronsäuren der Formeln (XIII) 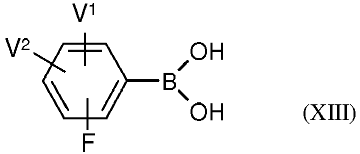 in welcher V¹ und V² die oben angegebenen Bedeutungen hat
   oder deren Ester in Gegenwart eines Lösungsmittels, in Gegenwart eines Katalysators (z. B. Pd-Komplexe) und in Gegenwart einer Base (z.B. Natriumcarbonat, Kaliumphosphat) kuppelt.

Weiterhin wurde gefunden, dass die neuen Verbindungen der Formel (I-a) eine sehr gute Wirksamkeit als Schädlingsbekämpfungsmittel, vorzugsweise als Insektizide, Akarizide und Herbizide aufweisen.

Überraschenderweise wurde nun auch gefunden, dass bestimmte substituierte, cyclische Ketoenole bei gemeinsamer Anwendung mit den im weiteren beschriebenen, die Kulturpflanzen-Verträglichkeit verbessernden Verbindungen (Safenem/Antidots) ausgesprochen gut die Schädigung der Kulturpflanzen verhindert und besonders vorteilhaft als breit wirksame Kombinationspräparate zur selektiven Bekämpfung von unerwünschten Pflanzen in Nutzpflanzenkulturen, wie z.B. in Getreide aber auch Mais, Soja und Reis, verwendet werden können.

Gegenstand der Erfindung sind auch selektiv-herbizide Mittel enthaltend einen wirksamen Gehalt an einer Wirkstoffkombination umfassend als Komponenten
a') mindestens eine Verbindung der Formel (I-a), in welcher A, W, X, Y , V¹ und V² die oben angegebene Bedeutung haben
   und
(b') zumindest eine die Kulturpflanzen-Verträglichkeit verbessernde Verbindung (Safener).
   Die Safener sind vorzugsweise ausgewählt aus der Gruppe bestehend aus:
   S1) Verbindungen der Formel (S1), 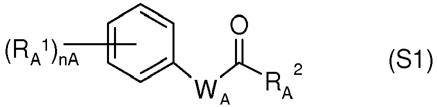 wobei die Symbole und Indizes folgende Bedeutungen haben:
      - n_{A}: ist eine natürliche Zahl von 0 bis 5, vorzugsweise 0 bis 3;
      - R_{A}¹: ist Halogen, (C₁-C₄)Alkyl, (C₁-C₄)Alkoxy, Nitro oder (C₁-C₄)Haloalkyl;
      - W_{A}: ist ein unsubstituierter oder substituierter divalenter heterocyclischer Rest aus der Gruppe der teilungesättigten oder aromatischen Fünfring-Heterocyclen mit 1 bis 3 Heteroringatomen aus der Gruppe N und O, wobei mindestens ein N-Atom und höchstens ein O-Atom im Ring enthalten ist, vorzugsweise ein Rest aus der Gruppe (W_{A}¹) bis (W_{A}⁴), 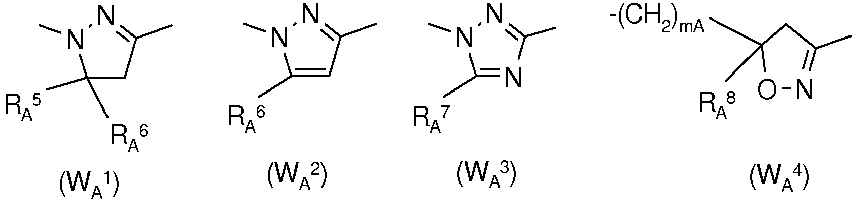
      - m_{A}: ist 0 oder 1;
      - R_{A}²: ist OR_{A}³, SR_{A}³ oder NR_{A}³R_{A}⁴ oder ein gesättigter oder ungesättigter 3- bis 7-gliedriger Heterocyclus mit mindestens einem N-Atom und bis zu 3 Heteroatomen, vorzugsweise aus der Gruppe O und S, der über das N-Atom mit der Carbonylgruppe in (S1) verbunden ist und unsubstituiert oder durch Reste aus der Gruppe (C₁-C₄)Alkyl, (C₁-C₄)Alkoxy oder gegebenenfalls substituiertes Phenyl substituiert ist, vorzugsweise ein Rest der Formel OR_{A}³, NHR_{A}⁴ oder N(CH₃)₂, insbesondere der Formel OR_{A}³;
      - R_{A}³: ist Wasserstoff oder ein unsubstituierter oder substituierter aliphatischer Kohlenwasserstoffrest, vorzugsweise mit insgesamt 1 bis 18 C-Atomen;
      - R_{A}⁴: ist Wasserstoff, (C₁-C₆)Alkyl, (C₁-C₆)Alkoxy oder substituiertes oder unsubstituiertes Phenyl;
      - R_{A}⁵: ist H, (C₁-C₈)Alkyl, (C₁-C₈)Haloalkyl, (C₁-C₄)Alkoxy(C₁-C₈)Alkyl, Cyano oder COOR_{A}⁹, worin R_{A}⁹ Wasserstoff, (C₁-C₈)Alkyl, (C₁-C₈)Haloalkyl, (C₁-C₄)Alkoxy-(C₁-C₄)alkyl, (C₁-C₆)Hydroxyalkyl, (C₃-C₁₂)Cycloalkyl oder Tri-(C₁-C₄)-alkyl-silyl ist;
      - R_{A}⁶, R_{A}⁷, R_{A}⁸: sind gleich oder verschieden Wasserstoff, (C₁-C₈)Alkyl, (C₁-C₈)Haloalkyl, (C₃-C₁₂)Cycloalkyl oder substituiertes oder unsubstituiertes Phenyl;
      vorzugsweise:
      a) Verbindungen vom Typ der Dichlorphenylpyrazolin-3-carbonsäure (S1^{a}), vorzugsweise Verbindungen wie 1-(2,4-Dichlorphenyl)-5-(ethoxycarbonyl)-5-methyl-2-pyrazolin-3-carbonsäure, 1-(2,4-Dichlorphenyl)-5-(ethoxycarbonyl)-5-methyl-2-pyrazolin-3-carbonsäureethylester (S1-1) ("Mefenpyr-diethyl"), und verwandte Verbindungen, wie sie in der WO-A-91/07874 beschrieben sind;
      b) Derivate der Dichlorphenylpyrazolcarbonsäure (S1^{b}), vorzugsweise Verbindungen wie 1-(2,4-Dichlorphenyl)-5-methyl-pyrazol-3-carbonsäureethylester (S1-2), 1-(2,4-Dichlorphenyl)-5-isopropyl-pyrazol-3-carbonsäureethylester (S1-3), 1-(2,4-Dichlorphenyl)-5-(1,1-dimethyl-ethyl)pyrazol-3-carbonsäureethyl-ester (S1-4) und verwandte Verbindungen, wie sie in EP-A-333 131 und EP-A-269 806 beschrieben sind;
      c) Derivate der 1,5-Diphenylpyrazol-3-carbonsäure (S1^{c}), vorzugsweise Verbindungen wie 1-(2,4-Dichlorphenyl)-5-phenylpyrazol-3-carbonsäureethylester (S1-5), 1-(2-Chlorphenyl)-5-phenylpyrazol-3-carbonsäuremethylester (S1-6) und verwandte Verbindungen wie sie beispielsweise in der EP-A-268554 beschrieben sind;
      d) Verbindungen vom Typ der Triazolcarbonsäuren (S1^{d}), vorzugsweise Verbindungen wie Fenchlorazol(-ethylester), d.h. 1-(2,4-Dichlorphenyl)-5-trichlormethyl-(1H)-1,2,4-triazol-3-carbonsäureethylester (S1-7), und verwandte Verbindungen wie sie in EP-A-174 562 und EP-A-346 620 beschrieben sind;
      e) Verbindungen vom Typ der 5-Benzyl- oder 5-Phenyl-2-isoxazolin-3- carbonsäure oder der 5,5-Diphenyl-2-isoxazolin-3-carbonsäure (S1^{e}), vorzugsweise Verbindungen wie 5-(2,4-Dichlorbenzyl)-2-isoxazolin-3-carbonsäureethylester (S1-8) oder 5-Phenyl-2-isoxazolin-3-carbonsäureethylester (S1-9) und verwandte Verbindungen, wie sie in WO-A-91/08202 beschrieben sind, bzw. 5,5-Diphenyl-2-isoxazolin-carbonsäure (S1-10) oder 5,5-Diphenyl-2-isoxazolin-carbonsäureethylester (S1-11) ("Isoxadifen-ethyl") oder -n-propylester (S1-12) oder der 5-(4-Fluorphenyl)-5-phenyl-2-isoxazolin-3-carbonsäureethylester (S1-13), wie sie in der Patentanmeldung WO-A-95/07897 beschrieben sind.
   S2) Chinolinderivate der Formel (S2), 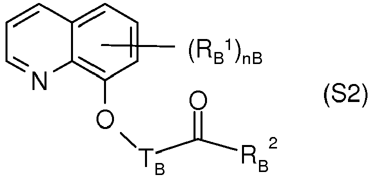 wobei die Symbole und Indizes folgende Bedeutungen haben:
      - **R**_{B}¹: ist Halogen, (C₁-C₄)Alkyl, (C₁-C₄)Alkoxy, Nitro oder (C₁-C₄)Haloalkyl;
      - **n**_{B}: ist eine natürliche Zahl von 0 bis 5, vorzugsweise 0 bis 3;
      - **R**_{B}²: ist OR_{B}³, SR_{B}³ oder NR_{B}³R_{B}⁴ oder ein gesättigter
      oder ungesättigter 3- bis 7-gliedriger Heterocyclus mit mindestens einem N-Atom und bis zu 3 Heteroatomen, vorzugsweise aus der Gruppe O und S, der über das N-Atom mit der Carbonylgruppe in (S2) verbunden ist und unsubstituiert oder durch Reste aus der Gruppe (C₁-C₄)Alkyl, (C₁-C₄)Alkoxy oder gegebenenfalls substituiertes Phenyl substituiert ist, vorzugsweise ein Rest der Formel OR_{B}³, NHR_{B}⁴ oder N(CH₃)₂, insbesondere der Formel OR_{B}³;
      - **R**_{B}³: ist Wasserstoff oder ein unsubstituierter oder substituierter aliphatischer Kohlenwasserstoffrest, vorzugsweise mit insgesamt 1 bis 18 C-Atomen;
      - **R**_{B}⁴: ist Wasserstoff, (C₁-C₆)Alkyl, (C₁-C₆)Alkoxy oder substituiertes oder unsubstituiertes Phenyl;
      - **T**_{B}: ist eine (C₁ oder C₂)-Alkandiylkette, die unsubstituiert oder mit einem oder zwei (C₁-C₄)Alkylresten oder mit [(C₁-C₃)-Alkoxy]-carbonyl substituiert ist;
      vorzugsweise:
      a) Verbindungen vom Typ der 8-Chinolinoxyessigsäure (S2^{a}), vorzugsweise (5-Chlor-8-chinolinoxy)essigsäure-(1-methylhexyl)ester ("Cloquintocet-mexyl") (S2-1), (5-Chlor-8-chinolinoxy)essigsäure-(1,3-dimethyl-but-1-yl)ester (S2-2), (5-Chlor-8-chinolinoxy)essigsäure-4-allyloxy-butylester (S2-3), (5-Chlor-8-chinolinoxy)essigsäure-1-allyloxy-prop-2-ylester (S2-4), (5-Chlor-8-chinolinoxy)essigsäureethylester (S2-5), (5-Chlor-8-chinolinoxy)essigsäuremethylester (S2-6), (5-Chlor-8-chinolinoxy)essigsäureallylester (S2-7), (5-Chlor-8-chinolinoxy)essigsäure-2-(2-propyliden-iminoxy)-1-ethylester (S2-8), (5-Chlor-8-chinolinoxy)essigsäure-2-oxo-prop-1-ylester (S2-9) und verwandte Verbindungen, wie sie in EP-A-86 750, EP-A-94 349 und EP-A-191 736 oder EP-A-0 492 366 beschrieben sind, sowie (5-Chlor-8-chinolinoxy)essigsäure (S2-10), deren Hydrate und Salze, beispielsweise deren Lithium-, Natrium-Kalium-, Kalzium-, Magnesium-, Aluminium-, Eisen-, Ammonium-, quartäre Ammonium-, Sulfonium-, oder Phosphoniumsalze wie sie in der WO-A-2002/34048 beschrieben sind;
      b) Verbindungen vom Typ der (5-Chlor-8-chinolinoxy)malonsäure (S2^{b}), vorzugsweise Verbindungen wie (5-Chlor-8-chinolinoxy)malonsäurediethylester, (5-Chlor-8-chinolinoxy)malonsäurediallylester, (5-Chlor-8-chinolin-oxy)malonsäure-methyl-ethylester und verwandte Verbindungen, wie sie in EP-A-0 582 198 beschrieben sind.
   S3) Verbindungen der Formel (S3) 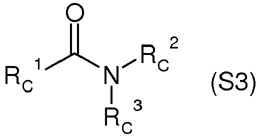 wobei die Symbole und Indizes folgende Bedeutungen haben:
      - R_{C}¹: ist (C₁-C₄)Alkyl, (C₁-C₄)Haloalkyl, (C₂-C₄)Alkenyl, (C₂-C₄)Haloalkenyl, (C₃-C₇)Cycloalkyl, vorzugsweise Dichlormethyl;
      - R_{C}², R_{C}³: sind gleich oder verschieden Wasserstoff, (C₁-C₄)Alkyl, (C₂-C₄)Alkenyl, (C₂-C₄)Alkinyl, (C₁-C₄)Haloalkyl, (C₂-C₄)Haloalkenyl, (C₁-C₄)Alkylcarbamoyl-(C₁-C₄)alkyl, (C₂-C₄)Alkenylcarbamoyl-(C₁-C₄)alkyl, (C₁-C₄)Alkoxy-(C₁-C₄)alkyl, Dioxolanyl-(C₁-C₄)alkyl, Thiazolyl, Furyl, Furylalkyl, Thienyl, Piperidyl, substituiertes oder unsubstituiertes Phenyl, oder R_{C}² und R_{C}³ bilden zusammen einen substituierten oder unsubstituierten heterocyclischen Ring, vorzugsweise einen Oxazolidin-, Thiazolidin-, Piperidin-, Morpholin-, Hexahydropyrimidin-oder Benzoxazinring;
      vorzugsweise:
      Wirkstoffe vom Typ der Dichloracetamide, die häufig als Vorauflaufsafener (bodenwirksame Safener) angewendet werden, wie z. B.
      "Dichlormid" (N,N-Diallyl-2,2-dichloracetamid) (S3-1),
      "R-29148" (3-Dichloracetyl-2,2,5-trimethyl-1,3-oxazolidin) der Firma Stauffer (S3-2),
      "R-28725" (3-Dichloracetyl-2,2,-dimethyl-1,3-oxazolidin) der Firma Stauffer (S3-3),
      "Benoxacor" (4-Dichloracetyl-3,4-dihydro-3-methyl-2H-1,4-benzoxazin) (S3-4), "PPG-1292" (N-Allyl-N-[(1,3-dioxolan-2-yl)-methyl]-dichloracetamid) der Firma PPG Industries (S3-5),
      "DKA-24" (N-Allyl-N-[(allylaminocarbonyl)methyl]-dichloracetamid) der Firma Sagro-Chem (S3-6),
      "AD-67" oder "MON 4660" (3-Dichloracetyl-1-oxa-3-aza-spiro[4,5]decan) der Firma Nitrokemia bzw. Monsanto (S3-7),
      "TI-35" (1-Dichloracetyl-azepan) der Firma TRI-Chemical RT (S3-8), "Diclonon" (Dicyclonon) oder "BAS145138" oder "LAB145138" (S3-9)
      (3-Dichloracetyl-2,5,5-trimethyl-1,3-diazabicyclo[4.3.0]nonan) der Firma BASF, "Furilazol" oder "MON 13900" ((RS)-3-Dichloracetyl-5-(2-furyl)-2,2-dimethyl-oxazolidin) (S3-10); sowie dessen (R)-Isomer (S3-11).
   S4) N-Acylsulfonamide der Formel (S4) und ihre Salze, 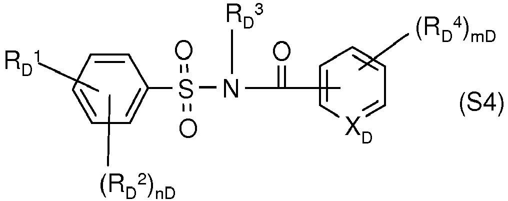 worin die Symbole und Indizes folgende Bedeutungen haben:
      - X_{D}: ist CH oder N;
      - R_{D}¹: ist CO-NR_{D}⁵R_{D}⁶ oder NHCO-R_{D}⁷;
      - R_{D}²: ist Halogen, (C₁-C₄)Haloalkyl, (C₁-C₄)Haloalkoxy, Nitro, (C₁-C₄)Alkyl, (C₁-C₄)Alkoxy, (C₁-C₄)Alkylsulfonyl, (C₁-C₄)Alkoxycarbonyl oder (C₁-C₄)Alkylcarbonyl;
      - R_{D}³: ist Wasserstoff, (C₁-C₄)Alkyl, (C₂-C₄)Alkenyl oder (C₂-C₄)Alkinyl;
      - R_{D}⁴: ist Halogen, Nitro, (C₁-C₄)Alkyl, (C₁-C₄)Haloalkyl, (C₁-C₄)Haloalkoxy, (C₃-C₆)Cycloalkyl, Phenyl, (C₁-C₄)Alkoxy, Cyano, (C₁-C₄)Alkylthio, (C₁-C₄)Alkylsulfinyl, (C₁-C₄)Alkylsulfonyl, (C₁-C₄)Alkoxycarbonyl oder (C₁-C₄)Alkylcarbonyl;
      - R_{D}⁵: ist Wasserstoff, (C₁-C₆)Alkyl, (C₃-C₆)Cycloalkyl, (C₂-C₆)Alkenyl, (C₂-C₆)Alkinyl, (C₅-C₆)Cycloalkenyl, Phenyl oder 3- bis 6-gliedriges Heterocyclyl enthaltend v_{D} Heteroatome aus der Gruppe Stickstoff, Sauerstoff und Schwefel, wobei die sieben letztgenannten Reste durch v_{D}Substituenten aus der Gruppe Halogen, (C₁-C₆)Alkoxy, (C₁-C₆)Haloalkoxy, (C₁-C₂)Alkylsulfinyl, (C₁-C₂)Alkylsulfonyl, (C₃-C₆)Cycloalkyl, (C₁-C₄)Alkoxycarbonyl, (C₁-C₄)Alkylcarbonyl und Phenyl und im Falle cyclischer Reste auch (C₁-C₄) Alkyl und (C₁-C₄)Haloalkyl substituiert sind;
      - R_{D}⁶: ist Wasserstoff, (C₁-C₆)Alkyl, (C₂-C₆)Alkenyl oder (C₂-C₆)Alkinyl, wobei die drei letztgenannten Reste durch v_{D}Reste aus der Gruppe Halogen, Hydroxy, (C₁-C₄)Alkyl, (C₁-C₄)Alkoxy und (C₁-C₄)Alkylthio substituiert sind, oder
      - R_{D}⁵ und R_{D}⁶: gemeinsam mit dem dem sie tragenden Stickstoffatom einen Pyrrolidinyl-oder Piperidinyl-Rest bilden;
      - R_{D}⁷: ist Wasserstoff, (C₁-C₄)Alkylamino, Di-(C₁-C₄)alkylamino, (C₁-C₆)Alkyl, (C₃-C₆)Cycloalkyl, wobei die 2 letztgenannten Reste durch v_{D}Substituenten aus der Gruppe Halogen, (C₁-C₄)Alkoxy, (C₁-C₆)Haloalkoxy und (C₁-C₄)Alkylthio und im Falle cyclischer Reste auch (C₁-C₄)Alkyl und (C₁-C₄)Haloalkyl substituiert sind;
      - n_{D}: ist 0, 1 oder 2;
      - m_{D}: ist 1 oder 2;
      - v_{D}: ist 0, 1, 2 oder 3;
      davon bevorzugt sind Verbindungen von Typ der N-Acylsulfonamide, z.B. der nachfolgenden Formel (S4^{a}), die z. B. bekannt sind aus WO-A-97/45016 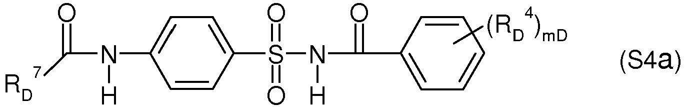 worin
      - R_{D}⁷: (C₁-C₆)Alkyl, (C₃-C₆)Cycloalkyl, wobei die 2 letztgenannten Reste durch v_{D} Substituenten aus der Gruppe Halogen, (C₁-C₄)Alkoxy, (C₁-C₆)Haloalkoxy und (C₁-C₄)Alkylthio und im Falle cyclischer Reste auch (C₁-C₄)Alkyl und (C₁-C₄)Haloalkyl substituiert sind;
      - R_{D}⁴: Halogen, (C₁-C₄)Alkyl, (C₁-C₄)Alkoxy, CF₃;
      - m_{D}: 1 oder 2;
      - v_{D}: ist 0, 1, 2 oder 3 bedeutet;
      sowie
      Acylsulfamoylbenzoesäureamide, z.B. der nachfolgenden Formel (S4^{b}), die z.B. bekannt sind aus WO-A-99/16744, 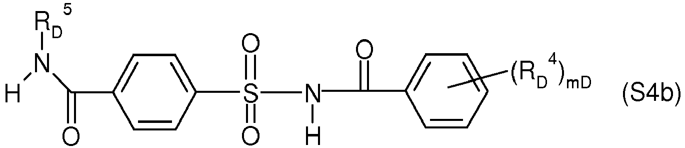 z.B. solche worin
      R_{D}⁵ = Cyclopropyl und (R_{D}⁴) = 2-OMe ist ("Cyprosulfamide", S4-1),
      R_{D}⁵ = Cyclopropyl und (R_{D}⁴) = 5-Cl-2-OMe ist (S4-2),
      R_{D}⁵ = Ethyl und (R_{D}⁴) = 2-OMe ist (S4-3),
      R_{D}⁵ = Isopropyl und (R_{D}⁴) = 5-Cl-2-OMe ist (S4-4) und
      R_{D}⁵ = Isopropyl und (R_{D}⁴) = 2-OMe ist (S4-5)
      sowie
      Verbindungen vom Typ der N-Acylsulfamoylphenylharnstoffe der Formel (S4^{c}), die z.B. bekannt sind aus der EP-A-365484, 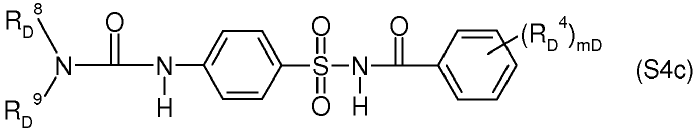 worin
      - R_{D}⁸ und R_{D}⁹: unabhängig voneinander Wasserstoff, (C₁-C₈)Alkyl, (C₃-C₈)Cycloalkyl, (C₃-C₆)Alkenyl, (C₃-C₆)Alkinyl,
      - R_{D}⁴: Halogen, (C₁-C₄)Alkyl, (C₁-C₄)Alkoxy, CF₃
      - m_{D}: 1 oder 2 bedeutet;
      beispielsweise
      1-[4-(N-2-Methoxybenzoylsulfamoyl)phenyl]-3-methylharnstoff,
      1-[4-(N-2-Methoxybenzoylsulfamoyl)phenyl]-3,3-dimethylharnstoff,
      1-[4-(N-4,5-Dimethylbenzoylsulfamoyl)phenyl]-3-methylharnstoff.
   S5) Wirkstoffe aus der Klasse der Hydroxyaromaten und der aromatisch-aliphatischen Carbonsäurederivate (S5), z.B.
      3,4,5-Triacetoxybenzoesäureethylester, 3,5-Dimethoxy-4-hydroxybenzoesäure, 3,5-Dihydroxybenzoesäure, 4-Hydroxysalicylsäure, 4-Fluorsalicyclsäure, 2-Hydroxyzimtsäure, 1,2-Dihydro-2-oxo-6-trifluoromethylpyridin-3-carboxamid, 2,4-Dichlorzimtsäure, wie sie in der WO-A-2004/084631, WO-A-2005/015994, WO-A-2005/016001 beschrieben sind.
   S6) Wirkstoffe aus der Klasse der 1,2-Dihydrochinoxalin-2-one (S6), z.B. 1-Methyl-3-(2-thienyl)-1,2-dihydrochinoxalin-2-on, 1-Methyl-3-(2-thienyl)-1,2-dihydrochinoxalin-2-thion, 1-(2-Aminoethyl)-3-(2-thienyl)-1,2-dihydro-chinoxalin-2-on-hydrochlorid, 1-[2-(Diethylamino)ethyl]-6,7-dimethyl-3-thiophen-2-ylchinoxalin-2(1H)-on, 1-(2-Methylsulfonylaminoethyl)-3-(2-thienyl)-1,2-dihydro-chinoxalin-2-on, wie sie in der WO-A-2005/112630 beschrieben sind.
   S7) Verbindungen der Formel (S7),wie sie in der WO-A-1998/38856 beschrieben sind 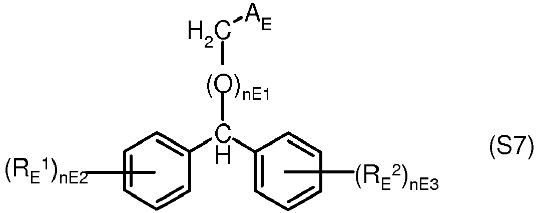 worin die Symbole und Indizes folgende Bedeutungen haben:
      - R_{E}¹, R_{E}²: sind unabhängig voneinander Halogen, (C₁-C₄)Alkyl, (C₁-C₄)Alkoxy, (C₁-C₄)Haloalkyl, (C₁-C₄)Alkylamino, Di-(C₁-C₄)Alkylamino, Nitro;
      - A_{E}: ist COOR_{E}³ oder COSR_{E}⁴
      - R_{E}³, R_{E}⁴: sind unabhängig voneinander Wasserstoff, (C₁-C₄)Alkyl, (C₂-C₆)Alkenyl, (C₂-C₄)Alkinyl, Cyanoalkyl, (C₁-C₄)Haloalkyl, Phenyl, Nitrophenyl, Benzyl, Halobenzyl, Pyridinylalkyl und Alkylammonium,
      - n_{E}¹: ist 0 oder 1
      - n_{E}², n_{E}³: sind unabhängig voneinander 0, 1 oder 2,
      vorzugsweise:
      Diphenylmethoxyessigsäure,
      Diphenylmethoxyessigsäureethylester,
      Diphenylmethoxyessigsäuremethylester (CAS-Reg.Nr. 41858-19-9) (S7-1).
   S8) Verbindungen der Formel (S8),wie sie in der WO-A-98/27049 beschrieben sind 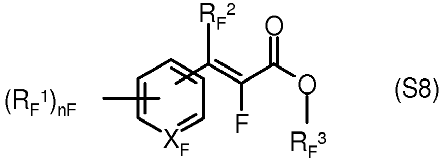 worin
      - X_{F}: CH oder N,
      - n_{F}: für den Fall, dass X_{F}=N ist, eine ganze Zahl von 0 bis 4 und
      für den Fall, dass X_{F}=CH ist, eine ganze Zahl von 0 bis 5 ,
      - R_{F}¹: Halogen, (C₁-C₄)Alkyl, (C₁-C₄)Haloalkyl, (C₁-C₄)Alkoxy, (C₁-C₄)Haloalkoxy, Nitro, (C₁-C₄)Alkylthio, (C₁-C₄)-Alkylsulfonyl, (C₁-C₄)Alkoxycarbonyl, ggf. substituiertes. Phenyl, ggf. substituiertes Phenoxy,
      - R_{F}²: Wasserstoff oder (C₁-C₄)Alkyl
      - R_{F}³: Wasserstoff, (C₁-C₈)Alkyl, (C₂-C₄)Alkenyl, (C₂-C₄)Alkinyl, oder Aryl, wobei jeder der vorgenannten C-haltigen Reste unsubstituiert oder durch einen oder mehrere, vorzugsweise bis zu drei gleiche oder verschiedene Reste aus der Gruppe, bestehend aus Halogen und Alkoxy substituiert ist;bedeuten, oder deren Salze,
      vorzugsweise Verbindungen worin
      - X_{F}: CH,
      - n_{F}: eine ganze Zahl von 0 bis 2 ,
      - R_{F}¹: Halogen, (C₁-C₄)Alkyl, (C₁-C₄)Haloalkyl, (C₁-C₄)Alkoxy, (C₁-C₄)Haloalkoxy,
      - R_{F}²: Wasserstoff oder (C₁-C₄)Alkyl,
      - R_{F}³: Wasserstoff, (C₁-C₈)Alkyl, (C₂-C₄)Alkenyl, (C₂-C₄)Alkinyl, oder Aryl, wobei jeder der vorgenannten C-haltigen Reste unsubstituiert oder durch einen oder mehrere, vorzugsweise bis zu drei gleiche oder verschiedene Reste aus der Gruppe, bestehend aus Halogen und Alkoxy substituiert ist, bedeuten, oder deren Salze.
   S9) Wirkstoffe aus der Klasse der 3-(5-Tetrazolylcarbonyl)-2-chinolone (S9), z.B. 1,2-Dihydro-4-hydroxy-1-ethyl-3-(5-tetrazolylcarbonyl)-2-chinolon (CAS-Reg.Nr. 219479-18-2), 1,2-Dihydro-4-hydroxy-1-methyl-3-(5-tetrazolyl-carbonyl)-2-chinolon (CAS-Reg.Nr. 95855-00-8), wie sie in der WO-A-1999/000020 beschrieben sind.
   S10) Verbindungen der Formeln (S10^{a}) oder (S10^{b})
      wie sie in der WO-A-2007/023719 und WO-A-2007/023764 beschrieben sind 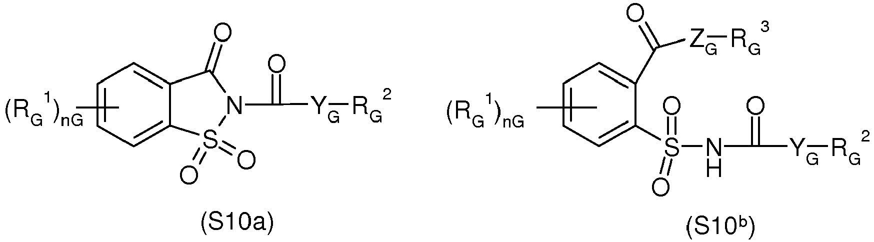 worin
      - R_{G}¹: Halogen, (C₁-C₄)Alkyl, Methoxy, Nitro, Cyano, CF₃, OCF₃
      - Y_{G}, Z_{G}: unabhängig voneinander O oder S,
      - n_{G}: eine ganze Zahl von 0 bis 4,
      - R_{G}²: (C₁-C₁₆)Alkyl, (C₂-C₆)Alkenyl, (C₃-C₆)Cycloalkyl, Aryl; Benzyl, Halogenbenzyl,
      - R_{G}³: Wasserstoff oder (C₁-C₆)Alkyl bedeutet.
   S11) Wirkstoffe vom Typ der Oxyimino-Verbindungen (S11), die als Saatbeizmittel bekannt sind, wie z. B.
      "Oxabetrinil" ((Z)-1,3-Dioxolan-2-ylmethoxyimino(phenyl)acetonitril) (S11-1), das als Saatbeiz-Safener für Hirse gegen Schäden von Metolachlor bekannt ist,
      "Fluxofenim" (1-(4-Chlorphenyl)-2,2,2-trifluor-1-ethanon-O-(1,3-dioxolan-2-ylmethyl)-oxim) (S11-2), das als Saatbeiz-Safener für Hirse gegen Schäden von Metolachlor bekannt ist, und
      "Cyometrinil" oder "CGA-43089" ((Z)-Cyanomethoxyimino(phenyl)acetonitril) (S11-3), das als Saatbeiz-Safener für Hirse gegen Schäden von Metolachlor bekannt ist.
   S12) Wirkstoffe aus der Klasse der Isothiochromanone (S12), wie z.B. Methyl-[(3-oxo-1H-2-benzothiopyran-4(3H)-yliden)methoxy]acetate (CAS-Reg.Nr. 205121-04-6) (S12-1) und verwandte Verbindungen aus WO-A-1998/13361.
   S13) Eine oder mehrere Verbindungen aus Gruppe (S13):
      "Naphthalic anhydrid" (1,8-Naphthalindicarbonsäureanhydrid) (S13-1), das als Saatbeiz-Safener für Mais gegen Schäden von Thiocarbamatherbiziden bekannt ist,
      "Fenclorim" (4,6-Dichlor-2-phenylpyrimidin) (S13-2), das als Safener für Pretilachlor in gesätem Reis bekannt ist,
      "Flurazole" (Benzyl-2-chlor-4-trifluormethyl-1,3-thiazol-5-carboxylat) (S13-3), das als Saatbeiz-Safener für Hirse gegen Schäden von Alachlor und Metolachlor bekannt ist,
      "CL 304415" (CAS-Reg.Nr. 31541-57-8) (4-Carboxy-3,4-dihydro-2H-1-benzopyran-4-essigsäure) (S13-4) der Firma American Cyanamid, das als Safener für Mais gegen Schäden von Imidazolinonen bekannt ist,
      "MG 191" (CAS-Reg.Nr. 96420-72-3) (2-Dichlormethyl-2-methyl-1,3-dioxolan) (S13-5) der Firma Nitrokemia, das als Safener für Mais bekannt ist,
      "MG-838" (CAS-Reg.Nr. 133993-74-5) (2-propenyl 1-oxa-4-azaspiro[4.5]decane-4-carbodithioate) (S13-6) der Firma Nitrokemia,
      "Disulfoton" (O,O-Diethyl S-2-ethylthioethyl phosphordithioat) (S13-7),
      "Dietholate" (O,O-Diethyl-O-phenylphosphorotioat) (S13-8),
      "Mephenate" (4-Chlorphenyl-methylcarbamat) (S13-9).
   S14) Wirkstoffe, die neben einer herbiziden Wirkung gegen Schadpflanzen auch Safenerwirkung an Kulturpflanzen wie Reis aufweisen, wie z. B. "Dimepiperate" oder "MY-93" (S-1-Methyl-1-phenylethyl-piperidin-1-carbothioat), das als Safener für Reis gegen Schäden des Herbizids Molinate bekannt ist,
      "Daimuron" oder "SK 23" (1-(1-Methyl-1-phenylethyl)-3-p-tolyl-harnstoff), das als Safener für Reis gegen Schäden des Herbizids Imazosulfuron bekannt ist,
      "Cumyluron" = "JC-940" (3-(2-Chlorphenylmethyl)-1-(1-methyl-1-phenyl-ethyl)harnstoff, siehe JP-A-60087254), das als Safener für Reis gegen Schäden einiger Herbizide bekannt ist,
      "Methoxyphenon" oder "NK 049" (3,3'-Dimethyl-4-methoxy-benzophenon), das als Safener für Reis gegen Schäden einiger Herbizide bekannt ist,
      "CSB" (1-Brom-4-(chlormethylsulfonyl)benzol) von Kumiai, (CAS-Reg.Nr. 54091-06-4), das als Safener gegen Schäden einiger Herbizide in Reis bekannt ist.
   S15) Verbindungen der Formel (S15) oder deren Tautomere 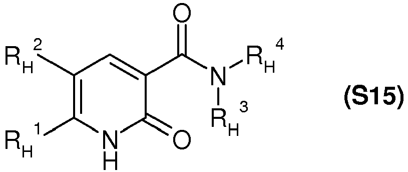 wie sie in der WO-A-2008/131861 und WO-A-2008/131860 beschrieben sind
      worin
      - R_{H}¹: einen (C₁-C₆)Haloalkylrest bedeutet und
      - R_{H}²: Wasserstoff oder Halogen bedeutet und
      - R_{H}³, R_{H}⁴: unabhängig voneinander Wasserstoff, (C₁-C₁₆)Alkyl, (C₂-C₁₆)Alkenyl oder (C₂-C₁₆)Alkinyl,
      wobei jeder der letztgenannten 3 Reste unsubstituiert oder durch einen oder mehrere Reste aus der Gruppe Halogen, Hydroxy, Cyano, (C₁-C₄)Alkoxy, (C₁-C₄)Haloalkoxy, (C₁-C₄)Alkylthio, (C₁-C₄)Alkylamino, Di[(C₁-C₄)alkyl]-amino, [(C₁-C₄)Alkoxy]-carbonyl, [(C₁-C₄)Haloalkoxy]-carbonyl, (C₃-C₆)Cycloalkyl, das unsubstituiert oder substituiert ist, Phenyl, das unsubstituiert oder substituiert ist, und Heterocyclyl, das unsubstituiert oder substituiert ist, substituiert ist,
      oder (C₃-C₆)Cycloalkyl, (C₄-C₆)Cycloalkenyl, (C₃-C₆)Cycloalkyl, das an einer Seite des Rings mit einem 4 bis 6-gliedrigen gesättigten oder ungesättigten carbocyclischen Ring kondensiert ist, oder (C₄-C₆)Cycloalkenyl, das an einer Seite des Rings mit einem 4 bis 6-gliedrigen gesättigten oder ungesättigten carbocyclischen Ring kondensiert ist,
      wobei jeder der letztgenannten 4 Reste unsubstituiert oder durch einen oder mehrere Reste aus der Gruppe Halogen, Hydroxy, Cyano, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Alkylamino, Di[(C₁-C₄)alkyl]-amino, [(C₁-C₄)Alkoxy]-carbonyl, [(C₁-C₄)Haloalkoxy]-carbonyl, (C₃-C₆)Cycloalkyl, das unsubstituiert oder substituiert ist, Phenyl, das unsubstituiert oder substituiert ist, und Heterocyclyl, das unsubstituiert oder substituiert ist, substituiert ist,
      bedeutet oder
      - R_{H}³: (C₁-C₄)-Alkoxy, (C₂-C₄)Alkenyloxy, (C₂-C₆)Alkinyloxy oder (C₂-C₄)Haloalkoxy bedeutet und
      - R_{H}⁴: Wasserstoff oder (C₁-C₄)-Alkyl bedeutet oder
      - R_{H}³ und R_{H}⁴: zusammen mit dem direkt gebundenen N-Atom einen vier- bis achtgliedrigen heterocyclischen Ring, der neben dem N-Atom auch weitere Heteroringatome, vorzugsweise bis zu zwei weitere Heteroringatome aus der Gruppe N, O und S enthalten kann und der unsubstituiert oder durch einen oder mehrere Reste aus der Gruppe Halogen, Cyano, Nitro, (C₁-C₄)Alkyl, (C₁-C₄)Haloalkyl, (C₁-C₄)Alkoxy, (C₁-C₄)Haloalkoxy und (C₁-C₄)Alkylthio substituiert ist, bedeutet.
   S16) Wirkstoffe, die vorrangig als Herbizide eingesetzt werden, jedoch auch Safenerwirkung auf Kulturpflanzen aufweisen, z.B.
      (2,4-Dichlorphenoxy)essigsäure (2,4-D),
      (4-Chlorphenoxy)essigsäure,
      (R,S)-2-(4-Chlor-o-tolyloxy)propionsäure (Mecoprop),
      4-(2,4-Dichlorphenoxy)buttersäure (2,4-DB),
      (4-Chlor-o-tolyloxy)essigsäure (MCPA),
      4-(4-Chlor-o-tolyloxy)buttersäure,
      4-(4-Chlorphenoxy)buttersäure,
      3,6-Dichlor-2-methoxybenzoesäure (Dicamba),
      1-(Ethoxycarbonyl)ethyl-3,6-dichlor-2-methoxybenzoat (Lactidichlor-ethyl).

Als die die Kulturpflanzen-Verträglichkeit verbessernde Verbindung [Komponente (b')] sind Cloquintocet-mexyl, Fenchlorazol-ethylester, Isoxadifen-ethyl, Mefenpyr-diethyl, Fenclorim, Cumyluron, S4-1 und S4-5 am meisten bevorzugt, wobei Cloquintocet-mexyl und Mefenpyr-diethyl besonders hervorgehoben seien.

Es wurde nun überraschend gefunden, dass die oben definierten Wirkstoffkombinationen aus Verbindungen der allgemeinen Formel (I-a) und Safenern (Antidots) aus der oben aufgeführten Gruppe (b') bei sehr guter Nutzpflanzen-Verträglichkeit eine besonders hohe herbizide Wirksamkeit aufweisen und in verschiedenen Kulturen, insbesondere in Getreide (vor allem Weizen), aber auch in Soja, Kartoffeln, Mais und Reis zur selektiven Unkrautbekämpfung verwendet werden können.

Dabei ist es als überraschend anzusehen, dass aus einer Vielzahl von bekannten Safenern oder Antidots, die befähigt sind, die schädigende Wirkung eines Herbizids auf die Kulturpflanzen zu antagonisieren, gerade die oben aufgeführten Verbindungen der Gruppe (b') geeignet sind, die schädigende Wirkung von Verbindungen der Formel (I-a) auf die Kulturpflanzen annähernd vollständig aufzuheben, ohne dabei die herbizide Wirksamkeit gegenüber den Unkräutern maßgeblich zu beeinträchtigen.

Hervorgehoben sei hierbei die besonders vorteilhafte Wirkung der besonders und am meisten bevorzugten Kombinationspartner aus der Gruppe (b'), insbesondere hinsichtlich der Schonung von Getreidepflanzen, wie z.B. Weizen, Gerste und Roggen, aber auch Mais und Reis, als Kulturpflanzen.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Gegebenenfalls substituierte Reste können, sofern nichts anderes angegeben ist, einfach oder mehrfach substituiert sein, wobei bei Mehrfachsubstitutionen die Substituenten gleich oder verschieden sein können.

Im einzelnen seien außer den bei den Beispielen genannten Verbindungen die folgenden Verbindungen der Formel (I-a) genannt:

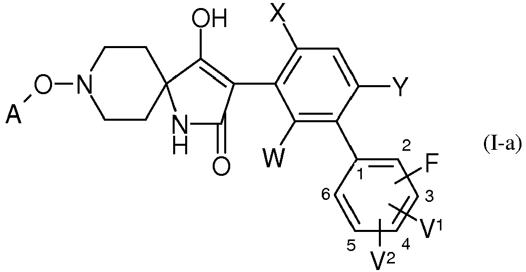

**Tabelle 1**

| **A** | **W** | **X** | **Y** | **F** | **V¹** | **V²** |
|---|---|---|---|---|---|---|
| | | | | | | |
| | | | | | | |
| | | | | | | |
| | | | | | | |
| | | | | | | |
| | | | | | | |
| | | | | | | |
| | | | | | | |
| | | | | | | |
| | | | | | | |
| | | | | | | |
| | | | | | | |
| | | | | | | |
| | | | | | | |
| | | | | | | |
| | | | | | | |
| | | | | | | |
| | | | | | | |
| | | | | | | |
| | | | | | | |
| | | | | | | |
| | | | | | | |
| | | | | | | |
| | | | | | | |
| | | | | | | |
| | | | | | | |
| | | | | | | |
| | | | | | | |
| | | | | | | |
| | | | | | | |
| | | | | | | |
| | | | | | | |
| | | | | | | |
| | | | | | | |
| | | | | | | |
| | | | | | | |
| CH₃ | CH₃ | CH₃ | H | 2 | H | H |
| CH₃ | CH₃ | CH₃ | H | 3 | H | H |
| CH₃ | CH₃ | CH₃ | H | 4 | H | H |
| CH₃ | CH₃ | CH₃ | H | 2 | 4-F | H |
| CH₃ | CH₃ | CH₃ | H | 2 | 4-Cl | H |
| CH₃ | CH₃ | CH₃ | H | 2 | 4-CH₃ | H |
| CH₃ | CH₃ | CH₃ | H | 2 | 4-OCH₃ | H |
| CH₃ | CH₃ | CH₃ | H | 3 | 4-F | H |
| CH₃ | CH₃ | CH₃ | H | 3 | 4-Cl | H |
| CH₃ | CH₃ | CH₃ | H | 3 | 4-CH₃ | H |
| CH₃ | CH₃ | CH₃ | H | 3 | 4-OCH₃ | H |
| CH₃ | CH₃ | CH₃ | H | 4 | 3-Cl | H |
| CH₃ | CH₃ | CH₃ | H | 4 | 3-CH₃ | H |
| CH₃ | CH₃ | CH₃ | H | 4 | 3-OCH₃ | H |
| CH₃ | CH₃ | CH₃ | H | 2 | 4-F | 5-F |
| CH₃ | CH₃ | CH₃ | H | 2 | 4-F | 6-F |
| CH₃ | CH₃ | CH₃ | H | 2 | 4-Cl | 5-F |
| CH₃ | CH₃ | CH₃ | H | 2 | 5-Cl | 4-F |
| CH₃ | CH₃ | CH₃ | H | 3 | 4-F | 5-F |
| | | | | | | |
| | | | | | | |
| | | | | | | |
| | | | | | | |
| | | | | | | |
| | | | | | | |
| | | | | | | |
| | | | | | | |
| | | | | | | |
| | | | | | | |
| | | | | | | |
| | | | | | | |
| | | | | | | |
| | | | | | | |
| | | | | | | |
| | | | | | | |
| | | | | | | |
| CH₃ | CH₃ | CH₃ | CH₃ | 2 | H | H |
| CH₃ | CH₃ | CH₃ | CH₃ | 3 | H | H |
| CH₃ | CH₃ | CH₃ | CH₃ | 4 | H | H |
| CH₃ | CH₃ | CH₃ | CH₃ | 2 | 4-F | H |
| CH₃ | CH₃ | CH₃ | CH₃ | 2 | 4-Cl | H |
| CH₃ | CH₃ | CH₃ | CH₃ | 2 | 4-CH₃ | H |
| CH₃ | CH₃ | CH₃ | CH₃ | 2 | 4-OCH₃ | H |
| CH₃ | CH₃ | CH₃ | CH₃ | 3 | 4-F | H |
| CH₃ | CH₃ | CH₃ | CH₃ | 3 | 4-Cl | H |
| CH₃ | CH₃ | CH₃ | CH₃ | 3 | 4-CH₃ | H |
| CH₃ | CH₃ | CH₃ | CH₃ | 3 | 4-OCH₃ | H |
| CH₃ | CH₃ | CH₃ | CH₃ | 4 | 3-Cl | H |
| CH₃ | CH₃ | CH₃ | CH₃ | 4 | 3-CH₃ | H |
| CH₃ | CH₃ | CH₃ | CH₃ | 4 | 3-OCH₃ | H |
| CH₃ | CH₃ | CH₃ | CH₃ | 2 | 4-F | 5-F |
| CH₃ | CH₃ | CH₃ | CH₃ | 2 | 4-F | 6-F |
| CH₃ | CH₃ | CH₃ | CH₃ | 2 | 4-Cl | 5-F |
| CH₃ | CH₃ | CH₃ | CH₃ | 2 | 5-Cl | 4-F |
| CH₃ | CH₃ | CH₃ | CH₃ | 3 | 4-F | 5-F |

Tabelle 2 W, X, Y, F, V¹ und V² wie in Tabelle 1 angegeben A = C₂H₅
Tabelle 3 W, X, Y, F, V¹ und V² wie in Tabelle 1 angegeben A = C₃H₇

In der Literatur wurde bereits beschrieben, dass sich die Wirkung verschiedener Wirkstoffe durch Zugabe von Ammoniumsalzen steigern lässt. Dabei handelt es sich jedoch um als Detergens wirkende Salze (z.B. WO 95/017817) bzw. Salze mit längeren Alkyl- und / oder Arylsubstituenten, die permeabilisierend wirken oder die Löslichkeit des Wirkstoffs erhöhen (z.B. EP-A 0 453 086, EP-A 0 664 081, FR-A 2 600 494, US 4 844 734, US 5 462 912, US 5 538 937, US-A 03/0224939, US-A 05/0009880, US-A 05/0096386). Weiterhin beschreibt der Stand der Technik die Wirkung nur für bestimmte Wirkstoffe und / oder bestimmte Anwendungen der entsprechenden Mittel. In wieder anderen Fällen handelt es sich um Salze von Sulfonsäuren, bei denen die Säuren selber paralysierend auf Insekten wirken (US 2 842 476). Eine Wirkungssteigerung z.B. durch Ammoniumsulfat ist beispielsweise für die Herbizide Glyphosat, Phosphinothricin und für phenylsubstituierte Cyclische Ketoenole beschrieben (US 6 645 914, EP-A2 0 036 106, z.B. WO 07/068427). Eine entsprechende Wirkungssteigerung bei Insektiziden wurde bereits durch z.B. WO 07/068428 beschrieben.

Auch der Einsatz von Ammoniumsulfat als Formulierhilfsmittel ist für bestimmte Wirkstoffe und Anwendungen beschrieben (WO 92/16108), es dient dort aber zur Stabilisierung der Formulierung, nicht zur Wirkungssteigerung.

Es wurde nun ebenfalls überraschend gefunden, dass sich die Wirkung von Insektiziden und/oder Akariziden und/oder Fungiziden und/oder Herbiziden aus der Klasse der spiroheterocyclisch-substituierte Tetramsäure-Derivate der Formel (I-a) durch den Zusatz von Ammonium- oder Phosphoniumsalzen zur Anwendungslösung oder durch den Einbau dieser Salze in eine Formulierung enthaltend spiroheterocyclisch-substituierte Tetramsäure-Derivate der Formel (I-a) deutlich steigern lässt. Gegenstand der vorliegenden Erfindung ist also die Verwendung von Ammonium- oder Phosphoniumsalzen zur Wirkungssteigerung von Pflanzenschutzmitteln, die herbizid und/oder funzigid und/oder insektizid und/oder akarizid wirksame spiroheterocyclisch-substituierte Tetramsäure-Derivate der Formel (I-a) als Wirkstoff enthalten. Gegenstand der Erfindung sind ebenfalls Mittel, die herbizid und/oder akarizid und/oder insektizid und/oder fungizid wirksame spiroheterocyclisch-substituierte Tetramsäure-Derivate der Formel (I-a) und die Wirkung steigernde Ammonium- oder Phosphoniumsalze enthalten und zwar sowohl formulierte Wirkstoffe als auch anwendungsfertige Mittel (Spritzbrühen). Gegenstand der Erfindung ist schließlich weiterhin die Verwendung dieser Mittel zur Bekämpfung von Schadinsekten und/oder Spinnmilben und/oder unerwünschten Pflanzenwuchs und/oder Mikroorganismen.

Die Verbindungen der Formel (I-a) besitzen eine breite insektizide und/oder akarizide und/oder fungizide und/oder herbizide Wirkung, die Wirkung und/oder Pflanzenverträglichkeit lässt im Einzelnen aber zu wünschen übrig.

Die Wirkstoffe können in den erfindungsgemäßen Zusammensetzungen in einem breiten Konzentrationsbereich eingesetzt werden. Die Konzentration der Wirkstoffe in der Formulierung beträgt dabei üblicherweise 0,1 - 50 Gen.-%.

Ammonium- und Phosphoniumsalze, die erfindungsgemäß die Wirkung von Pflanzenschutzmitteln enthaltend Verbindungen der Formel (I-a) steigern, werden durch Formel (III') definiert 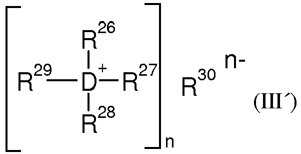 in welcher
- D: für Stickstoff oder Phosphor steht,
- D: bevorzugt für Stickstoff steht,
- R²⁶, R²⁷, R²⁸ und R²⁹: unabhängig voneinander für Wasserstoff oder jeweils gegebenenfalls substituiertes C₁-C₈-Alkyl oder einfach oder mehrfach ungesättigtes, gegebenenfalls substituiertes C₁-C₈-Alkylen stehen, wobei die Substituenten aus Halogen, Nitro und Cyano ausgewählt sein können,
- R²⁶, R²⁷, R²⁸ und R²⁹: bevorzugt unabhängig voneinander für Wasserstoff oder jeweils gegebenenfalls substituiertes C₁-C₄-Alkyl stehen, wobei die Substituenten aus Halogen, Nitro und Cyano ausgewählt sein können,
- R²⁶, R²⁷ , R²⁸ und R²⁹: besonders bevorzugt unabhängig voneinander für Wasserstoff, Methyl, Ethyl, n-Propyl, i-Propyl, n-Butyl, i-Butyl, s-Butyl oder t-Butyl stehen,
- R²⁶, R²⁷, R²⁸ und R²⁹: ganz besonders bevorzugt für Wasserstoff stehen,
- n: für 1, 2, 3 oder 4 steht,
- n: bevorzugt für 1 oder 2 steht,
- R³⁰: für ein anorganisches oder organisches Anion steht,
- R³⁰: bevorzugt für Hydrogencarbonat, Tetraborat, Fluorid, Bromid, Jodid, Chlorid, Monohydrogenphosphat, Dihydrogenphosphat, Hydrogensulfat, Tartrat, Sulfat, Nitrat, Thiosulfat, Thiocyanat, Formiat, Laktat, Acetat, Propionat, Butyrat, Pentanoat oder Oxalat steht,
- R³⁰: besonders bevorzugt für Laktat, Monohydrogenphosphat, Dihydrogenphospat, Sulfat, Nitrat, Thiosulfat, Thiocyanat, Oxalat oder Formiat steht,
- R³⁰: ganz besonders bevorzugt für Sulfat steht.

Die Ammonium- und Phosphoniumsalze der Formel (III') können in einem breiten Konzentrationsbereich zur Steigerung der Wirkung von Pflanzenschutzmitteln enthaltend spiroheterocyclisch-substituierte Tetramsäure-Derivate der Formel (I) eingesetzt werden. Im Allgemeinen werden die Ammonium- oder Phosphoniumsalze im anwendungsfertigen Pflanzenschutzmittel in einer Konzentration von 0,5 bis 80 mmol/l, bevorzugt 0,75 bis 37,5 mmol/l, besonders bevorzugt 1,5 bis 25 mmol/l eingesetzt. Im Fall eines formulierten Produktes wird die Ammonium- und/oder Phosphoniumsalzkonzentration in der Formulierung so gewählt, dass sie nach Verdünnung der Formulierung auf die gewünschte Wirkstoffkonzentration in diesen angegebenen allgemeinen, bevorzugten oder besonders bevorzugten Bereichen liegt. Die Konzentration des Salzes in der Formulierung beträgt dabei üblicherweise 1 - 50 Gew.-%.

In einer bevorzugten Ausführungsform der Erfindung wird den Pflanzenschutzmitteln zur Wirkungssteigerung nicht nur ein Ammonium- und/oder Phosphoniumsalz, sondern zusätzlich ein Penetrationsförderer zugegeben. Es ist als völlig überraschend zu bezeichnen, dass selbst in diesen Fällen eine noch weiter gehende Wirkungssteigerung zu beobachten ist. Gegenstand der vorliegenden Erfindung ist also ebenfalls die Verwendung einer Kombination von Penetrationsförderer und Ammonium- und/oder Phosphoniumsalzen zur Wirkungssteigerung von Pflanzenschutzmitteln, die insektizid und/oder akarizid und/oder fungizid und/oder herbizid wirksame, spiroheterocyclisch-substituierte Tetramsäure-Derivate der Formel (I-a) als Wirkstoff enthalten. Gegenstand der Erfindung sind ebenfalls Mittel, die herbizid und/oder fungizid und/oder akarizid und/oder insektizid wirksame spiroheterocyclisch-substituierte Tetramsäure-Derivate der Formel (I), Penetrationsförderer und Ammonium- und/oder Phosphoniumsalze enthalten und zwar sowohl formulierte Wirkstoffe als auch anwendungsfertige Mittel (Spritzbrühen). Gegenstand der Erfindung ist schließlich weiterhin die Verwendung dieser Mittel zur Bekämpfung von Schadinsekten, Spinnenmilben, Pflanzenwuchs und/oder Mikroorganismen.

Als Penetrationsförderer kommen im vorliegenden Zusammenhang alle diejenigen Substanzen in Betracht, die üblicherweise eingesetzt werden, um das Eindringen von agrochemischen Wirkstoffen in Pflanzen zu verbessern. Penetrationsförderer werden in diesem Zusammenhang dadurch definiert, dass sie aus der wässrigen Spritzbrühe und/oder aus dem Spritzbelag in die Kutikula der Pflanze eindringen und dadurch die Stoffbeweglichkeit (Mobilität) von Wirkstoffen in der Kutikula erhöhen können. Die in der Literatur (Baur et al., 1997, Pesticide Science 51, 131-152) beschriebene Methode kann zur Bestimmung dieser Eigenschaft eingesetzt werden.

Als Penetrationsförderer kommen beispielsweise Alkanol-alkoxylate in Betracht. Erfindungsgemäße Penetrationsförderer sind Alkanol-alkoxylate der Formel (IV')

R-O-(-AO)ᵥ-R' (IV')

in welcher
- R: für geradkettiges oder verzweigtes Alkyl mit 4 bis 20 Kohlenstoffatomen steht,
- R': für Wasserstoff, Methyl, Ethyl, n-Propyl, i-Propyl, n-Butyl, i-Butyl, t-Butyl, n-Pentyl oder n-Hexyl steht,
- AO: für einen Ethylenoxid-Rest, einen Propylenoxid-Rest, einen Butylenoxid-Rest oder für Gemische aus Ethylenoxid- und Propylenoxid-Resten oder Butylenoxid-Resten steht und
- v: für Zahlen von 2 bis 30 steht.

Eine bevorzugte Gruppe von Penetrationsförderern sind Alkanolalkoxylate der Formel

R-O-(-EO-)ₙ-R' (IV'-a)

in welcher
- R: die oben angegebene Bedeutung hat,
- R': die oben angegebene Bedeutung hat,
- EO: für -CH₂-CH₂-O- steht und
- n: für Zahlen von 2 bis 20 steht.

Eine weitere bevorzugte Gruppe von Penetrationsförderern sind Alkanol-alkoxylate der Formel

R-O-(-EO-)ₚ-(-PO-)_{q}-R' (IV'-b)

in welcher
- R: die oben angegebene Bedeutung hat,
- R': die oben angegebene Bedeutung hat,
- EO: für -CH₂-CH₂-O- steht,
- PO: für 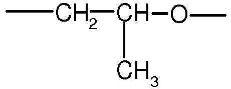 steht,
- p: für Zahlen von 1 bis 10 steht und
- q: für Zahlen von 1 bis 10 steht.

Eine weitere bevorzugte Gruppe von Penetrationsförderern sind Alkanol-Alkoxylate der Formel

R-O-(-PO-)ᵣ-(EO-)ₛ-R' (IV'-c)

in welcher
- R: die oben angegebene Bedeutung hat,
- R': die oben angegebene Bedeutung hat,
- EO: für -CH₂-CH₂-O- steht,
- PO: für 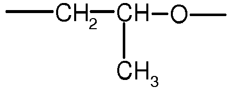 steht, CH₃
- r: für Zahlen von 1 bis 10 steht und
- s: für Zahlen von 1 bis 10 steht.

Eine weitere bevorzugte Gruppe von Penetrationsförderern sind Alkanol-alkoxylate der Formel

R-O-(-EO-)ₚ-(-BO-)_{q}-R' (IV'-d)

in welcher

R und R' die oben angegebenen Bedeutungen haben,
- EO: für -CH₂-CH₂-O- steht,

- BO: für 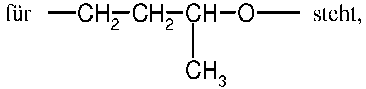 steht,
- p: für Zahlen von 1 bis 10 steht und
- q: für Zahlen von 1 bis 10 steht.

Eine weitere bevorzugte Gruppe von Penetrationsförderern sind Alkanol-alkoxylate der Formel

R-O-(-BO-)ᵣ-(-EO-)ₛ-R' (IV'-e)

in welcher
R und R' die oben angegebenen Bedeutungen haben,
- BO: für 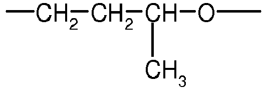 steht,
- EO: für -CH₂-CH₂-O- steht,
- r: für Zahlen von 1 bis 10 steht und
- s: für Zahlen von 1 bis 10 steht.

Eine weitere bevorzugte Gruppe von Penetrationsförderern sind Alkanol-Alkoxylate der Formel

CH₃-(CH₂)ₜ-CH₂-O-(-CH₂-CH₂-O-)ᵤ-R' (IV'-f)

in welcher
- R': die oben angegebene Bedeutung hat,
- t: für Zahlen von 8 bis 13 steht
- u: für Zahlen von 6 bis 17 steht.

In den zuvor angegebenen Formeln steht
- R: vorzugsweise für Butyl, i-Butyl, n-Pentyl, i-Pentyl, Neopentyl, n-Hexyl, i-Hexyl, n-Octyl, i-Octyl, 2-Ethyl-hexyl, Nonyl, i-Nonyl, Decyl, n-Dodecyl, i-Dodecyl, Lauryl, Myristyl, i-Tridecyl, Trimethyl-nonyl, Palmityl, Stearyl oder Eicosyl.

Als Beispiel für ein Alkanol-Alkoxylat der Formel (IV'-c) sei 2-Ethyl-hexyl-alkoxylat der Formel 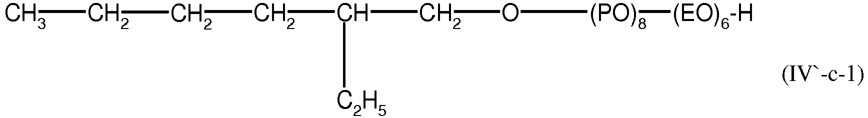 in welcher
- EO: für -CH₂-CH₂-O- steht,
- PO: für 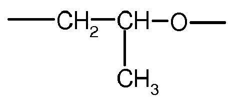 steht und
die Zahlen 8 und 6 Durchschnittswerte darstellen, genannt.

Als Beispiel für ein Alkanol-Alkoxylat der Formel (IV'-d) sei die Formel

CH₃-(CH₂)₁₀-O-(-EO-)₆-(-BO-)₂-CH₃ (IV'-d-1)

in welcher
- EO: für -CH₂-CH₂-O- steht,
- BO: für 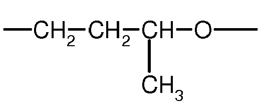 steht und
die Zahlen 10, 6 und 2 Durchschnittswerte darstellen, genannt.

Besonders bevorzugte Alkanol-Alkoxylate der Formel (IV'-f) sind Verbindungen dieser Formel, in denen
- t: für Zahlen von 9 bis 12 und
- u: für Zahlen von 7 bis 9
steht.

Ganz besonders bevorzugt genannt sei Alkanol-Alkoxylat der Formel (IV'-f-1)

CH₃-(CH₂)ₜ-CH₂-0-(-CH₂-CH₂-O-)ᵤ-H (IV'-f-1)

in welcher
- t: für den Durchschnittswert 10,5 steht und
- u: für den Durchschnittswert 8,4 steht.

Die Alkanol-Alkoxylate sind durch die obigen Formeln allgemein definiert. Bei diesen Substanzen handelt es sich um Gemische von Stoffen des angegebenen Typs mit unterschiedlichen Kettenlängen. Für die Indices errechnen sich deshalb Durchschnittswerte, die auch von ganzen Zahlen abweichen können.

Die Alkanol-Alkoxylate der angegebenen Formeln sind bekannt und sind teilweise kommerziell erhältlich oder lassen sich nach bekannten Methoden herstellen (vgl. WO 98/35 553, WO 00/35 278 und EP-A 0 681 865).

Als Penetrationsförderer kommen beispielsweise auch Substanzen in Betracht, die die Verfügbarkeit der Verbindungen der Formel (I-a) im Spritzbelag fördern. Dazu gehören beispielsweise mineralische oder vegetabile Öle. Als Öle kommen alle üblicherweise in agrochemischen Mitteln einsetzbaren mineralischen oder vegetabilen - gegebenenfalls modifizierte - Öle in Frage. Beispielhaft genannt seien Sonnenblumenöl, Rapsöl, Olivenöl, Rizinusöl, Rüböl, Maiskernöl, Baumwollsaatöl und Sojabohnenöl oder die Ester der genannten Öle. Bevorzugt sind Rapsöl, Sonnenblumenöl und deren Methyl- oder Ethylester.

Die Konzentration an Penetrationsförderer kann in den erfindungsgemäßen Mitteln in einem weiten Bereich variiert werden. Bei einem formulierten Pflanzenschutzmittel liegt sie im allgemeinen bei 1 bis 95 Gew.-%, bevorzugt bei 1 bis 55 Gew.-%, besonders bevorzugt bei 15 - 40 Gew.-%. In den anwendungsfertigen Mitteln (Spritzbrühen) liegen die Konzentration im allgemeinen zwischen 0,1 und 10 g/l, bevorzugt zwischen 0,5 und 5 g/l.

Erfindungsgemäße Pflanzenschutzmittel können auch weitere Komponente, beispielsweise Tenside bzw. Dispergierhilfsmittel oder Emulgatoren enthalten.

Als nicht-ionische Tenside bzw. Dispergierhilfsmittel kommen alle üblicherweise in agrochemischen Mitteln einsetzbaren Stoffe dieses Typs in Betracht. Vorzugsweise genannt seien Polyethylenoxid-polypropylenoxid-Blockcopolymere, Polyethylenglykolether von linearen Alkoholen, Umsetzungsprodukte von Fettsäuren mit Ethylenoxid und/oder Propylenoxid, ferner Polyvinylalkohol, Polyvinylpyrrolidon, Mischpolymerisate aus Polyvinylalkohol und Polyvinylpyrrolidon sowie Copolymerisate aus (Meth)acrylsäure und (Meth)acrylsäureestern, weiterhin Alkylethoxylate und Alkylarylethoxylate, die gegebenenfalls phosphatiert und gegebenenfalls mit Basen neutralisiert sein können, wobei Sorbitolethoxylate beispielhaft genannt seien, sowie Polyoxyalkylenamin-Derivate.

Als anionische Tenside kommen alle üblicherweise in agrochemischen Mitteln einsetzbaren Substanzen dieses Typs in Frage. Bevorzugt sind Alkalimetall- und Erdalkalimetall-Salze von Alkylsulfonsäuren oder Alkylarylsulfonsäuren.

Eine weitere bevorzugte Gruppe von anionischen Tensiden bzw. Dispergierhilfsmitteln sind in Pflanzenöl wenig lösliche Salze von Polystyrolsulfonsäuren, Salze von Polyvinylsulfonsäuren, Salze von Naphthalinsulfonsäure-Formaldehyd-Kondensationsprodukten, Salze von Kondensationsprodukten aus Naphthalinsulfonsäure, Phenolsulfonsäure und Formaldehyd sowie Salze von Ligninsulfonsäure.

Als Zusatzstoffe, die in den erfindungsgemäßen Formulierungen enthalten sein können, kommen Emulgatoren, schaumhemmende Mittel, Konservierungsmittel, Antioxydantien, Farbstoffe und inerte Füllmaterialien in Betracht.

Bevorzugte Emulgatoren sind ethoxylierte Nonylphenole, Umsetzungsprodukte von Alkylphenolen mit Ethylenoxid und/oder Propylenoxid, ethoxylierte Arylalkylphenole, weiterhin ethoxylierte und propoxylierte Arylalkylphenole, sowie sulfatierte oder phosphatierte Arylalkylethoxylate bzw. -ethoxy-propoxylate, wobei Sorbitan-Derivate, wie Polyethylenoxid-Sorbitan-Fettsäureester und Sorbitan-Fettsäureester, beispielhaft genannt seien.

Verwendet man gemäß Verfahren (A) 4-[(6-Methyl-3-(4-fluorphenyl)-phenyl)acetylamino]1-methoxy-piperidin-4-carbonsäuremethylester als Ausgangsstoff, so kann der Verlauf des erfindungsgemäßen Verfahrens durch folgendes Reaktionsschema wiedergegeben werden:

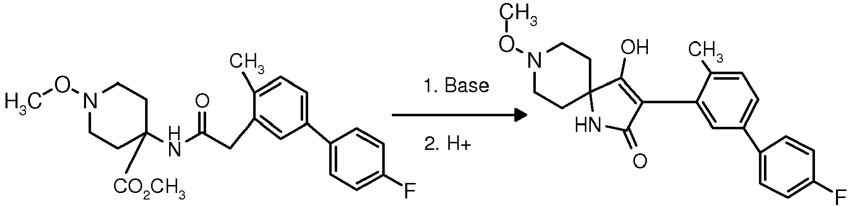

Verwendet man gemäß Verfahren (I) 3-[(6-Methyl-3-brom-phenyl)-4-hydroxy-8-methoxy-1,8-diaza-spiro[4.5]-dec-3-en-2-on und 4-Fluorphenylboronsäure als Ausgangsprodukte, so kann der Reaktionsverlauf durch folgendes Schema wiedergegeben werden:

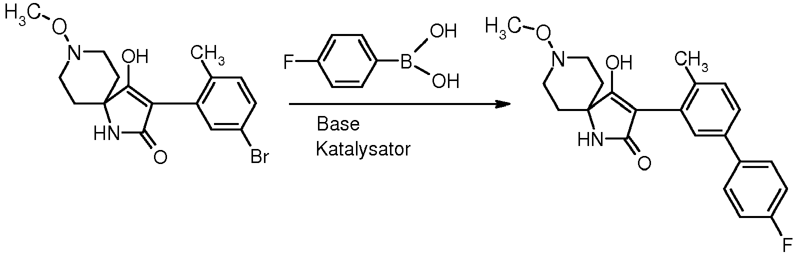

Die beim erfindungsgemäßen Verfahren (A) als Ausgangsstoffe benötigten Verbindungen der Formel (II) 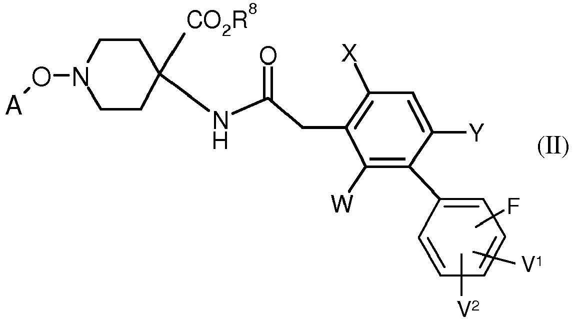 in welcher
A, W, X, Y, V¹ und V² die oben angegebenen Bedeutungen haben,
sind neu.

Man erhält Verbindungen der Formel (II) beispielsweise, wenn man Aminosäurederivate der Formel (XIV) 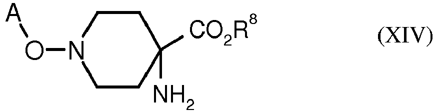 in welcher
A und R⁸ die oben angegebene Bedeutung haben,
mit substituierten Phenylessigsäurederivaten der Formel (XV) 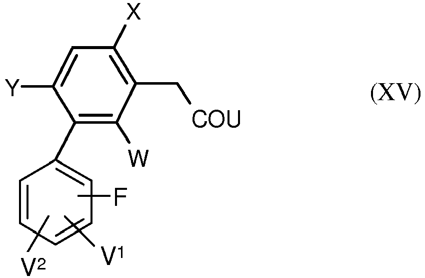 in welcher
- W, X, Y , V¹ und V²: die oben angegebenen Bedeutungen haben und
- U: für eine durch Carbonsäureaktivierungsreagenzien wie Carbonyldiimidazol, Carbonyldiimide (wie z.B. Dicyclohexylcarbondiimid), Phosphorylierungsreagenzien (wie z.B. POCl₃, BOP-Cl), Halogenierungsmittel wie z.B. Thionylchlorid, Oxalylchlorid, Phosgen oder Chlorameisensäureester eingeführte Abgangsgruppe steht,
acyliert (Chem. Reviews 52, 237-416 (1953); Bhattacharya, Indian J. Chem. 6, 341-5, 1968)
oder wenn man Verbindungen der Formel (XVI) 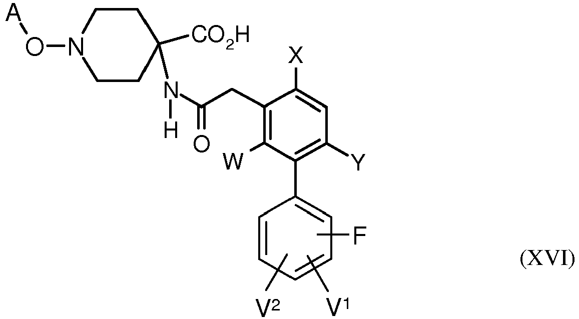 in welcher
- A, W, X, Y, V¹ und V²: die oben angegebenen Bedeutungen haben,
verestert (Chem. Ind. (London) 1568 (1968)).
Die Verbindungen der Formel (XVI) 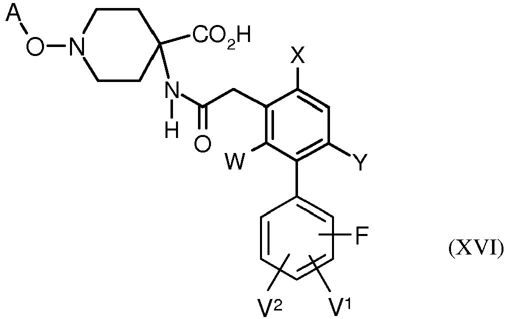 in welcher
- A, W, X, Y, V¹ und V²: die oben angegebenen Bedeutungen haben,
sind neu.

Man erhält die Verbindungen der Formel (XVI) beispielsweise, wenn man Verbindungen der Formel (XVII) 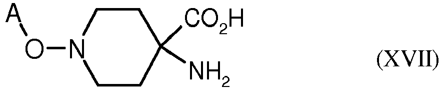 in welcher
A die oben angegebenen Bedeutung hat
mit substituierten Phenylessigsäurederivaten der Formel (XV) 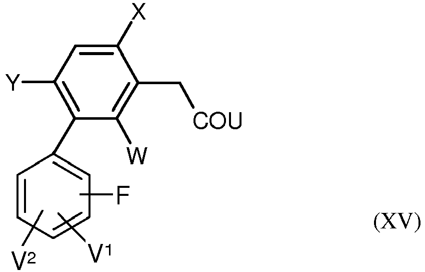 in welcher
U, W, X, Y, V¹ und V² die oben angegebenen Bedeutungen haben
z.B. nach Schotten-Baumann acyliert (Organikum, VEB Deutscher Verlag der Wissenschaften, Berlin 1977, S. 505).

Die Verbindungen der Formel (XV) sind teilweise bekannt und/oder lassen sich nach den bekannten Verfahren in den eingangs zitierten Offenlegungsschriften herstellen.

Die Verbindungen der Formel (XIV) und (XVII) sind aus WO 09/049851 bekannt.

Weiterhin lassen sich die bei dem obigen Verfahren (A) verwendeten Ausgangsstoffe der Formel (II) 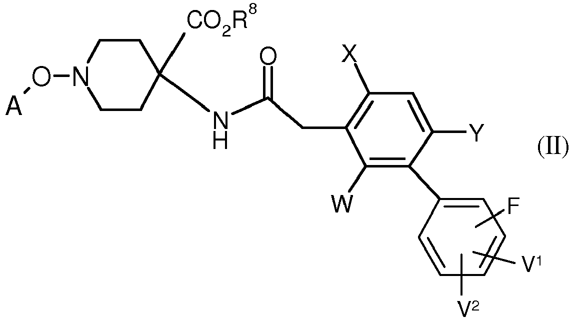 in welcher
A, W, X, Y, R⁸, V¹ und V²die oben angegebenen Bedeutungen haben,
herstellen, wenn man Verbindungen der Formel (XIX) 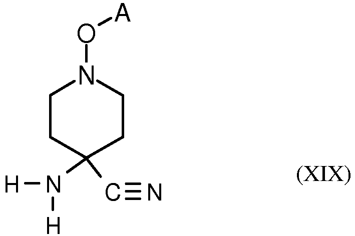 in welcher
A die oben angegebenen Bedeutung hat,
mit substituierten Phenylessigsäurederivaten der Formel (XV) 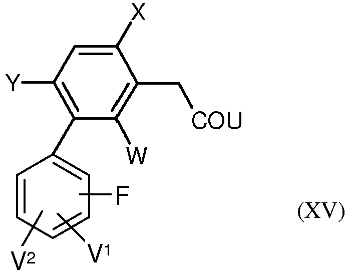 in welcher
U, W, X, Y, V¹ und V² die oben angegebenen Bedeutungen haben
zu Verbindungen der Formel (XX) 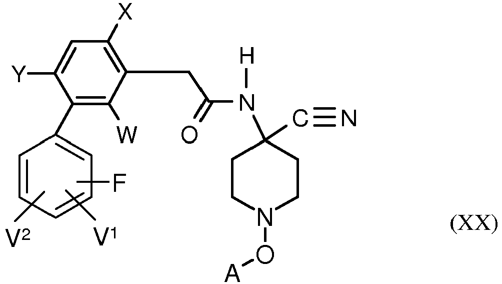 in welcher
A, W, X, Y V¹ und V² die in Anspruch 1 angegebenen Bedeutungen haben.
umsetzt,
und diese anschließend einer sauren Alkoholyse unterwirft.

Die Verbindungen der Formel (XX) sind ebenfalls neu und lassen sich nach bekannten Verfahren die in der eingangs zitierten Literatur beschrieben sind herstellen. Die Verbindungen der Formel (XIX) sind aus WO 09/049851 bekannt.

Die zur Durchführung des erfindungsgemäßen Verfahrens (I) außerdem als Ausgangsstoffe benötigten Boronsäuren der Formel (XIII) sind allgemein bekannte Verbindungen der organischen bzw. anorganischen Chemie.

Die Verbindungen der Formeln (XV) sind darüber hinaus aus den eingangs zitierten Patentanmeldungen bekannt und/oder lassen sich nach den dort angegebenen Methoden herstellen.

Die Verbindungen der Formeln (I-a') lassen sich nach den beschriebenen Verfahren A herstellen. Die Verbindungen der Formel (XIII) sind teilweise bekannt, teilweise käuflich oder lassen sich nach im Prinzip bekannten Verfahren herstellen.

Das Verfahren (A) ist dadurch gekennzeichnet, dass man Verbindungen der Formel (II), in welcher A, W, X, Y, Z und R⁸ die oben angegebenen Bedeutungen haben, in Gegenwart eines Verdünnungsmittels und in Gegenwart einer Base einer intramolekularen Kondensation unterwirft.

Als Verdünnungsmittel können bei dem erfindungsgemäßen Verfahren (A) alle gegenüber den Reaktionsteilnehmern inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon, sowie Alkohole wie Methanol, Ethanol, Propanol, Iso-Propanol, Butanol, Iso-Butanol und tert.-Butanol.

Als Base (Deprotonierungsmittel) können bei der Durchführung des erfindungsgemäßen Verfahrens (A) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (=Methyltrialkyl(C₈-C₁₀)ammoniumchlorid) oder TDA 1 (=Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natriummethylat, Natriumethylat und Kalium-tert.-butylat einsetzbar.

Die Reaktionstemperatur kann bei der Durchführung des erfindungsgemäßen Verfahrens (A) innerhalb eines größeren Bereiches variiert werden. Im allgemeinen arbeitet man bei Temperaturen zwischen -75°C und 200°C, vorzugsweise zwischen -50°C und 150°C. Das erfindungsgemäße Verfahren (A) wird im allgemeinen unter Normaldruck durchgeführt.

Bei der Durchführung des erfindungsgemäßen Verfahrens (A) setzt man die Reaktionskomponente der Formel (II) und die deprotonierende Base im allgemeinen in äquimolaren bis etwa doppeltäquimolaren Mengen ein. Es ist jedoch auch möglich, die eine oder andere Komponente in einem größeren Überschuss (bis zu 3 Mol) zu verwenden.

Zur Durchführung des erfindungsgemäßen Verfahrens (I) sind Palladium(0)-Komplexe als Katalysator geeignet. Bevorzugt wird beispielsweise Tetrakis-(triphenylphosphin)palladium. Gegebenenfalls können auch Palladium(II)-Verbindungen eingesetzt werden, beispielsweise PdCl₂, Pd(OAc)₂. Bei der Verwendung von Palladium(II)-Verbindungen werden in der Regel Phosphine als Komplexbildner wie beispielsweise Tricyclohexylphosphin eingesetzt.

Als Säureakzeptoren zur Durchführung des erfindungsgemäßen Verfahrens (I) kommen anorganische oder organische Basen in Frage. Hierzu gehören vorzugsweise Erdalkalimetall- oder Alkalimetallhydroxide, -acetate, -carbonate oder -hydrogencarbonate, wie beispielsweise Natrium-, Kalium-, Barium- oder Anmioniumhydroxid, Natrium-, Kalium-, Calcium- oder Ammoniumacetat, Natrium-, Kalium-, Cäsium- oder Ammoniumcarbonat, Natriumhydrogen- oder Kaliumhydrogencarbonat, Alkalifluoride, wie beispielsweise Cäsiumfluorid, Alkaliphosphate wie z.B. Kaliumdihydrogenphosphat, Kaliumphosphat sowie tertiäre Amine, wie Trimethylamin, Triethylamin, Tributylamin, N,N-Dimethylanilin, N,N-Dimethylbenzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Als Verdünnungsmittel zur Durchführung des erfindungsgemäßen Verfahrens (I) kommen Wasser, organische Lösungsmittel und beliebige Mischungen davon in Betracht. Beispielhaft seien genannt: aliphatische, alicyclische oder aromatische Kohlenwasserstoffe, wie beispielsweise Petrolether, Hexan, Heptan, Cyclohexan, Methylcyclohexan, Benzol, Toluol, Xylol oder Dicalin; halogenierte Kohlenwasserstoffe, wie beispielsweise Chlorbenzol, Dichlorbenzol, Methylenchlorid, Chloroform, Tetrachlormethan, Dichlor-, Trichlorethan oder Tetrachlorethylen; Ether, wie Diethyl-, Diisopropyl-, Methyl-t-butyl-, Methyl-t-amylether, Dioxan, Tetrahydrofuren, 1,2-Dimethoxyethan, 1,2-Diethoxyethan, Diethylenglykoldimethylether oder Anisol; Alkohole, wie Methanol, Ethanol, n- oder i-Propanol, n-, iso-, sek.- oder tert.-Butanol, Ethandiol, Propan-1,2-diol, Ethoxyethanol, Methoxyethanol, Diethylenglykolmonomethylether, Diethylglykolmonomethylether; Wasser.

Die Reaktionstemperatur kann bei dem erfindungsgemäßen Verfahren (I) innerhalb eines größeren Bereiches variiert werden. Im allgemeinen arbeitet man bei Temperaturen zwischen 0°C und +140°C, bevorzugt zwischen 50°C und +100°C.

Bei der Durchführung des erfindungsgemäßen Verfahrens (I) werden die Boronsäuren der Formel (XIII), in welcher Z die oben angegebene Bedeutung hat und Verbindungen der Formeln (I-a') bis (I-g'), in welchen A, G, W, X, Y und Z' die oben angegebene Bedeutung hat, im molaren Verhältnis 1:1 bis 3:1, vorzugsweise 1:1 bis 2:1 eingesetzt. Vom Katalysator setzt man im allgemeinen 0,005 bis 0,5 Mol, vorzugsweise 0,01 Mol bis 0,1 Mol pro Mol der Verbindungen der Formeln (I-a') bis (I-g') ein. Die Base setzt man im Allgemeinen in einem Überschuss ein. Die Aufarbeitung geschieht nach üblichen Methoden.

Die erfindungsgemäßen Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Forsten, in Gärten und Freizeiteinrichtungen, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:
Aus der Ordnung der Anoplura (Phthiraptera) z.B. Damalinia spp., Haematopinus spp., Linognathus spp., Pediculus spp., Trichodectes spp..

Aus der Klasse der Arachnida z.B. Acarus spp., Aceria sheldoni, Aculops spp., Aculus spp., Amblyomma spp., Amphitetranychus viennensis, Argas spp., Boophilus spp., Brevipalpus spp., Bryobia praetiosa, Chorioptes spp., Dermanyssus gallinae, Eotetranychus spp., Epitrimerus pyri, Eutetranychus spp., Eriophyes spp., Halotydeus destructor, Hemitarsonemus spp., Hyalomma spp., Ixodes spp., Latrodectus mactans, Metatetranychus spp., Nuphersa spp., Oligonychus spp., Ornithodoros spp., Panonychus spp., Phyllocoptruta oleivora, Polyphagotarsonemus latus, Psoroptes spp., Rhipicephalus spp., Rhizoglyphus spp., Sarcoptes spp., Scorpio maurus, Stenotarsonemus spp., Tarsonemus spp., Tetranychus spp., Vasates lycopersici.

Aus der Klasse der Bivalva z.B. Dreissena spp..

Aus der Ordnung der Chilopoda z.B. Geophilus spp., Scutigera spp..

Aus der Ordnung der Coleoptera z.B. Acalymma vittatum, Acanthoscelides obtectus, Adoretus spp., Agelastica alni, Agriotes spp., Amphimallon solstitialis, Anobium punctatum, Anoplophora spp., Anthonomus spp., Anthrenus spp., Apion spp., Apogonia spp., Atomaria spp., Attagenus spp., Bruchidius obtectus, Bruchus spp., Cassida spp., Cerotoma trifurcata, Ceutorrhynchus spp., Chaetocnema spp., Cleonus mendicus, Conoderus spp., Cosmopolites spp., Costelytra zealandica, Ctenicera spp., Curculio spp., Cryptorhynchus lapathi, Cylindrocopturus spp., Dermestes spp., Diabrotica spp., Dichocrocis spp., Diloboderus spp., Epilachna spp., Epitrix spp., Faustinus spp., Gibbium psylloides, Hellula undalis, Heteronychus arator, Heteronyx spp., Hylamorpha elegans, Hylotrupes bajulus, Hypera postica, Hypothenemus spp., Lachnosterna consanguinea, Lema spp., Leptinotarsa decemlineata, Leucoptera spp., Lissorhoptrus oryzophilus, Lixus spp., Luperodes spp., Lyctus spp., Megascelis spp., Melanotus spp., Meligethes aeneus, Melolontha spp., Migdolus spp., Monochamus spp., Naupactus xanthographus, Niptus hololeucus, Oryctes rhinoceros, Oryzaephilus surinamensis, Oryzaphagus oryzae, Otiorrhynchus spp., Oxycetonia jucunda, Phaedon cochleariae, Phyllophaga spp., Phyllotreta spp., Popillia japonica, Premnotrypes spp., Psylliodes spp., Ptinus spp., Rhizobius ventralis, Rhizopertha dominica, Sitophilus spp., Sphenophorus spp., Sternechus spp., Symphyletes spp., Tanymecus spp., Tenebrio molitor, Tribolium spp., Trogoderma spp., Tychius spp., Xylotrechus spp., Zabrus spp..

Aus der Ordnung der Collembola z.B. Onychiurus armatus.

Aus der Ordnung der Diplopoda z.B. Blaniulus guttulatus.

Aus der Ordnung der Diptera z.B. Aedes spp., Agromyza spp., Anastrepha spp., Anopheles spp., Asphondylia spp., Bactrocera spp., Bibio hortulanus, Calliphora erythrocephala, Ceratitis capitata, Chironomus spp., Chrysomyia spp., Cochliomyia spp., Contarinia spp., Cordylobia anthropophaga, Culex spp., Cuterebra spp., Dacus oleae, Dasyneura spp., Delia spp., Dermatobia hominis, Drosophila spp., Echinocnemus spp., Fannia spp., Gastrophilus spp., Hydrellia spp., Hylemyia spp., Hyppobosca spp., Hypoderma spp., Liriomyza spp.. Lucilia spp., Musca spp., Nezara spp., Oestrus spp., Oscinella frit, Pegomyia spp., Phorbia spp., Prodiplosis spp., Psila rosae, Rhagoletis spp., Stomoxys spp., Tabanus spp., Tannia spp., Tetanops spp., Tipula spp..

Aus der Klasse der Gastropoda z.B. Arion spp., Biomphalaria spp., Bulinus spp., Deroceras spp., Galba spp., Lymnaea spp., Oncomelania spp., Pomacea spp., Succinea spp..

Aus der Klasse der Helminthen z.B. Ancylostoma duodenale, Ancylostoma ceylanicum, Acylostoma braziliensis, Ancylostoma spp., Ascaris lubricoides, Ascaris spp., Brugia malayi, Brugia timori, Bunostomum spp., Chabertia spp., Clonorchis spp., Cooperia spp., Dicrocoelium spp, Dictyocaulus filaria, Diphyllobothrium latum, Dracunculus medinensis, Echinococcus granulosus, Echinococcus multilocularis, Enterobius vermicularis, Faciola spp., Haemonchus spp., Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp, Strongyloides fuelleborni, Strongyloides stercoralis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.

Weiterhin lassen sich Protozoen, wie Eimeria, bekämpfen.

Aus der Ordnung der Heteroptera z.B. Anasa tristis, Antestiopsis spp., Blissus spp., Calocoris spp., Campylomma livida, Cavelerius spp., Cimex spp., Collaria spp., Creontiades dilutus, Dasynus piperis, Dichelops furcatus, Diconocoris hewetti, Dysdercus spp., Euschistus spp., Eurygaster spp., Heliopeltis spp., Horcias nobilellus, Leptocorisa spp., Leptoglossus phyllopus, Lygus spp., Macropes excavatus, Miridae, Monalonion atratum, Nezara spp., Oebalus spp., Pentomidae, Piesma quadrata, Piezodorus spp., Psallus spp., Pseudacysta persea, Rhodnius spp., Sahlbergella singularis, Scaptocoris castanea, Scotinophora spp., Stephanitis nashi, Tibraca spp., Triatoma spp.

Aus der Ordnung der Homoptera z.B. Acyrthosipon spp., Acrogonia spp., Aeneolamia spp., Agonoscena spp., Aleurodes spp., Aleurolobus barodensis, Aleurothrixus spp., Amrasca spp., Anuraphis cardui, Aonidiella spp., Aphanostigma piri, Aphis spp., Arboridia apicalis, Aspidiella spp., Aspidiotus spp., Atanus spp., Aulacorthum solani, Bemisia spp., Brachycaudus helichrysii, Brachycolus spp., Brevicoryne brassicae, Calligypona marginata, Carneocephala fulgida, Ceratovacuna lanigera, Cercopidae, Ceroplastes spp., Chaetosiphon fragaefolii, Chionaspis tegalensis, Chlorita onukii, Chromaphis juglandicola, Chrysomphalus ficus, Cicadulina mbila, Coccomytilus halli, Coccus spp., Cryptomyzus ribis, Dalbulus spp., Dialeurodes spp., Diaphorina spp., Diaspis spp., Drosicha spp., Dysaphis spp., Dysmicoccus spp., Empoasca spp., Eriosoma spp., Erythroneura spp., Euscelis bilobatus, Ferrisia spp., Geococcus coffeae, Hieroglyphus spp., Homalodisca coagulata, Hyalopterus arundinis, Icerya spp., Idiocerus spp., Idioscopus spp., Laodelphax striatellus, Lecanium spp., Lepidosaphes spp., Lipaphis erysimi, Macrosiphum spp., Mahanarva spp., Melanaphis sacchari, Metcalfiella spp., Metopolophium dirhodum, Monellia costalis, Monelliopsis pecanis, Myzus spp., Nasonovia ribisnigri, Nephotettix spp., Nilaparvata lugens, Oncometopia spp., Orthezia praelonga, Parabemisia myricae, Paratrioza spp., Parlatoria spp., Pemphigus spp., Peregrinus maidis, Phenacoccus spp., Phloeomyzus passerinii, Phorodon humuli, Phylloxera spp., Pinnaspis aspidistrae, Planococcus spp., Protopulvinaria pyriformis, Pseudaulacaspis pentagona, Pseudococcus spp., Psylla spp., Pteromalus spp., Pyrilla spp., Quadraspidiotus spp., Quesada gigas, Rastrococcus spp., Rhopalosiphum spp., Saissetia spp., Scaphoides titanus, Schizaphis graminum, Selenaspidus articulatus, Sogata spp., Sogatella furcifera, Sogatodes spp., Stictocephala festina, Tenalaphara malayensis, Tinocallis caryaefoliae, Tomaspis spp., Toxoptera spp., Trialeurodes spp., Trioza spp., Typhlocyba spp., Unaspis spp., Viteus vitifolii, Zygina spp..

Aus der Ordnung der Hymenoptera z.B. Athalia spp., Diprion spp., Hoplocampa spp., Lasius spp., Monomorium pharaonis, Vespa spp..

Aus der Ordnung der Isopoda z.B. Armadillidium vulgare, Oniscus asellus, Porcellio scaber.

Aus der Ordnung der Isoptera z.B. Acromyrmex spp., Atta spp., Cornitermes cumulans, Microtermes obesi, Odontotermes spp., Reticulitermes spp,

Aus der Ordnung der Lepidoptera z.B. Acronicta major, Adoxophyes spp., Aedia leucomelas, Agrotis spp., Alabama spp., Amyelois transitella, Anarsia spp., Anticarsia spp., Argyroploce spp., Barathra brassicae, Borbo cinnara, Bucculatrix thurberiella, Bupalus piniarius, Busseola spp., Cacoecia spp., Caloptilia theivora, Capua reticulana, Carpocapsa pomonella, Carposina niponensis, Cheimatobia brumata, Chilo spp., Choristoneura spp., Clysia ambiguella, Cnaphalocerus spp., Cnephasia spp., Conopomorpha spp., Conotrachelus spp., Copitarsia spp., Cydia spp., Dalaca noctuides, Diaphania spp., Diatraea saccharalis, Earias spp., Ecdytolopha aurantium, Elasmopalpus lignosellus, Eldana saccharina, Ephestia kuehniella, Epinotia spp., Epiphyas postvittana, Etiella spp., Eulia spp., Eupoecilia ambiguella, Euproctis spp., Euxoa spp., Feltia spp., Galleria mellonella, Gracillaria spp., Grapholitha spp., Hedylepta spp., Helicoverpa spp., Heliothis spp., Hofmannophila pseudospretella, Homoeosoma spp., Homona spp., Hyponomeuta padella, Kakivoria flavofasciata, Laphygma spp., Laspeyresia molesta, Leucinodes orbonalis, Leucoptera spp., Lithocolletis spp., Lithophane antennata, Lobesia spp., Loxagrotis albicosta, Lymantria spp., Lyonetia spp., Malacosoma neustria, Maruca testulalis, Mamestra brassicae, Mocis spp., Mythimna separata, Nymphula spp., Oiketicus spp., Oria spp., Orthaga spp., Ostrinia spp., Oulema oryzae, Panolis flammea, Parnara spp., Pectinophora spp., Perileucoptera spp., Phthorimaea spp., Phyllocnistis citrella, Phyllonorycter spp., Pieris spp., Platynota stultana, Plusia spp., Plutella xylostella, Prays spp., Prodenia spp., Protoparce spp., Pseudaletia spp., Pseudoplusia includens, Pyrausta nubilalis, Rachiplusia nu, Schoenobius spp., Scirpophaga spp., Scotia segetum, Sesamia spp., Sparganothis spp., Spodoptera spp., Stathmopoda spp., Stomopteryx subsecivella, Synanthedon spp., Tecia solanivora, Thermesia gemmatalis, Tinea pellionella, Tineola bisselliella, Tortrix spp., Trichoplusia spp., Tuta absoluta, Virachola spp..

Aus der Ordnung der Orthoptera z.B. Acheta domesticus, Blatta orientalis, Blattella germanica, Dichroplus spp., Gryllotalpa spp., Leucophaea maderae, Locusta spp., Melanoplus spp., Periplaneta americana, Schistocerca gregaria.

Aus der Ordnung der Siphonaptera z.B. Ceratophyllus spp., Xenopsylla cheopis.

Aus der Ordnung der Symphyla z.B. Scutigerella spp..

Aus der Ordnung der Thysanoptera z.B. Anaphothrips obscurus, Baliothrips biformis, Drepanothris reuteri, Enneothrips flavens, Frankliniella spp., Heliothrips spp., Hercinothrips femoralis, Rhipiphorothrips cruentatus, Scirtothrips spp., Taeniothrips cardamoni, Thrips spp..

Aus der Ordnung der Thysanura z.B. Lepisma saccharina.

Zu den pflanzenparasitären Nematoden gehören z.B. Aphelenchoides spp., Bursaphelenchus spp., Ditylenchus spp., Globodera spp., Heterodera spp., Longidorus spp., Meloidogyne spp., Pratylenchus spp., Radopholus similis, Trichodorus spp., Tylenchulus semipenetrans, Xiphinema spp..

Die erfindungsgemäßen Verbindungen können in bestimmten Konzentrationen bzw. Aufwandmengen auch als Herbizide, Safener, Wachstumsregulatoren oder Mittel zur Verbesserung der Pflanzeneigenschaften, oder als Mikrobizide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich auch als Zwischen- oder Vorprodukte für die Synthese weiterer Wirkstoffe einsetzen.

Die Wirkstoffe können in die üblichen Formulierungen überführt werden, wie Lösungen, Emulsionen, Spritzpulver, wasser- und ölbasierte Suspensionen, Pulver, Stäubemittel, Pasten, lösliche Pulver, lösliche Granulate, Streugranulate, Suspensions-Emulsions-Konzentrate, Wirkstoff-imprägnierte Naturstoffe, Wirkstoff-imprägnierte synthetische Stoffe, Düngemittel sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Die Herstellung der Formulierungen erfolgt entweder in geeigneten Anlagen oder auch vor oder während der Anwendung.

Als Hilfsstoffe können solche Stoffe Verwendung finden, die geeignet sind, dem Mittel selbst oder und/oder davon abgeleitete Zubereitungen (z.B. Spritzbrühen, Saatgutbeizen) besondere Eigenschaften zu verleihen, wie bestimmte technische Eigenschaften und/oder auch besondere biologische Eigenschaften. Als typische Hilfsmittel kommen in Frage: Streckmittel, Lösemittel und Trägerstoffe.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (poly-)Ether, der einfachen und substituierten Amine, Amide, Lactame (wie N-Alkylpyrrolidone) und Lactone, der Sulfone und Sulfoxide (wie Dimethylsysulfoxid).

Im Falle der Benutzung von Wasser als Streckmittel können z.B. auch organische Lösemittel als Hilfslösungsmittel verwendet werden. Als flüssige Lösemittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylsulfoxid, sowie Wasser.

Erfindungsgemäß bedeutet Trägerstoff eine natürliche oder synthetische, organische oder anorganische Substanz, welcher fest oder flüssig sein kann, mit welchen die Wirkstoffe zur besseren Anwendbarkeit, insbesondere zum Aufbringen auf Pflanzen oder Pflanzenteile, gemischt oder verbunden sind. Der feste oder flüssige Trägerstoff ist im Allgemeinen inert und sollte in der Landwirtschaft verwendbar sein.

### Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Papier, Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage nicht-ionische und/oder ionische Stoffe, z.B. aus den Klassen der Alkohol-POE- und/oder POP-Ether, Säure- und/oder POP- POE-Ester, Alkyl-Aryl- und/oder POP- POE-Ether, Fett- und/oder POP- POE-Addukte, POE- und/oder POP-Polyol Derivate, POE- und/oder POP-Sorbitan- oder-Zucker-Addukte, Alky- oder Aryl-Sulfate, Sulfonate und Phosphate oder die entsprechenden PO-Ether-Addukte. Ferner geeignete Oligo- oder Polymere, z.B. ausgehend von vinylischen Monomeren, von Acrylsäure, aus EO und/oder PO allein oder in Verbindung mit z.B. (poly-) Alkoholen oder (poly-) Aminen. Ferner können Einsatz finden Lignin und seine Sulfonsäure-Derivate, einfache und modifizierte Cellulosen, aromatische und/oder aliphatische Sulfonsäuren sowie deren Addukte mit Formaldehyd.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Weitere Additive können Duftstoffe, mineralische oder vegetabile gegebenenfalls modifizierte Öle, Wachse und Nährstoffe (auch Spurennährstoffe), wie Salze von Eisen, Mangan, Bor, Kupfer, Kobalt, Molybdän und Zink sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel.

Die Formulierungen enthalten im allgemeinen zwischen 0,01 und 98 Gew.-% Wirkstoff, vorzugsweise zwischen 0,5 und 90%.

Die erfindungsgemäßen Wirkstoffe können als solche oder in ihren Formulierungen auch in Mischung mit einem oder mehreren geeigneten Fungiziden, Bakteriziden, Akariziden, Nematiziden, Insektiziden, Mikrobiziden, Düngemitteln, Lockstoffen, Sterilantien, Synergisten, Safenern, Semiochemicals und/oder Pflanzenwachstumsregulatoren verwendet werden, um so z.B. das Wirkungsspektrum zu verbreitern, die Wirkdauer zu verlängern, die Wirkgeschwindigkeit zu steigern, Repellenz zu verhindern oder Resistenzentwicklungen vorzubeugen. Des weiteren können solche Wirkstoffkombinationen das Pflanzenwachstum verbessern, die Toleranz gegenüber hohen oder niedrigen Temperaturen, gegen Trockenheit oder gegen Wasser- bzw. Bodensalzgehalt erhöhen, die Blühleistung steigern, die Ernte erleichtern und Ernteerträge steigern, die Reife beschleunigen, die Qualität und/oder den Ernährungswert der Ernteprodukte steigern, die Lagerfähigkeit verlängern und/oder die Bearbeitbarkeit der Ernteprodukte verbessern. Durch Kombination der erfindungsgemäßen Wirkstoffe mit Mischpartnern erhält man synergistische Effekte, d.h. die Wirksamkeit der jeweiligen Mischung ist größer als aufgrund der Wirksamkeiten der Einzelkomponenten zu erwarten war. Generell können die Kombinationen sowohl als Saatgutanwendungen als auch in Vor-, Tank- oder Fertigmischungen verwendet werden.

Jeder zusätzliche Wirkstoff kann in einem weiten Bereich, bevorzugt in einem Verhältnis von 100:1 bis 1.100, besonders bevorzugt von 5:1 bis1:5 mit den erfindungsgemäßen Wirkstoffen gemischt werden.

### Besonders günstige Mischungspartner sind z.B. die folgenden

### Insektizide / Akarizide / Nematizide:

Die hier mit ihrem "common name" genannten Wirkstoffe sind bekannt und beispielsweise im Pestizidhandbuch ("The Pesticide Manual" 14th Ed., British Crop Protection Council 2006) beschrieben oder im Internet recherchierbar (z.B. http://www.alanwood.net/pesticides).

### (1) Acetylcholinesterase (AChE) Inhibitoren, wie beispielsweise

Carbamate, z.B. Alanycarb, Aldicarb, Bendiocarb, Benfuracarb, Butocarboxim, Butoxycarboxim, Carbaryl, Carbofuran, Carbosulfan, Ethiofencarb, Fenobucarb, Formetanate, Furathiocarb, Isoprocarb, Methiocarb, Methomyl, Metolcarb, Oxamyl, Pirimicarb, Propoxur, Thiodicarb, Thiofanox, Triazamate, Trimethacarb, XMC und Xylylcarb; oder

Organophosphate, z.B. Acephate, Azamethiphos, Azinphos (-methyl, -ethyl), Cadusafos, Chlorethoxyfos, Chlorfenvinphos, Chlormephos, Chlorpyrifos (-methyl), Coumaphos, Cyanophos, Demeton-S-methyl, Diazinon, Dichlorvos/DDVP, Dicrotophos, Dimethoate, Dimethylvinphos, Disulfoton, EPN, Ethion, Ethoprophos, Famphur, Fenamiphos, Fenitrothion, Fenthion, Fosthiazate, Heptenophos, Isofenphos, Isopropyl *O*-(methoxyaminothio-phosphoryl) salicylat, Isoxathion, Malathion, Mecarbam, Methamidophos, Methidathion, Mevinphos, Monocrotophos, Naled, Omethoate, Oxydemeton-methyl, Parathion (-methyl), Phenthoate, Phorate, Phosalone, Phosmet, Phosphamidon, Phoxim, Pirimiphos (-methyl), Profenofos, Propetamphos, Prothiofos, Pyraclofos, Pyridaphenthion, Quinalphos, Sulfotep, Tebupirimfos, Temephos, Terbufos, Tetrachlorvinphos, Thiometon, Triazophos, Triclorfon und Vamidothion.

### (2) GABA-gesteuerte Chlorid-Kanal-Antagonisten, wie beispielsweise

Organochlorine, z.B. Chlordane und Endosulfan (alpha-); oder

Fiprole (Phenylpyrazole), z.B. Ethiprole, Fipronil, Pyrafluprole und Pyriprole.

### (3) Natrium-Kanal-Modulatoren / Spannungsabhängige Natrium-Kanal-Blocker, wie beispielsweise

Pyrethroide, z.B. Acrinathrin, Allethrin (d-cis-trans, d-trans), Bifenthrin, Bioallethrin, Bioallethrin-S-cyclopentenyl, Bioresmethrin, Cycloprothrin, Cyfluthrin (beta-), Cyhalothrin (gamma-, lambda-), Cypermethrin (alpha-, beta-, theta-, zeta-), Cyphenothrin [(1*R*)-*trans*-Isomere], Deltamethrin, Dimefluthrin, Empenthrin [(*EZ*)-(1*R*)-Isomere], Esfenvalerate, Etofenprox, Fenpropathrin, Fenvalerate, Flucythrinate, Flumethrin, Fluvalinate (tau-), Halfenprox, Imiprothrin, Metofluthrin, Permethrin, Phenothrin [(1*R*)-trans-Isomer], Prallethrin, Profluthrin, Pyrethrine (pyrethrum), Resmethrin, RU 15525, Silafluofen, Tefluthrin, Tetramethrin [(1*R*)- Isomere], Tralomethrin, Transfluthrin und ZXI 8901; oder

DDT; oder Methoxychlor.

### (4) Nikotinerge Acetylcholin-Rezeptor-Agonisten, wie beispielsweise

Neonikotinoide, z.B. Acetamiprid, Clothianidin, Dinotefuran, Imidacloprid, Nitenpyram,
Thiacloprid, Thiamethoxam; oder
Nikotin.

### (5) Allosterische Acetylcholin-Rezeptor-Modulatoren (Agonisten), wie beispielsweise

Spinosyne, z.B. Spinetoram und Spinosad.

### (6) Chlorid-Kanal-Aktivatoren, wie beispielsweise

Avermectine/Milbemycine, z.B. Abamectin, Emamectin-benzoate, Lepimectin und Milbemectin.

### (7) Juvenilhormon-Analoge, z.B. Hydroprene, Kinoprene, Methoprene; oder Fenoxycarb; Pyriproxyfen.

### (8) Wirkstoffe mit unbekannten oder nicht spezifischen Wirkmechanismen, wie beispielsweise

Begasungsmittel, z.B. Methylbromid und andere Alkylhalogenide; oder
Chloropicrin; Sulfurylfluorid; Borax; Brechweinstein.

### (9) Selektive Fraßhemmer, z.B. Pymetrozine; oder Flonicamid.

### (10) Milbenwachstumsinhibitoren, z.B. Clofentezine, Diflovidazin, Hexythiazox, Etoxazole.

### (11) Mikrobielle Disruptoren der Insektendarmmembran, wie beispielsweise Bacillus thuringiensis Subspezies israelensis, Bacillus sphaericus, Bacillus thuringiensis Subspezies aizawai, Bacillus thuringiensis Subspezies kurstaki, Bacillus thuringiensis Subspezies tenebrionis, und BT-Pflanzen-Proteine, z.B. Cry1Ab, Cry1Ac, Cry1Fa, Cry2Ab, mCry3A, Cry3Ab, Cry3Bb, Cry34/35Ab1.

### (12) Inhibitoren der oxidativen Phosphorylierung, ATP-Disruptoren, wie beispielsweise Diafenthiuron; oder

Organozinnverbindungen, z.B. Azocyclotin, Cyhexatin, Fenbutatin oxide; oder

Propargite; Tetradifon.

### (13) Entkoppler der oxidativen Phoshorylierung durch Unterbrechung des H-Protongradienten, wie beispielsweise Chlorfenapyr und DNOC.

### (14) Nikotinerge Acetylcholin-Rezeptor-Antagonisten, wie beispielsweise Bensultap, Cartap (-Hydrochlorid), Thiocyclam, und Thiosultap (-sodium).

### (15) Inhibitoren der Chitinbiosynthese, Typ 0, wie beispielsweise Benzoylharnstoffe, z.B. Bistrifluron, Chlorfluazuron, Diflubenzuron, Flucycloxuron, Flufenoxuron, Hexaflumuron, Lufenuron, Novaluron, Noviflumuron, Teflubenzuron und Triflumuron.

### (16) Inhibitoren der Chitinbiosynthese, Typ 1, wie beispielsweise Buprofezin.

### (17) Häutungsstörende Wirkstoffe, wie beispielsweise Cyromazine.

### (18) Ecdysonagonisten/-disruptoren, wie beispielsweise

Diacylhydrazine, z.B. Chromafenozide, Halofenozide, Methoxyfenozide und Tebufenozide.

### (19) Oktopaminerge Agonisten, wie beispielsweise Amitraz.

### (20) Komplex-III-Elektronentransportinhibitoren, wie beispielsweise Hydramethylnon; Acequinocyl; Fluacrypyrim.

### (21) Komplex-I-Elektronentransportinhibitoren, beispielsweise aus der Gruppe der METI-Akarizide, z.B. Fenazaquin, Fenpyroximate, Pyrimidifen, Pyridaben, Tebufenpyrad, Tolfenpyrad; oder

Rotenone (Derris).

### (22) Spannungsabhängige Natriumkanal-Blocker, z.B. Indoxacarb; Metaflumizone.

### (23) Inhibitoren der Acetyl-CoA-Carboxylase, wie beispielsweise Tetronsäure-Derivate, z.B.

Spirodiclofen und Spiromesifen; oder Tetramsäure-Derivate, z.B. Spirotetramat.

### (24) Komplex-IV-Elektronentransportinhibitoren, wie beispielsweise Phosphine, z.B.

Aluminiumphosphid, Kalziumphosphid, Phosphin, Zinkphosphid; oder Cyanid.

### (25) Komplex-II-Elektronentransportinhibitoren, wie beispielsweise Cyenopyrafen.

### (28) Ryanodinrezeptor-Effektoren, wie beispielsweise Diamide, z.B. Flubendiamide, Chlorantraniliprole (Rynaxypyr), Cyantraniliprole (Cyazypyr) sowie 3-Brom-N-{2-brom-4-chlor-6-[(1-cyclopropylethyl)carbamoyl]phenyl}-1-(3-chlorpyridin-2-yl)-1H-pyrazol-5-carboxamid (bekannt aus WO2005/077934) oder Methyl-2-[3,5-dibrom-2-({[3-brom-1-(3-chlorpyridin-2-yl)-1H-pyrazol-5-yl]carbonyl}amino)benzoyl]-1,2-dimethylhydrazincarboxylat (bekannt aus WO2007/043677).

Weitere Wirkstoffe mit unbekanntem Wirkmechanismus, wie beispielsweise Azadirachtin, Amidoflumet, Benzoximate, Bifenazate, Chinomethionat, Cryolite, Cyflumetofen, Dicofol, Fluensulfone (5-chloro-2-[(3,4,4-trifluorobut-3-en-1-yl)sulfonyl]-1,3-thiazole), Flufenerim, Fluopyram, Pyridalyl und Pyrifluquinazon; desweiteren Präparate auf Basis von Bacillus firmus (I-1582, BioNeem, Votivo) sowie folgende bekannte wirksame Verbindungen
4-{[(6-Brompyrid-3-yl)methyl](2-fluorethyl)amino}furan-2(5H)-on (bekannt aus WO 2007/115644), 4-{[(6-Fluorpyrid-3-yl)methyl](2,2-difluorethyl)amino}furan-2(5H)-on (bekannt aus WO 2007/115644), 4-{[(2-Chlor-1,3-thiazol-5-yl)methyl](2-fluorethyl)amino}furan-2(5H)-on (bekannt aus WO 2007/115644), 4-{[(6-Chlorpyrid-3-yl)methyl](2-fluorethyl)amino}furan-2(5H)-on (bekannt aus WO 2007/ 115644), 4-{[(6-Chlorpyrid-3-yl)methyl](2,2-difluorethyl)amino}furan-2(5H)-on (bekannt aus WO 2007/115644), 4-{[(6-Chlor-5-fluorpyrid-3-yl)methyl](methyl)amino}furan-2(5H)-on (bekannt aus WO 2007/115643), 4-{[(5,6-Dichlorpyrid-3-yl)methyl](2-fluorethyl)amino}furan-2(5H)-on (bekannt aus WO 2007/115646), 4-{[(6-Chlor-5-fluorpyrid-3-yl)methyl](cyclopropyl)amino}furan-2(5H)-on (bekannt aus WO 2007/115643), 4-{[(6-Chlorpyrid-3-yl)methyl](cyclopropyl)amino}furan-2(5H)-on (bekannt aus EP-A-0 539 588), 4-{[(6-Chlorpyrid-3-yl)methyl](methyl)amino}furan-2(5H)-on (bekannt aus EP-A-0 539 588), [(6-Chlorpyridin-3-yl)methyl](methyl)oxido-λ⁴-sulfanylidencyanamid (bekannt aus WO 2007/149134), [1-(6-Chlorpyridin-3-yl)ethyl](methyl)oxido-λ⁴-sulfanylidencyanamid (bekannt aus WO 2007/149134) und seine Diastereomere (A) und (B) 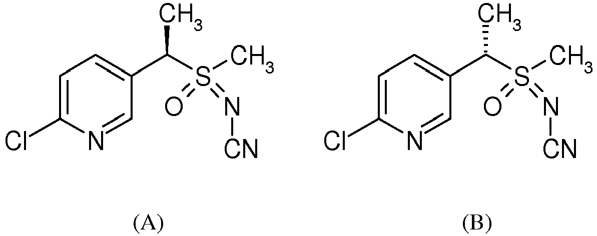 (ebenfalls bekannt aus WO 2007/149134), [(6-Trifluormethylpyridin-3-yl)methyl](methyl)oxido-λ⁴-sulfanylidencyanamid (bekannt aus WO 2007/095229), Sulfoxaflor
(ebenfalls bekannt aus WO 2007/149134), 11-(4-Chlor-2,6-dimethylphenyl)-12-hydroxy-1,4-dioxa-9-azadispiro[4.2.4.2]tetradec-11-en-10-on (bekannt aus WO 2006/089633), 3-(4'-Fluor-2,4-dimethylbiphenyl-3-yl)-4-hydroxy-8-oxa-1-azaspiro[4.5]dec-3-en-2-on (bekannt aus WO 2008/067911),
1-[2-fluoro-4-methyl-5-[(2,2,2-trifluoroethyl)sulfinyl]phenyl]-3-(trifluoromethyl)-1H-1,2,4-Triazol-5-amine (bekannt aus WO 2006/043635),
[(3S,4aR,12R,12aS,12bS)-3-[(Cyclopropylcarbonyl)oxy]-6,12-dihydroxy-4,12b-dimethyl-11-oxo-9-(pyridin-3-yl)-1,3,4,4a,5,6,6a,12,12a,12b-decahydro-2H,11H-benzo[f]pyrano[4,3-b]chromen-4-yl]methylcyclopropancarboxylat (bekannt aus WO 2006/129714),
2-Cyan-3-(difluormethoxy)-N,N-dimethylbenzolsulfonamid (bekannt aus WO2006/056433), 2-Cyan-3-(difluormethoxy)-N-methylbenzolsulfonamid (bekannt aus WO2006/100288), 2-Cyan-3-(difluormethoxy)-N-ethylbenzolsulfonamid (bekannt aus WO2005/035486), 4-(Difluormethoxy)-N-ethyl-N-methyl-1,2-benzothiazol-3-amin-1,1-dioxid (bekannt aus WO2007/057407) und N-[1-(2,3-Dimethylphenyl)-2-(3,5-dimethylphenyl)ethyl]-4,5-dihydro-1,3-thiazol-2-amin (bekannt aus WO2008/104503).

In einer bevorzugten Ausführungsform der Erfindung wird den Pflanzenschutzmitteln zur Wirkungssteigerung zusätzlich ein Penetrationsförderer zugegeben. Als Penetrationsförderer kommen beispielsweise auch Substanzen in Betracht, die die Verfügbarkeit der Verbindungen der Formel (I) im Spritzbelag fördern. Dazu gehören beispielsweise mineralische oder vegetabile Öle. Als Öle kommen alle üblicherweise in agrochemischen Mitteln einsetzbaren mineralischen oder vegetabilen - gegebenenfalls modifizierte - Öle in Frage. Beispielhaft genannt seien Sonnenblumenöl, Rapsöl, Olivenöl, Rizinusöl, Rüböl, Maiskernöl, Baumwollsaatöl und Sojabohnenöl oder die Ester der genannten Öle. Bevorzugt sind Rapsöl, Sonnenblumenöl und deren Methyl- oder Ethylester, insbesondere Rapsölmethylester.

Die Konzentration an Penetrationsförderer kann in den erfindungsgemäßen Mitteln in einem weiten Bereich variiert werden. Bei einem formulierten Pflanzenschutzmittel liegt sie im allgemeinen bei 1 bis 95 Gew.-%, bevorzugt bei 1 bis 55 Gew.-%, besonders bevorzugt bei 15 - 40 Gew.-%. In den anwendungsfertigen Mitteln (Spritzbrühen) liegen die Konzentration im allgemeinen zwischen 0,1 und 10 g/l, bevorzugt zwischen 0,5 und 5 g/l.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Die erfindungsgemäßen Wirkstoffe können ferner beim Einsatz als Insektizide in ihren handelsüblichen Formulierungen sowie in den aus diesen Formulierungen bereiteten Anwendungsformen in Mischungen mit Hemmstoffen vorliegen, die einen Abbau des Wirkstoffes nach Anwendung in der Umgebung der Pflanze, auf der Oberfläche von Pflanzenteilen oder in pflanzlichen Geweben vermindern.

Der Wirkstoffgehalt der aus den handelsüblichen Formulierungen bereiteten Anwendungsformen kann in weiten Bereichen variieren. Die Wirkstoffkonzentration der Anwendungsformen kann von 0,00000001 bis zu 95 Gew.-% Wirkstoff, vorzugsweise zwischen 0,00001 und 1 Gew.-% liegen.

Die Anwendung geschieht in einer den Anwendungsformen angepaßten üblichen Weise.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Beispielhaft seien die wichtigen Kulturpflanzen, wie Getreide (Weizen, Reis), Mais, Soja, Kartoffel, Zuckerrüben, Tomaten, Erbsen und andere Gemüsesorten, Baumwolle, Tabak, Raps, sowie Obstpflanzen (mit den Früchten Äpfel, Birnen, Zitrusfrüchten und Weintrauben) genannt. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Saatgut sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Saatgut.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Die Begriffe "Teile" bzw. "Teile von Pflanzen" oder "Pflanzenteile" wurden oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Zuckerrüben, Tomaten, Erbsen und andere Gemüsesorten, Baumwolle, Tabak, Raps, sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid-tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid- resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel (I) bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ekto- und Endoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:
Aus der Ordnung der Anoplurida z.B. Haematopinus spp., Linognathus spp., Pediculus spp., Phtirus spp., Solenopotes spp..

Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..

Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..

Aus der Ordnung der Siphonapterida z.B. Pulex spp., Ctenocephalides spp. (Ctenocephalides canis, Ctenocephalides felis), Xenopsylla spp., Ceratophyllus spp..

Aus der Ordnung der Heteropterida z.B. Cimex spp., Triatoma spp., Rhodnius spp., Panstrongylus spp..

Aus der Ordnung der Blattarida z.B. Blatta orientalis, Periplaneta americana, Blattela germanica, Supella spp..

Aus der Unterklasse der Acari (Acarina) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp..

Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor und bei der Tierhaltung in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen eine hohe insektizide Wirkung gegen Insekten zeigen, die technische Materialien zerstören.

Beispielhaft und vorzugsweise - ohne jedoch zu limitieren - seien die folgenden Insekten genannt:
Käfer wie Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus;

Hautflügler wie Sirex juvencus, Urocerus gigas, Urocerus gigas taignus, Urocerus augur;

Termiten wie Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus;

Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Die anwendungsfertigen Mittel können gegebenenfalls noch weitere Insektizide und gegebenenfalls noch ein oder mehrere Fungizide enthalten.

Hinsichtlich möglicher zusätzlicher Zumischpartner sei auf die oben genannten Insektizide und Fungizide verwiesen.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Weiter können die erfindungsgemäßen Verbindungen allein oder in Kombinationen mit anderen Wirkstoffen als Antifouling-Mittel eingesetzt werden.

Die Wirkstoffe eignen sich auch zur Bekämpfung von tierischen Schädlingen im Haushalts-, Hygiene- und Vorratsschutz, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:
Aus der Ordnung der Scorpionidea z.B. Buthus occitanus.

Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.

Aus der Ordnung der Araneae z.B. Aviculariidae, Araneidae.

Aus der Ordnung der Opiliones z.B. Pseudoscorpiones chelifer, Pseudoscorpiones cheiridium, Opiliones phalangium.

Aus der Ordnung der Isopoda z.B. Oniscus asellus, Porcellio scaber.

Aus der Ordnung der Diplopoda z.B. Blaniulus guttulatus, Polydesmus spp..

Aus der Ordnung der Chilopoda z.B. Geophilus spp..

Aus der Ordnung der Zygentoma z.B. Ctenolepisma spp., Lepisma saccharina, Lepismodes inquilinus.

Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.

Aus der Ordnung der Saltatoria z.B. Acheta domesticus.

Aus der Ordnung der Dermaptera z.B. Forficula auricularia.

Aus der Ordnung der Isoptera z.B. Kalotermes spp., Reticulitermes spp.

Aus der Ordnung der Psocoptera z.B. Lepinatus spp., Liposcelis spp.

Aus der Ordnung der Coleoptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.

Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.

Aus der Ordnung der Lepidoptera z.B. Achroia grisella, Galleria mellonella, Plodia interpunctella, Tinea cloacella, Tinea pellionella, Tineola bisselliella.

Aus der Ordnung der Siphonaptera z.B. Ctenocephalides canis, Ctenocephalides felis, Pulex irritans, Tunga penetrans, Xenopsylla cheopis.

Aus der Ordnung der Hymenoptera z.B. Camponotus herculeanus, Lasius fuliginosus, Lasius niger, Lasius umbratus, Monomorium pharaonis, Paravespula spp., Tetramorium caespitum.

Aus der Ordnung der Anoplura z.B. Pediculus humanus capitis, Pediculus humanus corporis, Pemphigus spp., Phylloera vastatrix, Phthirus pubis.

Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Neonicotinoiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

Die erfindungsgemäßen Verbindungen der Formel (I) (Wirkstoffe) weisen eine ausgezeichnete herbizide Wirksamkeit gegen ein breites Spektrum wirtschaftlich wichtiger mono- und dikotyler annueller Schadpflanzen auf. Auch schwer bekämpfbare perennierende Schadpflanzen, die aus Rhizomen, Wurzelstöcken oder anderen Dauerorganen austreiben, werden durch die Wirkstoffe gut erfaßt.

Die angewandte Wirkstoffmenge kann in einem größeren Bereich schwanken. Sie hängt im wesentlichen von der Art des gewünschten Effektes ab. Im Allgemeinen liegen die Aufwandmengen zwischen 1 g und 10 kg Wirkstoff pro Hektar Bodenfläche, vorzugsweise zwischen 5 g und 5 kg pro ha.

Der vorteilhafte Effekt der Kulturpflanzen-Verträglichkeit der erfindungsgemäßen Wirkstoffkombinationen ist bei bestimmten Konzentrationsverhältnissen besonders stark ausgeprägt. Jedoch können die Gewichtsverhältnisse der Wirkstoffe in den Wirkstoffkombinationen in relativ großen Bereichen variiert werden. Im allgemeinen entfallen auf 1 Gewichtsteil Wirkstoff der Formel (I) 0,001 bis 1000 Gewichtsteile, vorzugsweise 0,01 bis 100 Gewichtsteile, besonders bevorzugt 0,05 bis 20 Gewichtsteile einer der oben unter (b') genannten, die Kulturpflanzen Verträglichkeit verbessernden Verbindungen (Antidots/Safener).

Die erfindungsgemäßen Wirkstoffkombinationen werden im allgemeinen in Form von Fertigformulierungen zur Anwendung gebracht. Die in den Wirkstoffkombinationen enthaltenen Wirkstoffe können aber auch in Einzelformulierungen bei der Anwendung gemischt, d.h. in Form von Tankmischungen zur Anwendung gebracht werden.

Für bestimmte Anwendungszwecke, insbesondere im Nachauflauf-Verfahren, kann es ferner vorteilhaft sein, in die Formulierungen als weitere Zusatzstoffe pflanzenverträgliche mineralische oder vegetabilische Öle (z.B. das Handelspräparat "Rako Binol") oder Ammoniumsalze wie z.B. Ammoniumsulfat oder Ammoniumrhodanid aufzunehmen.

Die neuen Wirkstoffkombinationen können als solche, in Form ihrer Formulierungen oder der daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Stäuben oder Streuen.

Die Aufwandmengen der erfindungsgemäßen Wirkstoffkombinationen können in einem gewissen Bereich variiert werden; sie hängen u.a. vom Wetter und von den Bodenfaktoren ab. Im allgemeinen liegen die Aufwandmengen zwischen 0,001 und 5 kg pro ha, vorzugsweise zwischen 0,005 und 2 kg pro ha, besonders bevorzugt zwischen 0,01 und 0,5 kg pro ha.

Die erfindungsgemäß einzusetzenden Safener können je nach ihren Eigenschaften zur Vorbehandlung des Saatgutes der Kulturpflanze (Beizung der Samen) verwendet werden oder vor der Saat in die Saatfurchen eingebracht oder vor dem Herbizid separat angewendet werden oder zusammen mit dem Herbizid vor oder nach dem Ablaufen der Pflanzen angewendet werden.

Als Beispiele der Pflanzen werden die wichtigen Kulturpflanzen, wie Getreide (Weizen, Gerste, Reis), Mais, Soja, Kartoffel, Baumwolle, Raps, Rüben, Zuckerrohr sowie Obstpflanzen (mit den Früchten Äpfel, Birnen, Zitrusfrüchten und Weintrauben) erwähnt, wobei Getreide, Mais, Soja, Kartoffel, Baumwolle und Raps besonders hervorgehoben werden.

Mit den erfindungsgemäßen Wirkstoffen können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Saatgut sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Saatgut.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Gegenstand der vorliegenden Erfindung ist daher auch ein Verfahren zur Bekämpfung von unerwünschten Pflanzen oder zur Wachstumsregulierung von Pflanzen, vorzugsweise in Pflanzenkulturen, worin eine oder mehrere erfindungsgemäße Verbindung(en) auf die Pflanzen (z.B. Schadpflanzen wie mono- oder dikotyle Unkräuter oder unerwünschte Kulturpflanzen), das Saatgut (z.B. Körner, Samen oder vegetative Vermehrungsorgane wie Knollen oder Sprossteile mit Knospen) oder die Fläche, auf der die Pflanzen wachsen (z.B. die Anbaufläche), ausgebracht werden. Dabei können die erfindungsgemäßen Verbindungen z.B. im Vorsaat- (ggf. auch durch Einarbeitung in den Boden), Vorauflauf- oder Nachauflaufverfahren ausgebracht werden. Im einzelnen seien beispielhaft einige Vertreter der mono- und dikotylen Unkrautflora genannt, die durch die die erfindungsgemäßen Verbindungen kontrolliert werden können, ohne dass durch die Nennung eine Beschränkung auf bestimmte Arten erfolgen soll.

Monokotyle Schadpflanzen der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.

Dikotyle Unkräuter der Gattungen: Abutilon, Amaranthus, Ambrosia, Anoda, Anthemis, Aphanes, Artemisia, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

Die aufgeführten Pflanzen können besonders vorteilhaft erfindungsgemäß mit den Verbindungen der allgemeinen Formel I bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt nach der Behandlung Wachstumsstop ein und die Schadpflanzen bleiben in dem zum Applikationszeitpunkt vorhandenen Wachstumsstadium stehen oder sterben nach einer gewissen Zeit ganz ab, so dass auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen z.B. dikotyler Kulturen der Gattungen Arachis, Beta, Brassica, Cucumis, Cucurbita, Helianthus, Daucus, Glycine, Gossypium, Ipomoea, Lactuca, Linum, Lycopersicon, Miscanthus, Nicotiana, Phaseolus, Pisum, Solanum, Vicia, oder monokotyler Kulturen der Gattungen Allium, Ananas, Asparagus, Avena, Hordeum, Oryza, Panicum, Saccharum, Secale, Sorghum, Triticale, Triticum, Zea, abhängig von der Struktur der jeweiligen erfindungsgemäßen Verbindung und deren Aufwandmenge nur unwesentlich oder gar nicht geschädigt. Die vorliegenden Verbindungen eignen sich aus diesen Gründen sehr gut zur selektiven Bekämpfung von unerwünschtem Pflanzenwuchs in Pflanzenkulturen wie landwirtschaftlichen Nutzpflanzungen oder Zierpflanzungen.

Darüberhinaus weisen die erfindungsgemäßen Verbindungen (abhängig von ihrer jeweiligen Struktur und der ausgebrachten Aufwandmenge) hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da beispielsweise die Lagerbildung hierdurch verringert oder völlig verhindert werden kann.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Die Begriffe "Teile" bzw. "Teile von Pflanzen" oder "Pflanzenteile" wurden oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Aufgrund ihrer herbiziden und pflanzenwachstumsregulatorischen Eigenschaften können die Wirkstoffe auch zur Bekämpfung von Schadpflanzen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten, Nematoden oder Mikroorganismen wie Pilzen, Bakterien oder Viren. Andere besondere Eigenschaften betreffen z.B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt. Weitere besondere Eigenschaften können in einer Toleranz oder Resistenz gegen abiotische Stressoren z.B. Hitze, Kälte, Trockenheit, Salz und ultraviolette Strahlung liegen. Die Wirkstoffe können auch in transgenen Pflanzen, die sich durch höhere Erträge , z. B. durch eine verbesserte Photosyntheseleistung oder verbesserte Nährstoffaufnahme auszeichnen, eingesetzt werden.

Bevorzugt ist die Anwendung der erfindungsgemäßen Verbindungen der Formel (I) oder deren Salze in wirtschaftlich bedeutenden transgenen Kulturen von Nutz-und Zierpflanzen, z.B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten.

Vorzugsweise können die Verbindungen der Formel (I) als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z.B. EP 0221044, EP 0131624). Beschrieben wurden beispielsweise in mehreren Fällen
- gentechnische Veränderungen von Kulturpflanzen zwecks Modifikation der in den Pflanzen synthetisierten Stärke (z.B. WO 92/011376 A, WO 92/014827 A, WO 91/019806 A),
- transgene Kulturpflanzen, welche gegen bestimmte Herbizide vom Typ Glufosinate (vgl. z.B. EP 0242236 A, EP 0242246 A) oder Glyphosate (WO 92/000377 A) oder der Sulfonylharnstoffe (EP 0257993 A, US 5,013,659) oder gegen Kombinationen oder Mischungen dieser Herbizide durch "gene stacking" resistent sind, wie transgenen Kulturpflanzen z. B. Mais oder Soja mit dem Handelsnamen oder der Bezeichnung Optimum™ GAT™ (Glyphosate ALS Tolerant). Des weiteren wurden transgene Pflanzen beschrieben, die gegen synthetische Auxine (z. B 2,4 D) HRAC mode of action Class O und Aryloxy-phenoxy Propionate (fops, HRAC, Class A) resistent sind (DHT, Dow Agroscience Herbicide Tolerance Trait)
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP 0142924 A, EP 0193259 A).
- transgene Kulturpflanzen mit modifizierter Fettsäurezusammensetzung (WO 91/013972 A).
- Gentechnisch veränderte Pflanzen, die neue Insektenresistenzen, z. B. basierend auf der Expression von Toxinen aus Photorhabdus, Xenorhabdus Symbionten aus entomopathogenen Nematoden und Toxinen aus Spinnen, Skorpionen, Ameisen, parasitischen Wespen aufweisen.
- gentechnisch veränderte Kulturpflanzen mit neuen Inhalts- oder Sekundärstoffen z. B. neuen Phytoalexinen, die eine erhöhte Krankheitsresistenz verursachen (EP 0309862 A, EP 0464461 A)
- gentechnisch veränderte Pflanzen mit reduzierter Photorespiration, die höhere Erträge und höhere Stresstoleranz aufweisen (EP 0305398 A)
- transgene Kulturpflanzen, die pharmazeutisch oder diagnostisch wichtige Proteine produzieren ("molecular pharming")
- transgene Kulturpflanzen, die sich durch höhere Erträge oder bessere Qualitat auszeichnen
- Transgene Kulturpflanzen, die sich durch erhöhte Toleranzen gegen abiotische und biotische Stressoren auszeichnen
- transgene Kulturpflanzen die sich durch eine Kombinationen z.B. der o. g. neuen Eigenschaften auszeichnen ("gene stacking")

Zahlreiche molekularbiologische Techniken, mit denen neue transgene Pflanzen mit veränderten Eigenschaften hergestellt werden können, sind im Prinzip bekannt; siehe z.B. I. Potrykus und G. Spangenberg (eds.) Gene Transfer to Plants, Springer Lab Manual (1995), Springer Verlag Berlin, Heidelberg. oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe von Standardverfahren können z.B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden, siehe z.B. Sambrook et al., 1989, Molecular Cloning, A Laboratory Manual, 2. Aufl. Cold Spring Harbor Laboratory Press, Cold Spring Harbor, NY; oder Winnacker "Gene und Klone", VCH Weinheim 2. Auflage 1996

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet.

Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z.B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227; Wolter et al., Proc. Natl. Acad. Sci. USA 85 (1988), 846-850; Sonnewald et al., Plant J. 1 (1991), 95-106). Die Expression der Nukleinsäuremoleküle kann auch in den Organellen der Pflanzenzellen stattfinden.

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h., sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen (I) in transgenen Kulturen eingesetzt werden, welche gegen Wuchsstoffe, wie z.B. 2,4 D, Dicamba oder gegen Herbizide, die essentielle Pflanzenenzyme, z.B. Acetyl CoA Carboxylasen, Acetolactatsynthasen (ALS), EPSP Synthasen, Glutaminsynthasen (GS) oder Hydoxyphenylpyruvat Dioxygenasen (HPPD) hemmen, respektive gegen Herbizide aus der Gruppe der FOPs, Sulfonylharnstoffe, der Glyphosate, Glufosinate oder Benzoylisoxazole und analogen Wirkstoffe, oder gegen beliebige Kombinationen dieser Wirkstoffe, resistent sind.
- Besonders bevorzugt können die erfindungsgemäßen Verbindungen in transgenen Kulturpflanzen eingesetzt werden, die gegen eine Kombination von Glyphosaten und Glufosinaten, Glyphosaten und Sulfonylharnstoffen oder Imidazolinonen resistent sind. Ganz besonders bevorzugt können die erfindungsgemäßen Verbindungen in transgenen Kulturpflanzen wie z. B. Mais oder Soja mit dem Handelsnamen oder der Bezeichnung Optimum™ GAT™ (Glyphosate ALS Tolerant) eingesetzt werden. Des weiteren und besonders bevorzugt können die erfindungsgemäßen Verbindungen in transgenen Pflanzen, die gegen synthetische Auxine (z. B 2,4 D) mit "HRAC mode of action Class O" und Aryloxy-phenoxy Propionate (fops) mit "HRAC mode of action Class A" resistent sind (z. B. DHT, Dow Agroscience Herbicide Tolerance Trait) eingesetzt werden.

Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen der Formel (I) als Herbizide zur Bekämpfung von Schadpflanzen in transgenen Kulturpflanzen.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, welche die erfindungsgemäßen Verbindungen enthalten.

Die erfindungsgemäßen Verbindungen können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse.

Diese einzelnen Formulierungstypen sind im Prinzip bekannt und werden beispielsweise beschrieben in: Winnacker-Küchler, "Chemische Technologie", Band 7, C. Hanser Verlag München, 4. Aufl. 1986; Wade van Valkenburg, "Pesticide Formulations", Marcel Dekker, N.Y., 1973; K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hanser Verlag München, 4. Aufl. 1986.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix.

Als Kombinationspartner für die erfindungsgemäßen Verbindungen in Mischungsformulierungen oder im Tank-Mix sind beispielsweise bekannte Wirkstoffe, die auf einer Inhibition von beispielsweise Acetolactat-Synthase, Acetyl-CoA-Carboxylase, Cellulose-Synthase, Enolpyruvylshikimat-3-phosphat-Synthase, Glutamin-Synthetase, p-Hydroxyphenylpyruvat-Dioxygenase, Phytoendesaturase, Photosystem I, Photosystem II, Protoporphyrinogen-Oxidase beruhen, einsetzbar, wie sie z.B. in Weed Research 26 (1986) 441-445 oder in "The Pesticide Manual", 13th Auflage, The British Crop Protection Council and the Royal Soc. of Chemistry, 2003 und der darin zitierten Literatur beschrieben sind.

Als bekannte Herbizide oder Pflanzenwachstumsregulatoren, die mit den erfindungsgemäßen Verbindungen kombiniert werden können, sind z.B. folgende Wirkstoffe zu nennen:
Acetochlor, Acibenzolar, Acibenzolar-S-methyl, Acifluorfen, Acifluorfen-sodium, Aclonifen, Alachlor, Allidochlor, Alloxydim, Alloxydim-sodium, Ametryn, Amicarbazone, Amidochlor, Amidosulfuron, Aminopyralid, Amitrole, Ammoniumsulfamat, Ancymidol, Anilofos, Asulam, Atrazine, Azafenidin, Azimsulfuron, Aziprotryn, BAH-043, BAS-140H, BAS-693H, BAS-714H, BAS-762H, BAS-776H, BAS-800H, Beflubutamid, Benazolin, Benazolin-ethyl, bencarbazone, Benfluralin, Benfuresate, Bensulide, Bensulfuron-methyl, Bentazone, Benzfendizone, Benzobicyclon, Benzofenap, Benzofluor, Benzoylprop, Bifenox, Bilanafos, Bilanafos-natrium, Bispyribac, Bispyribac-natrium, Bromacil, Bromobutide, Bromofenoxim, Bromoxynil, Bromuron, Buminafos, Busoxinone, Butachlor, Butafenacil, Butamifos, Butenachlor, Butralin, Butroxydim, Butylate, Cafenstrole, Carbetamide, Carfentrazone, Carfentrazone-ethyl, Chlomethoxyfen, Chloramben, Chlorazifop, Chlorazifop-butyl, Chlorbromuron, Chlorbufam, Chlorfenac, Chlorfenac-natrium, Chlorfenprop, Chlorflurenol, Chlorflurenol-methyl, Chloridazon, Chlorimuron, Chlorimuron-ethyl, Chlormequat-chlorid, Chlornitrofen, Chlorophthalim, Chlorthaldimethyl, Chlorotoluron, Chlorsulfuron, Cinidon, Cinidon-ethyl, Cinmethylin, Cinosulfuron, Clethodim, Clodinafop Clodinafop-propargyl, Clofencet, Clomazone, Clomeprop, Cloprop, Clopyralid, Cloransulam, Cloransulam-methyl, Cumyluron, Cyanamide, Cyanazine, Cyclanilide, Cycloate, Cyclosulfamuron, Cycloxydim, Cycluron, Cyhalofop, Cyhalofop-butyl, Cyperquat, Cyprazine, Cyprazole, 2,4-D, 2,4-DB, Daimuron/Dymron, Dalapon, Daminozide, Dazomet, n-Decanol, Desmedipham, Desmetryn, Detosyl-Pyrazolate (DTP), Diallate, Dicamba, Dichlobenil, Dichlorprop, Dichlorprop-P, Diclofop, Diclofop-methyl, Diclofop-P-methyl, Diclosulam, Diethatyl, Diethatyl-ethyl, Difenoxuron, Difenzoquat, Diflufenican, Diflufenzopyr, Diflufenzopyrnatrium, Dimefuron, Dikegulac-sodium, Dimefuron, Dimepiperate, Dimethachlor, Dimethametryn, Dimethenamid, Dimethenamid-P, Dimethipin, Dimetrasulfuron, Dinitramine, Dinoseb, Dinoterb, Diphenamid, Dipropetryn, Diquat, Diquat-dibromide, Dithiopyr, Diuron, DNOC, Eglinazine-ethyl, Endothal, EPTC, Esprocarb, Ethalfluralin, Ethametsulfuron-methyl, Ethephon, Ethidimuron, Ethiozin, Ethofumesate, Ethoxyfen, Ethoxyfen-ethyl, Ethoxysulfuron, Etobenzanid, F-5331, d.h. N-[2-Chlor-4-fluor-5-[4-(3fluorpropyl)-4,5-dihydro-5-oxo-1H-tetrazol-1-yl]-phenyl]-ethansulfonamid, Fenoprop, Fenoxaprop, Fenoxaprop-P, Fenoxaprop-ethyl, Fenoxaprop-P-ethyl, Fentrazamide, Fenuron, Flamprop, Flamprop-M-isopropyl, Flamprop-M-methyl, Flazasulfuron, Florasulam, Fluazifop, Fluazifop-P, Fluazifop-butyl, Fluazifop-P-butyl, Fluazolate, Flucarbazone, Flucarbazone-sodium, Flucetosulfuron, Fluchloralin, Flufenacet (Thiafluamide), Flufenpyr, Flufenpyr-ethyl, Flumetralin, Flumetsulam, Flumiclorac, Flumiclorac-pentyl, Flumioxazin, Flumipropyn, Fluometuron, Fluorodifen, Fluoroglycofen, Fluoroglycofen-ethyl, Flupoxam, Flupropacil, Flupropanate, Flupyrsulfuron, Flupyrsulfuron-methyl-sodium, Flurenol, Flurenolbutyl, Fluridone, Flurochloridone, Fluroxypyr, Fluroxypyr-meptyl, Flurprimidol, Flurtamone, Fluthiacet, Fluthiacet-methyl, Fluthiamide, Fomesafen, Foramsulfuron, Forchlorfenuron, Fosamine, Furyloxyfen, Gibberellinsäure, Glufosinate, L-Glufosinate, L-Glufosinate-ammonium, Glufosinate-ammonium, Glyphosate, Glyphosate-isopropylammonium, H-9201, Halosafen, Halosulfuron, Halosulfuron-methyl, Haloxyfop, Haloxyfop-P, Haloxyfop-ethoxyethyl, Haloxyfop-P-ethoxyethyl, Haloxyfop-methyl, Haloxyfop-P-methyl, Hexazinone, HNPC-9908, HOK-201, HW-02, Imazamethabenz, Imazamethabenz-methyl, Imazamox, Imazapic, Imazapyr, Imazaquin, Imazethapyr, Imazosulfuron, Inabenfide, Indanofan, Indolessigsäure (IAA), 4-Indol-3-ylbuttersäure (IBA), Iodosulfuron, Iodosulfuron-methyl-natrium, Ioxynil, Isocarbamid, Isopropalin, Isoproturon, Isouron, Isoxaben, Isoxachlortole, Isoxaflutole, Isoxapyrifop, IDH-100, KUH-043, KUH-071, Karbutilate, Ketospiradox, Lactofen, Lenacil, Linuron, Maleinsäurehydrazid, MCPA, MCPB, MCPB-methyl, -ethyl und -natrium, Mecoprop, Mecoprop-natrium, Mecoprop-butotyl, Mecoprop-P-butotyl, Mecoprop-P-dimethylammonium, Mecoprop-P-2-ethylhexyl, Mecoprop-P-kalium, Mefenacet, Mefluidide, Mepiquat-chlorid, Mesosulfuron, Mesosulfuron-methyl, Mesotrione, Methabenzthiazuron, Metam, Metamifop, Metamitron, Metazachlor, Methazole, Methoxyphenone, Methyldymron, 1-Methylcyclopropen, Methylisothiocyanat, Metobenzuron, Metobenzuron, Metobromuron, Metolachlor, S-Metolachlor, Metosulam, Metoxuron, Metribuzin, Metsulfuron, Metsulfuron-methyl, Molinate, Monalide, Monocarbamide, Monocarbamide-dihydrogensulfat, Monolinuron, Monosulfuron, Monuron, MT 128, MT-5950, d. h. N-[3-Chlor-4-(1-methylethyl)-phenyl]-2-methylpentanamid, NGGC-011, Naproanilide, Napropamide, Naptalam, NC-310, d.h. 4-(2,4-dichlorobenzoyl)-1-methyl-5-benzyloxypyrazole, Neburon, Nicosulfuron, Nipyraclofen, Nitralin, Nitrofen, Nitrophenolat-natrium (Isomerengemisch), Nitrofluorfen, Nonansäure, Norflurazon, Orbencarb, Orthosulfamuron, Oryzalin, Oxadiargyl, Oxadiazon, Oxasulfuron, Oxaziclomefone, Oxyfluorfen, Paclobutrazol, Paraquat, Paraquat-dichlorid, Pelargonsäure (Nonansäure), Pendimethalin, Pendralin, Penoxsulam, Pentanochlor, Pentoxazone, Perfluidone, Pethoxamid, Phenisopham, Phenmedipham, Phenmedipham-ethyl, Picloram, Picolinafen, Pinoxaden, Piperophos, Pirifenop, Pirifenop-butyl, Pretilachlor, Primisulfuron, Primisulfuron-methyl, Probenazole, Profluazol, Procyazine, Prodiamine, Prifluraline, Profoxydim, Prohexadione, Prohexadione-calcium, Prohydrojasmone, Prometon, Prometryn, Propachlor, Propanil, Propaquizafop, Propazine, Propham, Propisochlor, Propoxycarbazone, Propoxycarbazone-natrium, Propyzamide, Prosulfalin, Prosulfocarb, Prosulfuron, Prynachlor, Pyraclonil, Pyraflufen, Pyraflufen-ethyl, Pyrasulfotole, Pyrazolynate (Pyrazolate), Pyrazosulfuron-ethyl, Pyrazoxyfen, Pyribambenz, Pyribambenz-isopropyl, Pyribenzoxim, Pyributicarb, Pyridafol, Pyridate, Pyriftalid, Pyriminobac, Pyriminobac-methyl, Pyrimisulfan, Pyrithiobac, Pyrithiobac-natrium, Pyroxasulfone, Pyroxsulam, Quinclorac, Quinmerac, Quinoclamine, Quizalofop, Quizalofop-ethyl, Quizalofop-P, Quizalofop-P-ethyl, Quizalofop-P-tefuryl, Rimsulfuron, Secbumeton, Sethoxydim, Siduron, Simazine, Simetryn, SN-106279, Sulcotrione, Sulfallate (CDEC), Sulfentrazone, Sulfometuron, Sulfometuron-methyl, Sulfosate (Glyphosate-trimesium), Sulfosulfuron, SYN-523, SYP-249, SYP-298, SYP-300, Tebutam, Tebuthiuron, Tecnazene, Tefuryltrione, Tembotrione, Tepraloxydim, Terbacil, Terbucarb, Terbuchlor, Terbumeton, Terbuthylazine, Terbutryn, TH-547, Thenylchlor, Thiafluamide, Thiazafluron, Thiazopyr, Thidiazimin, Thidiazuron, Thiencarbazone, Thiencarbazone-methyl, Thifensulfuron, Thifensulfuron-methyl, Thiobencarb, Tiocarbazil, Topramezone, Tralkoxydim, Triallate, Triasulfuron, Triaziflam, Triazofenamide, Tribenuron, Tribenuron-methyl, Trichloressigsäure (TCA), Triclopyr, Tridiphane, Trietazine, Trifloxysulfuron, Trifloxysulfuron-natrium, Trifluralin, Triflusulfuron, Triflusulfuron-methyl, Trimeturon, Trinexapac, Trinexapac-ethyl, Tritosulfuron, Tsitodef, Uniconazole, Uniconazole-P, Vernolate, ZJ-0166, ZJ-0270, ZJ-0543, oder ZJ-0862 sowie die folgenden Verbindungen

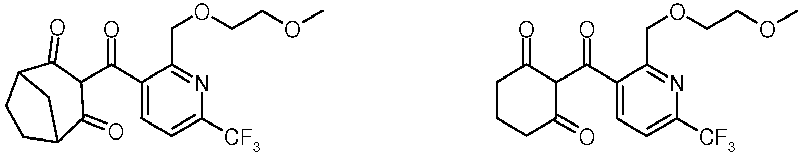

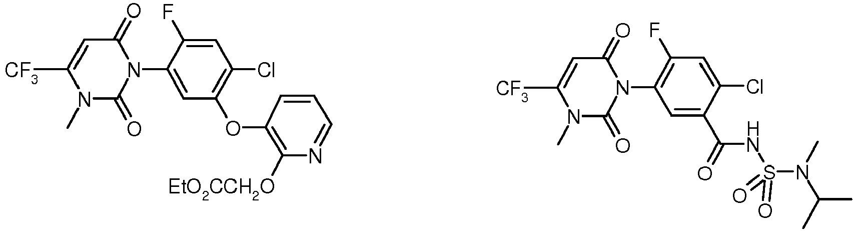

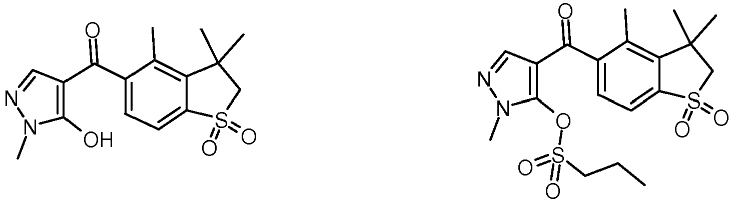

Verbindungen sind entweder mit dem "common name" nach der International Organization for Standardization (ISO) oder mit ihrem chemischen Namen oder Codenummer bezeichnet und umfassen stets sämtliche Anwendungsformen wie Säuren, Salze, Ester oder Modifikationen, wie Isomere, Stereoisomere und optische Isomere. Beispielhaft sind eine oder auch mehrere Anwendungsformen oder Modifikationen genannt.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-Dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylen-sorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulate siehe z.B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0.1 bis 99 Gew.-%, insbesondere 0.1 bis 95 Gew.-%, erfindungsgemäße Verbindungen.

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0.05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Die erfindungsgemäßen Verbindungen weisen eine starke mikrobizide Wirkung auf und können zur Bekämpfung von unerwünschten Mikroorganismen, wie Fungi (Pilze) und Bakterien, im Pflanzenschutz und im Materialschutz eingesetzt werden.

Fungizide lassen sich Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Bakterizide lassen sich im Pflanzenschutz zur Bekämpfung von Pseudomonadaceae, Rhizobiaceae, Enterobacteriaceae, Corynebacteriaceae und Streptomycetaceae einsetzen.

Beispielhaft aber nicht begrenzend seien einige Erreger von pilzlichen und bakteriellen Erkrankungen, die unter die oben aufgezählten Oberbegriffe fallen, genannt:
Erkrankungen, hervorgerufen durch Erreger des Echten Mehltaus wie z.B.
Blumeria-Arten, wie beispielsweise Blumeria graminis;
Podosphaera-Arten, wie beispielsweise Podosphaera leucotricha;
Sphaerotheca-Arten, wie beispielsweise Sphaerotheca fuliginea;
Uncinula-Arten, wie beispielsweise Uncinula necator;
Erkrankungen, hervorgerufen durch Erreger von Rostkrankheiten wie z.B.
Gymnosporangium-Arten, wie beispielsweise Gymnosporangium sabinae
Hemileia-Arten, wie beispielsweise Hemileia vastatrix;
Phakopsora-Arten, wie beispielsweise Phakopsora pachyrhizi und Phakopsora meibomiae;
Puccinia-Arten, wie beispielsweise Puccinia recondita;
Uromyces-Arten, wie beispielsweise Uromyces appendiculatus;
Erkrankungen, hervorgerufen durch Erreger der Gruppe der Oomyceten wie z.B.
Bremia-Arten, wie beispielsweise Bremia lactucae;
Peronospora-Arten, wie beispielsweise Peronospora pisi oder P. brassicae;
Phytophthora-Arten, wie beispielsweise Phytophthora infestans;
Plasmopara-Arten, wie beispielsweise Plasmopara viticola;
Pseudoperonospora-Arten, wie beispielsweise Pseudoperonospora humuli oder
Pseudoperonospora cubensis;
Pythium-Arten, wie beispielsweise Pythium ultimum;
Blattfleckenkrankheiten und Blattwelken, hervorgerufen durch z.B.
Alternaria-Arten, wie beispielsweise Alternaria solani;
Cercospora-Arten, wie beispielsweise Cercospora beticola;
Cladosporium-Arten, wie beispielsweise Cladosporium cucumerinum;
Cochliobolus-Arten, wie beispielsweise Cochliobolus sativus
(Konidienform: Drechslera, Syn: Helminthosporium);
Colletotrichum-Arten, wie beispielsweise Colletotrichum lindemuthanium;
Cycloconium-Arten, wie beispielsweise Cycloconium oleaginum;
Diaporthe-Arten, wie beispielsweise Diaporthe citri;
Elsinoe-Arten, wie beispielsweise Elsinoe fawcettii;
Gloeosporium-Arten, wie beispielsweise Gloeosporium laeticolor;
Glomerella-Arten, wie beispielsweise Glomerella cingulata;
Guignardia-Arten, wie beispielsweise Guignardia bidwelli;
Leptosphaeria-Arten, wie beispielsweise Leptosphaeria maculans;
Magnaporthe-Arten, wie beispielsweise Magnaporthe grisea;
Mycosphaerella-Arten, wie beispielsweise Mycosphaerella graminicola und Mycosphaerella fijiensis;
Phaeosphaeria-Arten, wie beispielsweise Phaeosphaeria nodorum;
Pyrenophora-Arten, wie beispielsweise Pyrenophora teres;
Ramularia-Arten, wie beispielsweise Ramularia collo-cygni;
Rhynchosporium-Arten, wie beispielsweise Rhynchosporium secalis;
Septoria-Arten, wie beispielsweise Septoria apii;
Typhula-Arten, wie beispielsweise Typhula incarnata;
Venturia-Arten, wie beispielsweise Venturia inaequalis;
Wurzel- und Stengelkrankheiten, hervorgerufen durch z.B.
Corticium-Arten, wie beispielsweise Corticium graminearum;
Fusarium-Arten, wie beispielsweise Fusarium oxysporum;
Gaeumannomyces-Arten, wie beispielsweise Gaeumannomyces graminis;
Rhizoctonia-Arten, wie beispielsweise Rhizoctonia solani;
Tapesia-Arten, wie beispielsweise Tapesia acuformis;
Thielaviopsis-Arten, wie beispielsweise Thielaviopsis basicola;
Ähren- und Rispenerkrankungen (inklusive Maiskolben), hervorgerufen durch z.B.
Alternaria-Arten, wie beispielsweise Alternaria spp.;
Aspergillus-Arten, wie beispielsweise Aspergillus flavus;
Cladosporium-Arten, wie beispielsweise Cladosporium cladosporioides;
Claviceps-Arten, wie beispielsweise Claviceps purpurea;
Fusarium-Arten, wie beispielsweise Fusarium culmorum;
Gibberella-Arten, wie beispielsweise Gibberella zeae;
Monographella-Arten, wie beispielsweise Monographella nivalis;
Erkrankungen, hervorgerufen durch Brandpilze wie z.B.
Sphacelotheca-Arten, wie beispielsweise Sphacelotheca reiliana;
Tilletia-Arten, wie beispielsweise Tilletia caries;
Urocystis-Arten, wie beispielsweise Urocystis occulta;
Ustilago-Arten, wie beispielsweise Ustilago nuda;
Fruchtfäule hervorgerufen durch z.B.
Aspergillus-Arten, wie beispielsweise Aspergillus flavus;
Botrytis-Arten, wie beispielsweise Botrytis cinerea;
Penicillium-Arten, wie beispielsweise Penicillium expansum und Penicillium purpurogenum;
Sclerotinia-Arten, wie beispielsweise Sclerotinia sclerotiorum;
Verticilium-Arten, wie beispielsweise Verticilium alboatrum;
Samen- und bodenbürtige Fäulen und Welken, sowie Sämlingserkrankungen, hervorgerufen durch z.B.
Alternaria-Arten, wie beispielsweise Alternaria brassicicola
Aphanomyces-Arten, wie beispielsweise Aphanomyces euteiches
Ascochyta-Arten, wie beispielsweise Ascochyta lentis
Aspergillus-Arten, wie beispielsweise Aspergillus flavus
Cladosporium-Arten, wie beispielsweise Cladosporium herbarum
Cochliobolus-Arten, wie beispielsweise Cochliobolus sativus
(Konidienform: Drechslera, Bipolaris Syn: Helminthosporium);
Colletotrichum-Arten, wie beispielsweise Colletotrichum coccodes;
Fusarium-Arten, wie beispielsweise Fusarium culmorum;
Gibberella-Arten, wie beispielsweise Gibberella zeae;
Macrophomina-Arten, wie beispielsweise Macrophomina phaseolina
Monographella-Arten, wie beispielsweise Monographella nivalis;
Penicillium-Arten, wie beispielsweise Penicillium expansum
Phoma-Arten, wie beispielsweise Phoma lingam
Phomopsis-Arten, wie beispielsweise Phomopsis sojae;
Phytophthora Arten, wie beispielsweise Phytophthora cactorum;
Pyrenophora-Arten, wie beispielsweise Pyrenophora graminea
Pyricularia-Arten, wie beispielsweise Pyricularia oryzae;
Pythium-Arten, wie beispielsweise Pythium ultimum;
Rhizoctonia-Arten, wie beispielsweise Rhizoctonia solani;
Rhizopus-Arten, wie beispielsweise Rhizopus oryzae
Sclerotium-Arten, wie beispielsweise Sclerotium rolfsii;
Septoria-Arten, wie beispielsweise Septoria nodorum;
Typhula-Arten, wie beispielsweise Typhula incarnata;
Verticillium-Arten, wie beispielsweise Verticillium dahliae
Krebserkrankungen, Gallen und Hexenbesen, hervorgerufen durch z.B.
Nectria-Arten, wie beispielsweise Nectria galligena;
Welkeerkrankungen hervorgerufen durch z.B.
Monilinia-Arten, wie beispielsweise Monilinia laxa;
Deformationen von Blättern, Blüten und Früchten, hervorgerufen durch z.B.
Taphrina-Arten, wie beispielsweise Taphrina deformans;
Degenerationserkrankungen holziger pflanzen, hervorgerufen durch z.B.
Esca-Arten, wie beispielsweise Phaeomoniella chlamydospora und Phaeoacremonium aleophilum und Fomitiporia mediterranea;
Blüten- und Samenerkrankungen, hervorgerufen durch z.B.
Botrytis-Arten, wie beispielsweise Botrytis cinerea;
Erkrankungen von Pflanzenknollen, hervorgerufen durch z.B.
Rhizoctonia-Arten, wie beispielsweise Rhizoctonia solani;
Helminthosporium-Arten, wie beispielsweise Helminthosporium solani;
Erkrankungen, hervorgerufen durch bakterielle Erreger wie z.B.
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Bevorzugt können die folgenden Krankheiten von Soja-Bohnen bekämpft werden:
Pilzkrankheiten an Blättern, Stängeln, Schoten und Samen verursacht durch z.B.
Alternaria leaf spot (Alternaria spec. atrans tenuissima), Anthracnose (Colletotrichum gloeosporoides dematium var. truncatum), Brown spot (Septoria glycines), Cercospora leaf spot and blight (Cercospora kikuchii), Choanephora leaf blight (Choanephora infundibulifera trispora (Syn.)), Dactuliophora leaf spot (Dactuliophora glycines), Downy Mildew (Peronospora manshurica), Drechslera blight (Drechslera glycini), Frogeye Leaf spot (Cercospora sojina), Leptosphaerulina Leaf Spot (Leptosphaerulina trifolii), Phyllostica Leaf Spot (Phyllosticta sojaecola), Pod and Stem Blight (Phomopsis sojae), Powdery Mildew (Microsphaera diffusa), Pyrenochaeta Leaf Spot (Pyrenochaeta glycines), Rhizoctonia Aerial, Foliage, and Web Blight (Rhizoctonia solani), Rust (Phakopsora pachyrhizi), Scab (Sphaceloma glycines), Stemphylium Leaf Blight (Stemphylium botryosum), Target Spot (Corynespora cassiicola)
Pilzkrankheiten an Wurzeln und der Stängelbasis verursacht durch z.B.
Black Root Rot (Calonectria crotalariae), Charcoal Rot (Macrophomina phaseolina), Fusarium Blight or Wilt, Root Rot, and Pod and Collar Rot (Fusarium oxysporum, Fusarium orthoceras, Fusarium semitectum, Fusarium equiseti), Mycoleptodiscus Root Rot (Mycoleptodiscus terrestris), Neocosmospora (Neocosmopspora vasinfecta), Pod and Stem Blight (Diaporthe phaseolorum), Stem Canker (Diaporthe phaseolorum var. caulivora), Phytophthora Rot (Phytophthora megasperma), Brown Stem Rot (Phialophora gregata), Pythium Rot (Pythium aphanidermatum, Pythium irregulare, Pythium debaryanum, Pythium myriotylum, Pythium ultimum), Rhizoctonia Root Rot, Stem Decay, and Damping-Off (Rhizoctonia solani), Sclerotinia Stem Decay (Sclerotinia sclerotiorum), Sclerotinia Southern Blight (Sclerotinia rolfsii), Thielaviopsis Root Rot (Thielaviopsis basicola).

Die erfindungsgemäßen Wirkstoffe weisen auch eine stärkende Wirkung in Pflanzen auf. Sie eignen sich daher zur Mobilisierung pflanzeneigener Abwehrkräfte gegen Befall durch unerwünschte Mikroorganismen.

Unter pflanzenstärkenden (resistenzinduzierenden) Stoffen sind im vorliegenden Zusammenhang solche Substanzen zu verstehen, die in der Lage sind, das Abwehrsystem von Pflanzen so zu stimulieren, dass die behandelten Pflanzen bei nachfolgender Inokulation mit unerwünschten Mikroorganismen weitgehende Resistenz gegen diese Mikroorganismen entfalten.

Unter unerwünschten Mikroorganismen sind im vorliegenden Fall phytopathogene Pilze, Bakterien und Viren zu verstehen. Die erfindungsgemäßen Stoffe können also eingesetzt werden, um Pflanzen innerhalb eines gewissen Zeitraums nach der Behandlung gegen den Befall durch die genannten Schaderreger zu schützen. Der Zeitraum, innerhalb dessen Schutz herbeigeführt wird, erstreckt sich im Allgemeinen von 1 bis 10 Tage, vorzugsweise 1 bis 7 Tage nach der Behandlung der Pflanzen mit den Wirkstoffen.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Getreidekrankheiten, wie z.B. gegen Puccinia-Arten und von Krankheiten im Wein-, Obst- und Gemüseanbau, wie z.B. gegen Botrytis-, Venturia- oder Alternaria-Arten, einsetzen.

Die erfindungsgemäßen Wirkstoffe eignen sich auch zur Steigerung des Ernteertrages. Sie sind außerdem mindertoxisch und weisen eine gute Pflanzenverträglichkeit auf.

Darüber hinaus kann durch die erfindungsgemäße Behandlung der Mykotoxingehalt im Erntegut und den daraus hergestellten Nahrungs- und Futtermitteln verringert werden. Besonders, aber nicht ausschließlich sind hierbei folgende Mykotoxine zu nennen: Deoxynivalenol (DON), Nivalenol, 15-Ac-DON, 3-Ac-DON, T2- und HT2- Toxin, Fumonisine, Zearalenon, Moniliformin, Fusarin, Diaceotoxyscirpenol (DAS), Beauvericin, Enniatin, Fusaroproliferin, Fusarenol, Ochratoxine, Patulin, Mutterkornalkaloide und Aflatoxine, die beispielsweise von den folgenden Pilzen verursacht werden können: Fusarium spec., wie Fusarium acuminatum, F. avenaceum, F. crookwellense, F. culmorum, F. graminearum (Gibberella zeae), F. equiseti, F. fujikoroi, F. musarum, F. oxysporum, F. proliferatum, F. poae, F. pseudograminearum, F. sambucinum, F. scirpi, F. semitectum, F. solani, F. sporotrichoides, F. langsethiae, F. subglutinans, F. tricinctum, F. verticillioides u.a. sowie auch von Aspergillus spec., Penicillium spec., Claviceps purpurea, Stachybotrys spec. u.a.

Im Materialschutz lassen sich die erfindungsgemäßen Stoffe zum Schutz von technischen Materialien gegen Befall und Zerstörung durch unerwünschte Mikroorganismen einsetzen.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten) sowie gegen Schleimorganismen und Algen.

Es seien beispielsweise Mikroorganismen der folgenden Gattungen genannt:
Alternaria, wie Alternaria tenuis,
Aspergillus, wie Aspergillus niger,
Chaetomium, wie Chaetomium globosum,
Coniophora, wie Coniophora puetana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Die vorliegende Erfindung betrifft ein Mittel zum Bekämpfen unerwünschter Mikroorganismen, umfassend wenigstens eines der erfindungsgemäßen Verbindungen.

Die erfindungsgemäßen Verbindungen können dazu in Abhängigkeit von ihren jeweiligen physikalischen und/ oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/ oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im Wesentlichen infrage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen infrage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen infrage: z.B. gebrochene und fraktionierte natürliche Gesteine wie Calcit, Bims, Marmor, Sepiolith, Dolomit sowie synthetische Granulate aus anorganischen und organischen Mehlen sowie Granulate aus organischem Material wie Sägemehl, Kokosnussschalen, Maiskolben und Tabakstängel. Als Emulgier und/oder schaumerzeugende Mittel kommen infrage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen infrage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im Allgemeinen zwischen 0,1 und 95 Gewichtsprozent Wirkstoff, vorzugsweise zwischen 0,5 und 90 %.

Die zuvor beschriebenen Formulierungen können in einem erfindungsgemäßen Verfahren zum Bekämpfen unerwünschter Mikroorganismen verwendet werden, bei dem die erfindungsgemäßen Verbindungen auf die Mikroorganismen und/oder in deren Lebensraum ausgebracht werden.

Die Bekämpfung von phytopathogenen Pilzen durch die Behandlung des Saatguts von Pflanzen ist seit langem bekannt und ist Gegenstand ständiger Verbesserungen. Dennoch ergeben sich bei der Behandlung von Saatgut eine Reihe von Problemen, die nicht immer zufrieden stellend gelöst werden können. So ist es erstrebenswert, Verfahren zum Schutz des Saatguts und der keimenden Pflanze zu entwickeln, die das zusätzliche Ausbringen von Pflanzenschutzmitteln nach der Saat oder nach dem Auflaufen der Pflanzen überflüssig machen oder zumindest deutlich verringern. Es ist weiterhin erstrebenswert, die Menge des eingesetzten Wirkstoffs dahingehend zu optimieren, dass das Saatgut und die keimende Pflanze vor dem Befall durch phytopathogene Pilze bestmöglich geschützt wird, ohne jedoch die Pflanze selbst, durch den eingesetzten Wirkstoff zu schädigen. Insbesondere sollten Verfahren zur Behandlung von Saatgut auch die intrinsischen fungiziden Eigenschaften transgener Pflanzen einbeziehen, um einen optimalen Schutz des Saatguts und der keimenden Pflanze bei einem minimalen Aufwand an Pflanzenschutzmitteln zu erreichen.

Die vorliegende Erfindung bezieht sich daher insbesondere auch auf ein Verfahren zum Schutz von Saatgut und keimenden Pflanzen vor dem Befall von phytopathogenen Pilzen, indem das Saatgut mit einem erfindungsgemäßen Mittel behandelt wird.

Die Erfindung bezieht sich ebenfalls auf die Verwendung der erfindungsgemäßen Mittel zur Behandlung von Saatgut zum Schutz des Saatguts und der keimenden Pflanze vor phytopathogenen Pilzen.

Weiterhin bezieht sich die Erfindung auf Saatgut, welches zum Schutz vor phytopathogenen Pilzen mit einem erfindungsgemäßen Mittel behandelt wurde.

Einer der Vorteile der vorliegenden Erfindung ist es, dass aufgrund der besonderen systemischen Eigenschaften der erfindungsgemäßen Mittel die Behandlung des Saatguts mit diesen Mitteln nicht nur das Saatgut selbst, sondern auch die daraus hervorgehenden Pflanzen nach dem Auflaufen vor phytopathogenen Pilzen schützt. Auf diese Weise kann die unmittelbare Behandlung der Kultur zum Zeitpunkt der Aussaat oder kurz danach entfallen.

Ebenso ist es als vorteilhaft anzusehen, dass die erfindungsgemäßen Mischungen insbesondere auch bei transgenem Saatgut eingesetzt werden können.

Die erfindungsgemäßen Mittel eignen sich zum Schutz von Saatgut jeglicher Pflanzensorte, die in der Landwirtschaft, im Gewächshaus, in Forsten oder im Gartenbau eingesetzt wird. Insbesondere handelt es sich dabei um Saatgut von Getreide (wie Weizen, Gerste, Roggen, Hirse und Hafer), Mais, Baumwolle, Soja, Reis, Kartoffeln, Sonnenblume, Bohne, Kaffee, Rübe (z.B. Zuckerrübe und Futterrübe), Erdnuss, Gemüse (wie Tomate, Gurke, Zwiebeln und Salat), Rasen und Zierpflanzen. Besondere Bedeutung kommt der Behandlung des Saatguts von Getreide (wie Weizen, Gerste, Roggen und Hafer), Mais und Reis zu.

Im Rahmen der vorliegenden Erfindung wird das erfindungsgemäße Mittel alleine oder in einer geeigneten Formulierung auf das Saatgut aufgebracht. Vorzugsweise wird das Saatgut in einem Zustand behandelt, in dem so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hülle, Wolle oder Fruchtfleisch befreit wurde. So kann zum Beispiel Saatgut verwendet werden, das geerntet, gereinigt und bis zu einem Feuchtigkeitsgehalt von unter 15 Gew.-% getrocknet wurde. Alternativ kann auch Saatgut verwendet werden, das nach dem Trocknen z.B. mit Wasser behandelt und dann erneut getrocknet wurde.

Im Allgemeinen muss bei der Behandlung des Saatguts darauf geachtet werden, dass die Menge des auf das Saatgut aufgebrachten erfindungsgemäßen Mittels und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei Wirkstoffen zu beachten, die in bestimmten Aufwandmengen phytotoxische Effekte zeigen können.

Die erfindungsgemäßen Mittel können unmittelbar aufgebracht werden, also ohne weitere Komponenten zu enthalten und ohne verdünnt worden zu sein. In der Regel ist es vorzuziehen, die Mittel in Form einer geeigneten Formulierung auf das Saatgut aufzubringen. Geeignete Formulierungen und Verfahren für die Saatgutbehandlung sind dem Fachmann bekannt und werden z.B. in den folgenden Dokumenten beschrieben: US 4,272,417 A, US 4,245,432 A, US 4,808,430 A, US 5,876,739 A, US 2003/0176428 A1, WO 2002/080675 A1, WO 2002/028186 A2.

Die erfindungsgemäß verwendbaren Wirkstoffkombinationen können in die üblichen Beizmittel-Formulierungen überführt werden, wie Lösungen, Emulsionen, Suspensionen, Pulver, Schäume, Slurries oder andere Hüllmassen für Saatgut, sowie ULV-Formulierungen.

Diese Formulierungen werden in bekannter Weise hergestellt, indem man die Wirkstoffe oder Wirkstoffkombinationen mit üblichen Zusatzstoffen vermischt, wie zum Beispiel übliche Streckmittel sowie Lösungs- oder Verdünnungsmittel, Farbstoffe, Netzmittel, Dispergiermittel, Emulgatoren, Entschäumer, Konservierungsmittel, sekundäre Verdickungsmittel, Kleber, Gibberelline und auch Wasser.

Als Farbstoffe, die in den erfindungsgemäß verwendbaren Beizmittel-Formulierungen enthalten sein können, kommen alle für derartige Zwecke üblichen Farbstoffe in Betracht. Dabei sind sowohl in Wasser wenig lösliche Pigmente als auch in Wasser lösliche Farbstoffe verwendbar. Als Beispiele genannt seien die unter den Bezeichnungen Rhodamin B, C.I. Pigment Red 112 und C.I. Solvent Red 1 bekannten Farbstoffe.

Als Netzmittel, die in den erfindungsgemäß verwendbaren Beizmittel-Formulierungen enthalten sein können, kommen alle zur Formulierung von agrochemischen Wirkstoffen üblichen, die Benetzung fördernden Stoffe in Frage. Vorzugsweise verwendbar sind Alkylnaphthalin-Sulfonate, wie Diisopropyl- oder Diisobutyl-naphthalin-Sulfonate.

Als Dispergiermittel und/oder Emulgatoren, die in den erfindungsgemäß verwendbaren Beizmittel-Formulierungen enthalten sein können, kommen alle zur Formulierung von agrochemischen Wirkstoffen üblichen nichtionischen, anionischen und kationischen Dispergiermittel in Betracht. Vorzugsweise verwendbar sind nichtionische oder anionische Dispergiermittel oder Gemische von nichtionischen oder anionischen Dispergiermitteln. Als geeignete nichtionische Dispergiermittel sind insbesondere Ethylenoxid-Propylenoxid Blockpolymere, Alkylphenolpolyglykolether sowie Tristryrylphenolpolyglykolether und deren phosphatierte oder sulfatierte Derivate zu nennen. Geeignete anionische Dispergiermittel sind insbesondere Ligninsulfonate, Polyacrylsäuresalze und Arylsulfonat-Formaldehydkondensate.

Als Entschäumer können in den erfindungsgemäß verwendbaren Beizmittel-Formulierungen alle zur Formulierung von agrochemischen Wirkstoffen üblichen schaumhemmenden Stoffe enthalten sein. Vorzugsweise verwendbar sind Silikonentschäumer und Magnesiumstearat.

Als Konservierungsmittel können in den erfindungsgemäß verwendbaren Beizmittel-Formulie-rungen alle für derartige Zwecke in agrochemischen Mitteln einsetzbaren Stoffe vorhanden sein. Beispielhaft genannt seien Dichlorophen und Benzylalkoholhemiformal.

Als sekundäre Verdickungsmittel, die in den erfindungsgemäß verwendbaren Beizmittel-Formulierungen enthalten sein können, kommen alle für derartige Zwecke in agrochemischen Mitteln einsetzbaren Stoffe in Frage. Vorzugsweise in Betracht kommen Cellulosederivate, Acrylsäurederivate, Xanthan, modifizierte Tone und hochdisperse Kieselsäure.

Als Kleber, die in den erfindungsgemäß verwendbaren Beizmittel-Formulierungen enthalten sein können, kommen alle üblichen in Beizmitteln einsetzbaren Bindemittel in Frage. Vorzugsweise genannt seien Polyvinylpyrrolidon, Polyvinylacetat, Polyvinylalkohol und Tylose.

Als Gibberelline, die in den erfindungsgemäß verwendbaren Beizmittel-Formulierungen enthalten sein können, kommen vorzugsweise die Gibberelline A1, A3 (= Gibberellinsäure), A4 und A7 infrage, be-sonders bevorzugt verwendet man die Gibberellinsäure. Die Gibberelline sind bekannt (vgl. R. Wegler "Chemie der Pflanzenschutz- und Schädlingsbekämpfungsmittel", Bd. 2, Springer Verlag, 1970, S. 401-412).

Die erfindungsgemäß verwendbaren Beizmittel-Formulierungen können entweder direkt oder nach vorherigem Verdünnen mit Wasser zur Behandlung von Saatgut der verschiedensten Art eingesetzt werden. So lassen sich die Konzentrate oder die daraus durch Verdünnen mit Wasser erhältlichen Zubereitungen einsetzen zur Beizung des Saatgutes von Getreide, wie Weizen, Gerste, Roggen, Hafer und Triticale, sowie des Saatgutes von Mais, Reis, Raps, Erbsen, Bohnen, Baumwolle, Sonnenblumen und Rüben oder auch von Gemüsesaatgut der verschiedensten Natur. Die erfindungsgemäß verwendbaren Beizmittel-Formulierungen oder deren verdünnte Zubereitungen können auch zum Beizen von Saatgut transgener Pflanzen eingesetzt werden. Dabei können im Zusammenwirken mit den durch Expression gebildeten Substanzen auch zusätzliche synergistische Effekte auftreten.

Zur Behandlung von Saatgut mit den erfindungsgemäß verwendbaren Beizmittel-Formulierungen oder den daraus durch Zugabe von Wasser hergestellten Zubereitungen kommen alle üblicherweise für die Beizung einsetzbaren Mischgeräte in Betracht. Im einzelnen geht man bei der Beizung so vor, dass man das Saatgut in einen Mischer gibt, die jeweils gewünschte Menge an Beizmittel-Formulierungen entweder als solche oder nach vorherigem Verdünnen mit Wasser hinzufügt und bis zur gleichmäßigen Verteilung der Formulierung auf dem Saatgut mischt. Gegebenenfalls schließt sich ein Trocknungsvorgang an.

Die Aufwandmenge an den erfindungsgemäß verwendbaren Beizmittel-Formulierungen kann innerhalb eines größeren Bereiches variiert werden. Sie richtet sich nach dem jeweiligen Gehalt der Wirkstoffe in den Formulierungen und nach dem Saatgut. Die Aufwandmengen an Wirkstoffkombination liegen im Allgemeinen zwischen 0,001 und 50 g pro Kilogramm Saatgut, vorzugsweise zwischen 0,01 und 15 g pro Kilogramm Saatgut.

Die erfindungsgemäßen Verbindungen können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen.

Auch eine Mischung mit anderen bekannten Wirkstoffen, wie Herbiziden oder mit Düngemitteln und Wachstumsregulatoren, Safener bzw. Semiochemicals ist möglich.

Darüber hinaus weisen die erfindungsgemäßen Verbindungen der Formel (I) auch sehr gute antimykotische Wirkungen auf. Sie besitzen ein sehr breites antimykotisches Wirkungsspektrum, insbesondere gegen Dermatophyten und Sprosspilze, Schimmel und diphasische Pilze (z.B. gegen Candida-Spezies wie Candida albicans, Candida glabrata) sowie Epidermophyton floccosum, Aspergillus-Spezies wie Aspergillus niger und Aspergillus fumigatus, Trichophyton-Spezies wie Trichophyton mentagrophytes, Microsporon-Spezies wie Microsporon canis und audouinii. Die Aufzählung dieser Pilze stellt keinesfalls eine Beschränkung des erfassbaren mykotischen Spektrums dar, sondern hat nur erläuternden Charakter.

Die erfindungsgemäßen Verbindungen können daher sowohl in medizinische als auch in nichtmedizinische Anwendungen eingesetzt werden.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren.

Es kann auch das Saatgut der Pflanzen behandelt werden.

Beim Einsatz der erfindungsgemäßen Verbindungen als Fungizide können die Aufwandmengen je nach Applikationsart innerhalb eines größeren Bereiches variiert werden. Bei der Behandlung von Pflanzenteilen liegen die Aufwandmengen an Wirkstoff im allgemeinen zwischen 0,1 und 10.000 g/ha, vorzugsweise zwischen 10 und 1.000 g/ha. Bei der Saatgutbehandlung liegen die Aufwandmengen an Wirkstoff im allgemeinen zwischen 0,001 und 50 g pro Kilogramm Saatgut, vorzugsweise zwischen 0,01 und 10 g pro Kilogramm Saatgut. Bei der Behandlung des Bodens liegen die Aufwandmengen an Wirkstoff im Allgemeinen zwischen 0,1 und 10.000 g/ha, vorzugsweise zwischen 1 und 5.000 g/ha.

Das erfindungsgemäße Behandlungsverfahren wird vorzugsweise auf genetisch modifizierten Organismen, wie beispielsweise Pflanzen oder Pflanzenteile, verwendet.

Genetisch modifizierte Pflanzen, sogenannte transgene Pflanzen, sind Pflanzen, bei denen ein heterologes Gen stabil in das Genom integriert worden ist.

Der Begriff "heterologes Gen" bedeutet im wesentlichen ein Gen, das außerhalb der Pflanze bereitgestellt oder assembliert wird und das bei Einführung in das Zellkerngenom, das Chloroplastengenom oder das Hypochondriengenom der transformierten Pflanze dadurch neue oder verbesserte agronomische oder sonstige Eigenschaften verleiht, daß es ein interessierendes Protein oder Polypeptid exprimiert oder daß es ein anderes Gen, das in der Pflanze vorliegt bzw. andere Gene, die in der Pflanze vorliegen, herunterreguliert oder abschaltet (zum Beispiel mittels Antisense-Technologie, Cosuppressionstechnologie oder RNAi-Technologie [RNA Interference]). Ein heterologes Gen, das im Genom vorliegt, wird ebenfalls als Transgen bezeichnet. Ein Transgen, das durch sein spezifisches Vorliegen im Pflanzengenom definiert ist, wird als Transformations- bzw. transgenes Event bezeichnet.

In Abhängigkeit von den Pflanzenarten oder Pflanzensorten, ihrem Standort und ihren Wachstumsbedingungen (Böden, Klima, Vegetationsperiode, Ernährung) kann die erfindungsgemäße Behandlung auch zu überadditiven ("synergistischen") Effekten führen. So sind zum Beispiel die folgenden Effekte möglich, die über die eigentlich zu erwartenden Effekte hinausgehen: verringerte Aufwandmengen und/oder erweitertes Wirkungsspektrum und/oder erhöhte Wirksamkeit der Wirkstoffe und Zusammensetzungen, die erfindungsgemäß eingesetzt werden können, besseres Pflanzenwachstum, erhöhte Toleranz gegenüber hohen oder niedrigen Temperaturen, erhöhte Toleranz gegenüber Trockenheit oder Wasser- oder Bodensalzgehalt, erhöhte Blühleistung, Ernteerleichterung, Reifebeschleunigung, höhere Erträge, größere Früchte, größere Pflanzenhöhe, intensiver grüne Farbe des Blatts, frühere Blüte, höhere Qualität und/oder höherer Nährwert der Ernteprodukte, höhere Zuckerkonzentration in den Früchten, bessere Lagerfähigkeit und/oder Verarbeitbarkeit der Ernteprodukte.

In gewissen Aufwandmengen können die erfindungsgemäßen Wirkstoffkombinationen auch eine stärkende Wirkung auf Pflanzen ausüben. Sie eignen sich daher für die Mobilisierung des pflanzlichen Abwehrsystems gegen Angriff durch unerwünschte phytopathogene Pilze und/oder Mikroorganismen und/oder Viren. Dies kann gegebenenfalls einer der Gründe für die erhöhte Wirksamkeit der erfindungsgemäßen Kombinationen sein, zum Beispiel gegen Pilze. Pflanzenstärkende (resistenzinduzierende) Substanzen sollen im vorliegenden Zusammenhang auch solche Substanzen oder Substanzkombinationen bedeuten, die fähig sind, das pflanzliche Abwehrsystem so zu stimulieren, daß die behandelten Pflanzen, wenn sie im Anschluß daran mit unerwünschten phytopathogenen Pilzen und/oder Mikroorganismen und/oder Viren inokkuliert werde, einen beträchtlichen Resistenzgrad gegen diese unerwünschten phytopathogenen Pilze und/oder Mikroorganismen und/oder Viren aufweisen. Im vorliegenden Fall versteht man unter unerwünschten phytopathogenen Pilzen und/oder Mikroorganismen und/oder Viren phytopathogene Pilze, Bakterien und Viren. Die erfindungsgemäßen Substanzen lassen sich daher zum Schutz von Pflanzen gegen Angriff durch die erwähnten Pathogene innerhalb eines gewissen Zeitraums nach der Behandlung einsetzen. Der Zeitraum, über den eine Schutzwirkung erzielt wird, erstreckt sich im allgemeinen von 1 bis 10 Tagen, vorzugsweise 1 bis 7 Tagen, nach der Behandlung der Pflanzen mit den Wirkstoffen.

Zu Pflanzen und Pflanzensorten, die vorzugsweise erfindungsgemäß behandelt werden, zählen alle Pflanzen, die über Erbgut verfügen, das diesen Pflanzen besonders vorteilhafte, nützliche Merkmale verleiht (egal, ob dies durch Züchtung und/oder Biotechnologie erzielt wurde).

Pflanzen, die weiterhin vorzugsweise erfindungsgemäß behandelt werden, sind gegen einen oder mehrere biotische Streßfaktoren resistent, d. h. diese Pflanzen weisen eine verbesserte Abwehr gegen tierische und mikrobielle Schädlinge wie Nematoden, Insekten, Milben, phytopathogene Pilze, Bakterien, Viren und/oder Viroide auf.

Neben den vorgenannten Pflanzen und Pflanzensorten, können auch solche erfindungsgemäß behandelt werden, die gegen einen oder mehrere abiotische Streßfaktoren widerstandsfähig sind.

Zu den abiotischen Streßbedingungen können zum Beispiel Dürre, Kälte- und Hitzebedingungen, osmotischer Streß, Staunässe, erhöhter Bodensalzgehalt, erhöhtes Ausgesetztsein an Mineralien, Ozonbedingungen, Starklichtbedingungen, beschränkte Verfügbarkeit von Stickstoffnährstoffen, beschränkte Verfügbarkeit von Phosphornährstoffen oder Vermeidung von Schatten zählen.

Pflanzen und Pflanzensorten, die ebenfalls erfindungsgemäß behandelt werden können, sind solche Pflanzen, die durch erhöhte Ertragseigenschaften gekennzeichnet sind. Ein erhöhter Ertrag kann bei diesen Pflanzen z. B. auf verbesserter Pflanzenphysiologie, verbessertem Pflanzenwuchs und verbesserter Pflanzenentwicklung, wie Wasserverwertungseffizienz, Wasserhalteeffizienz, verbesserter Stickstoffverwertung, erhöhter Kohlenstoffassimilation, verbesserter Photosynthese, verstärkter Keimkraft und beschleunigter Abreife beruhen. Der Ertrag kann weiterhin durch eine verbesserte Pflanzenarchitektur (unter Streß- und nicht-Streß-Bedingungen) beeinflußt werden, darunter frühe Blüte, Kontrolle der Blüte für die Produktion von Hybridsaatgut, Keimpflanzenwüchsigkeit, Pflanzengröße, Internodienzahl und -abstand, Wurzelwachstum, Samengröße, Fruchtgröße, Schotengröße, Schoten- oder Ährenzahl, Anzahl der Samen pro Schote oder Ähre, Samenmasse, verstärkte Samenfüllung, verringerter Samenausfall, verringertes Schotenplatzen sowie Standfestigkeit. Zu weiteren Ertragsmerkmalen zählen Samenzusammensetzung wie Kohlenhydratgehalt, Proteingehalt, Ölgehalt und Ölzusammensetzung, Nährwert, Verringerung der nährwidrigen Verbindungen, verbesserte Verarbeitbarkeit und verbesserte Lagerfähigkeit.

Pflanzen, die erfindungsgemäß behandelt werden können, sind Hybridpflanzen, die bereits die Eigenschaften der Heterosis bzw. des Hybrideffekts exprimieren, was im allgemeinen zu höherem Ertrag, höherer Wüchsigkeit, besserer Gesundheit und besserer Resistenz gegen biotische und abiotische Streßfaktoren führt. Solche Pflanzen werden typischerweise dadurch erzeugt, daß man eine ingezüchtete pollensterile Elternlinie (den weiblichen Kreuzungspartner) mit einer anderen ingezüchteten pollenfertilen Elternlinie (dem männlichen Kreuzungspartner) kreuzt. Das Hybridsaatgut wird typischerweise von den pollensterilen Pflanzen geerntet und an Vermehrer verkauft. Pollensterile Pflanzen können manchmal (z. B. beim Mais) durch Entfahnen (d. h. mechanischem Entfernen der männlichen Geschlechtsorgane bzw. der männlichen Blüten), produziert werden; es ist jedoch üblicher, daß die Pollensterilität auf genetischen Determinanten im Pflanzengenom beruht. In diesem Fall, insbesondere dann, wenn es sich bei dem gewünschten Produkt, da man von den Hybridpflanzen ernten will, um die Samen handelt, ist es üblicherweise günstig, sicherzustellen, daß die Pollenfertilität in Hybridpflanzen, die die für die Pollensterilität verantwortlichen genetischen Determinanten enthalten, völlig restoriert wird. Dies kann erreicht werden, indem sichergestellt wird, daß die männlichen Kreuzungspartner entsprechende Fertilitätsrestorergene besitzen, die in der Lage sind, die Pollenfertilität in Hybridpflanzen, die die genetischen Determinanten, die für die Pollensterilität verantwortlich sind, enthalten, zu restorieren. Genetische Determinanten für Pollensterilität können im Cytoplasma lokalisiert sein. Beispiele für cytoplasmatische Pollensterilität (CMS) wurden zum Beispiel für Brassica-Arten beschrieben. Genetische Determinanten für Pollensterilität können jedoch auch im Zellkerngenom lokalisiert sein. Pollensterile Pflanzen können auch mit Methoden der pflanzlichen Biotechnologie, wie Gentechnik, erhalten werden. Ein besonders günstiges Mittel zur Erzeugung von pollensterilen Pflanzen ist in WO 89/10396 beschrieben, wobei zum Beispiel eine Ribonuklease wie eine Barnase selektiv in den Tapetumzellen in den Staubblättern exprimiert wird. Die Fertilität kann dann durch Expression eines Ribonukleasehemmers wie Barstar in den Tapetumzellen restoriert werden.

Pflanzen oder Pflanzensorten (die mit Methoden der Pflanzenbiotechnologie, wie der Gentechnik, erhalten werden), die erfindungsgemäß behandelt werden können, sind herbizidtolerante Pflanzen, d. h. Pflanzen, die gegenüber einem oder mehreren vorgegebenen Herbiziden tolerant gemacht worden sind. Solche Pflanzen können entweder durch genetische Transformation oder durch Selektion von Pflanzen, die eine Mutation enthalten, die solch eine Herbizidtoleranz verleiht, erhalten werden.

Herbizidtolerante Pflanzen sind zum Beispiel glyphosatetolerante Pflanzen, d. h. Pflanzen, die gegenüber dem Herbizid Glyphosate oder dessen Salzen tolerant gemacht worden sind. So können zum Beispiel glyphosatetolerante Pflanzen durch Transformation der Pflanze mit einem Gen, das für das Enzym 5-Enolpyruvylshikimat-3-phosphatsynthase (EPSPS) kodiert, erhalten werden. Beispiele für solche EPSPS-Gene sind das AroA-Gen (Mutante CT7) des Bakterium *Salmonella typhimurium*, das CP4-Gen des Bakteriums *Agrobacterium sp.,* die Gene, die für eine EPSPS aus der Petunie, für eine EPSPS aus der Tomate oder für eine EPSPS aus Eleusine kodieren. Es kann sich auch um eine mutierte EPSPS handeln. Glyphosatetolerante Pflanzen können auch dadurch erhalten werden, daß man ein Gen exprimiert, das für ein Glyphosate-Oxidoreduktase-Enzym kodiert. Glyphosatetolerante Pflanzen können auch dadurch erhalten werden, daß man ein Gen exprimiert, das für ein Glyphosate-acetyltransferase-Enzym kodiert. Glyphosatetolerante Pflanzen können auch dadurch erhalten werden, daß man Pflanzen, die natürlich vorkommende Mutationen der oben erwähnten Gene selektiert.

Sonstige herbizidresistente Pflanzen sind zum Beispiel Pflanzen, die gegenüber Herbiziden, die das Enzym Glutaminsynthase hemmen, wie Bialaphos, Phosphinotricin oder Glufosinate, tolerant gemacht worden sind. Solche Pflanzen können dadurch erhalten werden, daß man ein Enzym exprimiert, das das Herbizid oder eine Mutante des Enzyms Glutaminsynthase, das gegenüber Hemmung resistent ist, entgiftet. Solch ein wirksames entgiftendes Enzym ist zum Beispiel ein Enzym, das für ein Phosphinotricin-acetyltransferase kodiert (wie zum Beispiel das bar- oder pat-Protein aus Streptomyces-Arten). Pflanzen, die eine exogene Phosphinotricin-acetyltransferase exprimieren, sind beschrieben.

Weitere herbizidtolerante Pflanzen sind auch Pflanzen, die gegenüber den Herbiziden, die das Enzym Hydroxyphenylpyruvatdioxygenase (HPPD) hemmen, tolerant gemacht worden sind. Bei den Hydroxyphenylpyruvatdioxygenasen handelt es sich um Enzyme, die die Reaktion, in der para-Hydroxyphenylpyruvat (HPP) zu Homogentisat umgesetzt wird, katalysieren. Pflanzen, die gegenüber HPPD-Hemmern tolerant sind, können mit einem Gen, das für ein natürlich vorkommendes resistentes HPPD-Enzym kodiert, oder einem Gen, das für ein mutiertes HPPD-Enzym kodiert, transformiert werden. Eine Toleranz gegenüber HPPD-Hemmern kann auch dadurch erzielt werden, daß man Pflanzen mit Genen transformiert, die für gewisse Enzyme kodieren, die die Bildung von Homogentisat trotz Hemmung des nativen HPPD-Enzyms durch den HPPD-Hemmer ermöglichen. Die Toleranz von Pflanzen gegenüber HPPD-Hemmern kann auch dadurch verbessert werden, daß man Pflanzen zusätzlich zu einem Gen, das für ein HPPDtolerantes Enzym kodiert, mit einem Gen transformiert, das für ein Prephenatdehydrogenase-Enzym kodiert.

Weitere herbizidresistente Pflanzen sind Pflanzen, die gegenüber Acetolactatsynthase (ALS)-Hemmern tolerant gemacht worden sind. Zu bekannten ALS-Hemmern zählen zum Beispiel Sulfonylharnstoff, Imidazolinon, Triazolopyrimidine, Pyrimidinyloxy(thio)benzoate und/oder Sulfonylaminocarbonyltriazolinon-Herbizide. Es ist bekannt, daß verschiedene Mutationen im Enzym ALS (auch als Acetohydroxysäure-Synthase, AHAS, bekannt) eine Toleranz gegenüber unterschiedlichen Herbiziden bzw. Gruppen von Herbiziden verleihen. Die Herstellung von sulfonylharnstofftoleranten Pflanzen und imidazolinontoleranten Pflanzen ist in der internationalen Veröffentlichung WO 1996/033270 beschrieben. Weitere sulfonylharnstoff- und imidazolinontolerante Pflanzen sind auch in z.B. WO 2007/024782 beschrieben.

Weitere herbizidresistente Pflanzen sind Pflanzen, die gegenüber ACCase-Hemmern tolerant gemacht worden sind.

Weitere Pflanzen, die gegenüber Imidazolinon und/oder Sulfonylharnstoff tolerant sind, können durch induzierte Mutagenese, Selektion in Zellkulturen in Gegenwart des Herbizids oder durch Mutationszüchtung erhalten werden.

Pflanzen oder Pflanzensorten (die nach Methoden der pflanzlichen Biotechnologie, wie der Gentechnik, erhalten wurden), die ebenfalls erfindungsgemäß behandelt werden können, sind insektenresistente transgene Pflanzen, d.h. Pflanzen, die gegen Befall mit gewissen Zielinsekten resistent gemacht wurden. Solche Pflanzen können durch genetische Transformation oder durch Selektion von Pflanzen, die eine Mutation enthalten, die solch eine Insektenresistenz verleiht, erhalten werden.

Der Begriff "insektenresistente transgene Pflanze" umfaßt im vorliegenden Zusammenhang jegliche Pflanze, die mindestens ein Transgen enthält, das eine Kodiersequenz umfaßt, die für folgendes kodiert:
1) ein insektizides Kristallprotein aus *Bacillus thuringiensis* oder einen insektiziden Teil davon, wie die insektiziden Kristallproteine, die online bei: http://www.lifesci.sussex.ac.uk/Home/Neil Crickmore/Bt/ beschrieben sind, zusammengestellt wurden, oder insektizide Teile davon, z.B. Proteine der Cry-Proteinklassen Cry1Ab, Cry1Ac, Cry1F, Cry2Ab, Cry3Ae oder Cry3Bb oder insektizide Teile davon; oder
2) ein Kristallprotein aus *Bacillus thuringiensis* oder einen Teil davon, der in Gegenwart eines zweiten, anderen Kristallproteins als *Bacillus thuringiensis* oder eines Teils davon insektizid wirkt, wie das binäre Toxin, das aus den Kristallproteinen Cy34 und Cy35 besteht; oder
3) ein insektizides Hybridprotein, das Teile von zwei unterschiedlichen insektiziden Kristallproteinen aus *Bacillus thuringiensis* umfaßt, wie zum Beispiel ein Hybrid aus den Proteinen von 1) oben oder ein Hybrid aus den Proteinen von 2) oben, z. B. das Protein Cry1A.105, das von dem Mais-Event MON98034 produziert wird (WO 2007/027777); oder
4) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden, wie das Protein Cry3Bb1 in Mais-Events MON863 oder MON88017 oder das Protein Cry3A im Mais-Event MIR 604;
5) ein insektizides sezerniertes Protein aus *Bacillus thuringiensis* oder *Bacillus cereus* oder einen insektiziden Teil davon, wie die vegetativ wirkenden insektentoxischen Proteine (vegetative insekticidal proteins, VIP), die unter http://www.lifesci.sussex.ac.uk/Home/Neil_Crickmore/Bt/vip.html angeführt sind, z. B. Proteine der Proteinklasse VIP3Aa; oder
6) ein sezerniertes Protein aus *Bacillus thuringiensis* oder *Bacillus cereus,* das in Gegenwart eines zweiten sezernierten Proteins aus *Bacillus thuringiensis* oder *B. cereus* insektizid wirkt, wie das binäre Toxin, das aus den Proteinen VIP1A und VIP2A besteht.
7) ein insektizides Hybridprotein, das Teile von verschiedenen sezernierten Proteinen von *Bacillus thuringiensis* oder *Bacillus cereus* umfaßt, wie ein Hybrid der Proteine von 1) oder ein Hybrid der Proteine von 2) oben; oder
8) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden (wobei die Kodierung für ein insektizides Protein erhalten bleibt), wie das Protein VIP3Aa im Baumwoll-Event COT 102.

Natürlich zählt zu den insektenresistenten transgenen Pflanzen im vorliegenden Zusammenhang auch jegliche Pflanze, die eine Kombination von Genen umfaßt, die für die Proteine von einer der oben genannten Klassen 1 bis 8 kodieren. In einer Ausführungsform enthält eine insektenresistente Pflanze mehr als ein Transgen, das für ein Protein nach einer der oben genannten 1 bis 8 kodiert, um das Spektrum der entsprechenden Zielinsektenarten zu erweitern oder um die Entwicklung einer Resistenz der Insekten gegen die Pflanzen dadurch hinauszuzögern, daß man verschiedene Proteine einsetzt, die für dieselbe Zielinsektenart insektizid sind, jedoch eine unterschiedliche Wirkungsweise, wie Bindung an unterschiedliche Rezeptorbindungsstellen im Insekt, aufweisen.

Pflanzen oder Pflanzensorten (die nach Methoden der pflanzlichen Biotechnologie, wie der Gentechnik, erhalten wurden), die ebenfalls erfindungsgemäß behandelt werden können, sind gegenüber abiotischen Streßfaktoren tolerant. Solche Pflanzen können durch genetische Transformation oder durch Selektion von Pflanzen, die eine Mutation enthalten, die solch eine Streßresistenz verleiht, erhalten werden. Zu besonders nützlichen Pflanzen mit Streßtoleranz zählen folgende:
a. Pflanzen, die ein Transgen enthalten, das die Expression und/oder Aktivität des Gens für die Poly(ADP-ribose)polymerase (PARP) in den Pflanzenzellen oder Pflanzen zu reduzieren vermag.
b. Pflanzen, die ein streßtoleranzförderndes Transgen enthalten, das die Expression und/oder Aktivität der für PARG kodierenden Gene der Pflanzen oder Pflanzenzellen zu reduzieren vermag;
c. Pflanzen, die ein streßtoleranzförderndes Transgen enthalten, das für ein in Pflanzen funktionelles Enzym des Nicotinamidadenindinukleotid-Salvage-Biosynthesewegs kodiert, darunter Nicotinamidase, Nicotinatphosphoribosyltransferase, Nicotinsäuremononukleotidadenyltransferase, Nicotinamidadenindinukleotidsynthetase oder Nicotinamidphosphoribosyltransferase.

Pflanzen oder Pflanzensorten (die nach Methoden der pflanzlichen Biotechnologie, wie der Gentechnik, erhalten wurden), die ebenfalls erfindungsgemäß behandelt werden können, weisen eine veränderte Menge, Qualität und/oder Lagerfähigkeit des Ernteprodukts und/oder veränderte Eigenschaften von bestimmten Bestandteilen des Ernteprodukts auf, wie zum Beispiel:
1) Transgene Pflanzen, die eine modifizierte Stärke synthetisieren, die bezüglich ihrer chemisch-physikalischen Eigenschaften, insbesondere des Amylosegehalts oder des Amylose/Amylopektin-Verhältnisses, des Verzweigungsgrads, der durchschnittlichen Kettenlänge, der Verteilung der Seitenketten, des Viskositätsverhaltens, der Gelfestigkeit, der Stärkekorngröße und/oder Stärkekornmorphologie im Vergleich mit der synthetisierten Stärke in Wildtyppflanzenzellen oder -pflanzen verändert ist, so daß sich diese modifizierte Stärke besser für bestimmte Anwendungen eignet.
2) Transgene Pflanzen, die Nichtstärkekohlenhydratpolymere synthetisieren, oder Nichtstärkekohlenhydratpolymere, deren Eigenschaften im Vergleich zu Wildtyppflanzen ohne genetische Modifikation verändert sind. Beispiele sind Pflanzen, die Polyfructose, insbesondere des Inulin- und Levantyps, produzieren, Pflanzen, die alpha-1,4-Glucane produzieren, Pflanzen, die alpha-1,6-verzweigte alpha-1,4-Glucane produzieren und Pflanzen, die Alternan produzieren.
3) Transgene Pflanzen, die Hyaluronan produzieren.

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Baumwollpflanzen mit veränderten Fasereigenschaften. Solche Pflanzen können durch genetische Transformation oder durch Selektion von Pflanzen, die eine Mutation enthalten, die solche veränderten Fasereigenschaften verleiht, erhalten werden; dazu zählen:
a) Pflanzen wie Baumwollpflanzen, die eine veränderte Form von Cellulosesynthasegenen enthalten,
b) Pflanzen wie Baumwollpflanzen, die eine veränderte Form von rsw2- oder rsw3-homologen Nukleinsäuren enthalten;
c) Pflanzen wie Baumwollpflanzen mit einer erhöhten Expression der Saccharosephosphatsynthase;
d) Pflanzen wie Baumwollpflanzen mit einer erhöhten Expression der Saccharosesynthase;
e) Pflanzen wie Baumwollpflanzen bei denen der Zeitpunkt der Durchlaßsteuerung der Plasmodesmen an der Basis der Faserzelle verändert ist, z. B. durch Herunterregulieren der faserselektiven β-1,3-Glucanase;
f) Pflanzen wie Baumwollpflanzen mit Fasern mit veränderter Reaktivität, z. B. durch Expression des N-Acetylglucosamintransferasegens, darunter auch nodC, und von Chitinsynthasegenen.

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Raps oder verwandte Brassica-Pflanzen mit veränderten Eigenschaften der Ölzusammensetzung. Solche Pflanzen können durch genetische Transformation oder durch Selektion von Pflanzen, die eine Mutation enthalten, die solche veränderten Öleigenschaften verleiht, erhalten werden; dazu zählen:
a) Pflanzen wie Rapspflanzen, die Öl mit einem hohen Ölsäuregehalt produzieren;
b) Pflanzen wie Rapspflanzen, die Öl mit einem niedrigen Linolensäuregehalt produzieren.
c) Pflanzen wie Rapspflanzen, die Öl mit einem niedrigen gesättigten Fettsäuregehalt produzieren.

Besonders nützliche transgene Pflanzen, die erfindungsgemäß behandelt werden können, sind Pflanzen mit einem oder mehreren Genen, die für ein oder mehrere Toxine kodieren, sind die transgenen Pflanzen, die unter den folgenden Handelsbezeichnungen angeboten werden: YIELD GARD® (zum Beispiel Mais, Baumwolle, Sojabohnen), KnockOut® (zum Beispiel Mais), BiteGard® (zum Beispiel Mais), BT-Xtra® (zum Beispiel Mais), StarLink® (zum Beispiel Mais), Bollgard® (Baumwolle), Nucotn® (Baumwolle), Nucotn 33B® (Baumwolle), NatureGard® (zum Beispiel Mais), Protecta® und NewLeaf® (Kartoffel). Herbizidtolerante Pflanzen, die zu erwähnen sind, sind zum Beispiel Maissorten, Baumwollsorten und Sojabohnensorten, die unter den folgenden Handelsbezeichnungen angeboten werden: Roundup Ready® (Glyphosatetoleranz, zum Beispiel Mais, Baumwolle, Sojabohne), Liberty Link® (Phosphinotricintoleranz, zum Beispiel Raps), IMI® (Imidazolinontoleranz) und SCS® (Sylfonylharnstofftoleranz), zum Beispiel Mais. Zu den herbizidresistenten Pflanzen (traditionell auf Herbizidtoleranz gezüchtete Pflanzen), die zu erwähnen sind, zählen die unter der Bezeichnung Clearfield® angebotenen Sorten (zum Beispiel Mais).

Besonders nützliche transgene Pflanzen, die erfindungsgemäß behandelt werden können, sind Pflanzen, die Transformations-Events, oder eine Kombination von Transformations-Events, enthalten und die zum Beispiel in den Dateien von verschiedenen nationalen oder regionalen Behörden angeführt sind (siehe zum Beispiel http://gmoinfo.jrc.it/gmp_browse.aspx und http://www.agbios.com/dbase.php).

Die Bezeichnung "Wirkstoffe" bzw. "Verbindungen" schließt immer auch die hier genannten Wirkstoffkombinationen mit ein.

### Herstellungsbeispiele

### Beispiel I-a-1

### Verfahren A

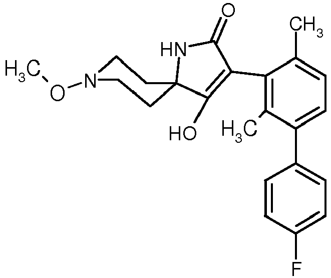

2.1 g (4.9 mmol) der Verbindung gemäß Bsp. II-1 werden in 5 ml N,N-Dimethylacetamid vorgelegt und bei 30-40°C 1.45 (12.3 mmol) Kalium-tert.-butylat in 5 ml N,N-Dimethylacetamid zugetropft. Es wird 6 Stunden bei 40°C nachgerührt.

Nach dünnschichtchromatographischer Probe wird das Reaktionsgemisch bei 0-10°C mit 1N Salzsäure auf pH 4 gebracht und die Mischung im Vakuum eingedampft. Der Rückstand wird mit n-Hexan/Isopropanol (Gradient) an Kieselgel chromatographiert.

Ausbeute: 1.05 g (50.6 % d. Theorie) Fp. Zersetzung

¹H-NMR (400 MHz, d₆-DMSO): δ = 1.46 (br, 2H, CH₂), 1.97, 2.12 (2s, je 3H, Ar-CH₃), 2.67-270 (mbr, 2H, CH₂-N), 3.44 (s, 3H, N-OCH₃), 7.03-7.05 (d, 1H, Ar-H) 7.10-7.12 (d, 1H, Ar-H), 7.22 - 7.32 (m, 4H, Ar-H), 8.22 (br, 1H, NH), 10.78 (br, 1H OH).

In Analogie zu Beispiel (I-a-1) und gemäß den allgemeinen Angaben zur Herstellung erhält man folgende Verbindungen der Formel (I-a):

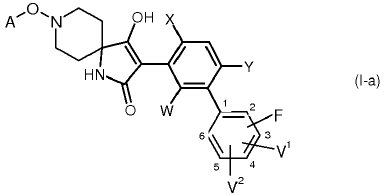

| **Bsp**.-**Nr**. | **W** | **X** | **Y** | **F** | **V¹** | **V²** | **A** | **Fp°C** |
|---|---|---|---|---|---|---|---|---|
| I-a-2 | CH₃ | CH₃ | H | 4 | H | H | C₂H₅- | 216 |
| I-a-3 | H | Cl | H | 4 | H | H | C₂H₅ | 172 |
| I-a-4 | H | Cl | H | 4 | H | H | CH₃ | 146 |
| I-a-5 | H | CH₃ | H | 4 | H | H | CH₃ | 358 |
| I-a-6 | H | CH₃ | H | 4 | H | H | C₂H₅ | |
| I-a-7 | Cl | OCH₃ | H | 4 | H | H | C₂H₅ | |
| I-a-8 | CH₃ | C₂H₅ | H | 4 | H | H | CH₃ | 131 |
| I-a-9 | CH₃ | C₂H₅ | H | 4 | H | H | C₂H₅ | 149 |
| I-a-10 | Cl | OCH₃ | H | 4 | H | H | CH₃ | 201 |
| I-a-11 | CH₃ | CH₃ | H | 4 | 3-F | H | C₂H₅ | 250 |
| I-a-12 | CH₃ | CH₃ | H | 4 | 3-Cl | H | C₂H₅ | 261 |
| I-a-13 | CH₃ | CH₃ | H | 4 | 3-F | H | CH₃ | 263 |
| I-a-14 | CH₃ | CH₃ | H | 3 | 4-Cl | H | C₂H₅ | 274 |
| I-a-15 | CH₃ | CH₃ | H | 3 | 4-Cl | H | CH₃ | 268 |

Analytik:
(I-a-2) ¹H NMR (600 MHz, CD₃OD): δ = 1.78 (t, 3H, CH₂CH₃), 1.60-1.65 (tm, 2H), 2.04, 2.20 (2s, je 3H, Ar-CH₃), 2.25-2.34 (qm, 2H), 2.75 (m, br, 2H), 3.35-3.38 (cm, 2H), 3.76-3.79 (m, 2H, OCH₂CH₃), 7.06-7.08 (d, 1H, ArH), 7.10-7.14 (m, 3H, ArH), 7.26-7.30 (m, 2H) ppm.
(I-a-3) ¹H NMR (600 MHz, CD₃OD): δ = 1.18 (t, 3H, CH₂CH₃), 1.63-1.67 (dm, 2H), 2.28 (ct. 2H), 2.75 (ct, 2H), 3.36-3.38 (dm, 2H), 3.77-3.79 (m, 2H, O-CH₂CH₃), 7.15-7.22 (m, 2H,ArH), 7.49-7.58 (m, 3H, ArH), 7.63-7.67 (m, 2H, ArH) ppm.
(I-a-4) ¹H NMR (400 MHz, d₆-DMSO): δ = 1.46-1.49 (dm, br, 2H), 2.12 (tm, br, 2H), 2.65-2.73 (m, br, 2H), 3.26-3.28 (dm, br, 2H), 3.44 (s, 3H, NOCH₃), 7.27-7.32 ("t", 2H, ArH), 7.47-7.61 (m, 3H, ArH), 7.68-7.71 (m, 2H, ArH), 8.31 (s, br, 1H, NH), 11.15 (s, 1H, OH) ppm.
(I-a-5) ¹H NMR (600 MHz, CD₃OD): δ = 1.84-1.87, 2.06-2.08 (2d, br, 2H), 2.25 (s, 3H, ArCH₃), 2.48-2.54, 2.62-2.66 (2tm, 2H), 3.53-3.57, 3.74-3.78 (2tm, 2H), 3.98-4.01, 4.08-4.10 (2dm, 2H), 4.02, 4.03 (2s, 3H, NOCH₃), 7.13-7.16 (tm, 2H, ArH), 7.34-7.37 (m, 2H, ArH), 7.48-7.50 (m, 1H, ArH), 7.60-7.63 (m, 2H, ArH) ppm.
(I-a-6) ¹H NMR (400 MHz, d₆-DMSO): δ = 1.11 (t, 3H, CH₂CH₃), 1.44-1.46 (dm, br, 2H), 2.18 (s und m, br, 5H, ArCH₃), 2.76 (cm, 2H), 3.20-3.22 (d, 2H), 3.64-3.69 (q, br, 2H, NOCH₂CH₃), 7.23-7.31 (m, 4H, ArH), 7.45-7.47 (m, 1H, ArH), 7.62-7.67 (m, 2H, ArH), 8.26 (s, br, 1H, NH), 10.86 (s, br, 1H, OH) ppm.
(I-a-7) ¹H NMR (400 MHz, d₆-DMSO): δ = 1.10 (t, 3H, CH₂CH₃), 1.42 (m, br, 2H), 2.05-2.09 (m, br, 2H), 2.66-2.71 (m, br, 2H), 3.19 (m, br, 2H), 3.63-3.68 (q, br, 2H, NOCH₂CH₃), 3.74 (s, 3H, ArOCH₃), 7.04-7.06 (d, 1H, ArH), 7.24-7.32 (m, 3H, ArH), 7.39-7.43 (m, 2H, ArH), 8.09 (s, br, 1H, NH), 10.83 (s, br, 1H, OH) ppm.
(I-a-8) ¹H NMR (400 MHz, d₆-DMSO): δ = 1.06 (t, 3H, CH₂CH₃), 1.42-1.45 (d, br, 2H), 1.96 (s, 3H, ArCH₃), 2.07-2.12 (br, 2H), 2.42-2.48 (q, 2H, ArCH₂CH₃), 2.64-2.70 (m, 2H), 3.44 (s, 3H, NOCH₃), 7.07-7.13 (m, 2H), 7.22-7.33 (m, 4H), 8.20 (s, br, 1H, NH), 10.8 (s, br, 1H, OH) ppm.
(I-a-9) ¹H NMR (400 MHz, d₆-DMSO): δ = 1.06, 1.11 (2t, je 3H, CH₂CH₃), 1.41-1.44 (d, br, 2H), 1.96 (s, 3H, ArCH₃), 2.14 (br, 2H), 2.44-2.47 (q, 2H, ArCH₂CH₃), 2.72 (m, br, 2H), 3.21 (m, br, 2H), 3.64-3.69 (q, br, 2H, NOCH₂CH₃), 7.07-7.13 (m, 2H, ArH), 7.22-7.33 (m, 4H, ArH), 8.21 (s, br, 1H, NH), 10.8 (s, br, 1H, OH) ppm.
(I-a-10) ¹H NMR (400 MHz, d₆-DMSO): δ = 1.43 (cm, br, 2H), 2.08 (cm, br, 2H), 2,67 (cm, br, 2H), 3.25 (cm, br, 2H), 3.43 (s, 3H, NOCH₃), 3.74 (s, 3H, ArOCH₃), 7.04-7.06 (d, 1H, ArH), 7.24-7.32 (m, 3H, ArH), 7.38-7.43 (m, 2H, ArH), 8.10 (s, br, 1H, NH), 10.83 (s, br, 1H, OH) ppm.
(I-a-11) ¹H NMR (400 MHz, d₆-DMSO): δ = 1.11 (t, 3H, CH₂CH₃), 1.44 (m, br, 2H), 1.98 (s, 3H, ArCH₃), 2.12 (s+mbr, 3+2H), 2.73 (m, br, 2H), 3.20-3.22 (m, br, 2H), 3.64-3.69 (q, br, 2H, OCH₂CH₃), 7.05-7.07 (d, 1H, ArH), 7.10-7.13 (m, 2H), 7.28-7.34 (m, 1H, ArH), 7.44-7.51 (m, 1H, ArH), 8.22 (s, br, 1H, NH), 10.79 (s, br, 1H, OH) ppm.
(I-a-12) ¹H NMR (400 MHz, d₆-DMSO): δ = 1.11 (t, 3H, CH₂CH₃), 1.45 (m, br, 2H), 1.97 (s, 3H, ArCH₃), 2.12 (s+mbr, 3+2H), 2.73 (m, br, 2H), 3.17-3.20 (m, br, 2H), 3.64-3.69 (q, br, 2H, OCH₂CH₃), 7.06-7.08 (d, 1H, ArH), 7.11-7.13 (d, 1H, ArH), 7.26-7.30 (m, 1H, ArH), 7.43-7.49 (m, 2H, ArH), 8.23 (s, br, 1H, NH), 10.78 (s, br, 1H, OH) ppm.
(I-a-13) ¹H NMR (400 MHz, d₆-DMSO): δ = 1.46 (m, br, 2H), 1.98 (s, 3H, ArCH₃), 2.12 (s+mbr, 3+2H), 2.66-2.70 (m, br, 2H), 3.25 (m, br, 2H), 3.44 (s, 3H, NOCH₃), 7.05-7.07 (d, 1H, ArH), 7.10-7.13 (m, 2H, ArH), 7.30-7.34 (m, 1H, ArH), 7.44-7.51 (m, 1H, ArH), 8.23 (s, br, 1H, NH), 10.79 (s, br, 1H, OH) ppm.
(I-a-14) ¹H NMR (400 MHz, d₆-DMSO): δ = 1.11 (t, 3H, CH₂CH₃), 1.43 (m, br, 2H), 1.99 (s, 3H, ArCH₃), 2.12 (s+mbr, 3H+2H), 2.65-2.68 (m, br, 2H), 3.20-3.22 (m, br, 2H), 3.64-3.69 (q, br, 2H, N-OCH₂CH₃), 7.07-7.09 (d, 1H, NH), 7.12-7.16 (m, 2H, ArH), 7.28-7.32 (dd, 1H, ArH), 7.63 (t, 1H, ArH), 8.22 (s, br, 1H, NH), 10.80 (s, br, 1H, OH) ppm.
(I-a-15) ¹H NMR (400 MHz, d₆-DMSO): δ = 1.46 (m, br, 2H), 1.99 (s, 3H, ArCH₃), 2.12 (s+mbr, 3H+2H), 2.64-2.70 (m, br, 2H), 3.44 (s, 3H, NOCH₃), 7.07-7.09 (d, 1H, ArH), 7.12-7.16 (m, 2H, ArH), 7.29-7.32 (dm, 1H. ArH), 7.61-7.65 (t, 1H, ArH), 8.23 (s, br, 1H, NH), 10.80 (s, br, OH) ppm.

### Beispiel II-1

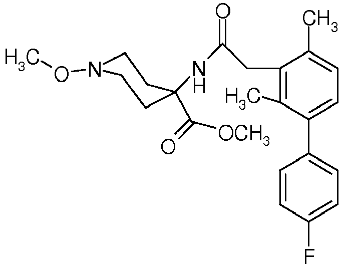

Unter Argon werden 2.5 g (11 mmol) 4-Amino-1-methoxy-piperidin-4-carbonsäuremethylester-Hydrochlorid und 100 ml wasserfreies Tetrahydrofuran vorgelegt.

Bei 20°C werden 6,4 ml (46 mmol) Trimethylamin zugetropft.

Man rührt 1 Stunde bei 50°C nach und versetzt bei 20°C mit 3,2 g (12.1 mmol) 2,6-Dimethyl-3-(4-fluorphenyl)-phenylessigsäure. Nach 15 Minuten tropft man 4,6 ml Trimethylamin (33 mmol) dazu und sofort danach 0,52 ml Phosphoroxychlorid (6,6 mmol), die Lösung soll mäßig sieden. Es wird 4 Stunden bei 40°C nachgerührt und die Reaktionsmischung auf ein Viertel des Volumens im Vakuum eingedampft, 100 ml gesättigte Natriumhydrogencarbonatlösung eingerührt, mit Methylenchlorid extrahiert, getrocknet und im Vakuum eingedampft.

Der Rückstand wird säulenchromatoghraphisch an Kieselgel gereinigt (n-Hexan: Isopropanol (Gradient)

Ausbeute: 2,15 g (36% d. Theorie), Fp. 131°C

¹H-NMR (400 MHz, CD₃CN): δ = 2.14, 2.33 (2s, je 3H, Ar-CH₃), 3.44 (s, 3H, NOCH₃), 3.58 (s, 3H, CO₂CH₃), 3.68 (s, 2H, CO-CH₂), 6.63 (br, 1H, NH), 7.00 -7.01 (d, 1H, ArH), 7.07-7.09 (d, 1H, Ar-H), 7.13-7.18 (m, 2H, Ar-H), 7.28-7.31(m, 2H, Ar-H).

In Analogie zu Beispiel (II-1) und gemäß den allgemeinen Angaben zur Herstellung erhält man folgende Verbindungen der Formel (II):

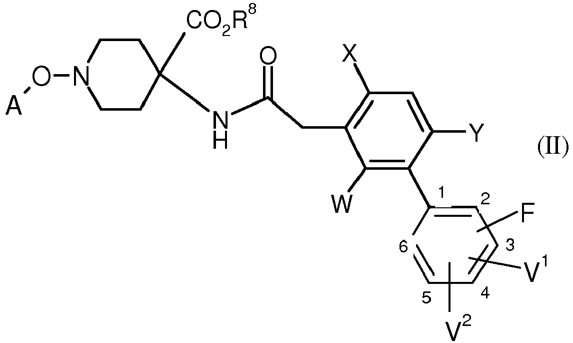

| **Bsp**.-**Nr**. | **W** | **X** | **Y** | **F** | **V¹** | **V²** | **A** | **R⁸** | **Fp** °C |
|---|---|---|---|---|---|---|---|---|---|
| II-2 | CH₃ | CH₃ | H | 4 | H | H | C₂H₅ | CH₃ | 160 |
| II-3 | H | CH₃ | H | 4 | H | H | CH₃ | CH₃ | 125 |
| II-4 | H | CH₃ | H | 4 | H | H | C₂H₅ | CH₃ | 129 |
| II-5 | H | Cl | H | 4 | H | H | C₂H₅ | CH₃ | 161 |
| II-6 | H | Cl | H | 4 | H | H | CH₃ | CH₃ | 153 |
| II-7 | CH₃ | C₂H₅ | H | 4 | H | H | CH₃ | CH₃ | 139 |
| II-8 | CH₃ | C₂H₅ | H | 4 | H | H | C₂H₅ | CH₃ | 75 |
| II-9 | Cl | OCH₃ | H | 4 | H | H | CH₃ | CH₃ | 157 |
| II-10 | Cl | OCH₃ | H | 4 | H | H | C₂H₅ | CH₃ | 167 |

### Beispiel I-a'-1

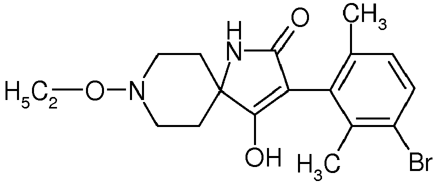

2.9 g (6.78 mmol) der Verbindung II'-1 werden in 10 ml N,N-Dimethylacetamid (DMA) vorgelegt. Bei 40°C tropft man eine Lösung aus 2.00 g (16.9 mmol) Kalium-tert.-butylat in 10 ml DMA zu und rührt 4 h bei 40°C weiter. Das Reaktionsgemisch wird mit 1N HCl auf pH4 eingestellt und das Lösungsmittel abgedampft. Der Rückstand wird mit einer Combi Flash Chromatographieanlage an Kieselgel mit einem Methylenchlorid/Isopropanol Gradienten gereinigt. Man erhält 2.1 g (≈ 71 % d. Theorie) der Verbindung I-a'-1 vom Fp. 201°C.

¹H NMR (400 MHz, d₆-DMSO): δ = 1.10 (t, 3H, CH₂CH₃), 1.43 (br, 2H), 2.04 (s, 3H, ArCH₃), 2.08 (br, 2H), 2.16 (s, 3H, Ar-CH₃), 2.67-2.74 (cm, br, 2H), 3.19-3.21 (d, br, 2H), 3.65-3.69 (m, br, 2H, OCH₂CH₃), 6.99-7.00 (d, 1H, ArH), 7.42-7.44 (d, 1H, ArH), 8.25 (br, 1H, NH), 10.92 (br, 1H, OH) ppm.

In Analogie zu Beispiel (I-a'-1) erhält man Beispiel (I-a'-2):

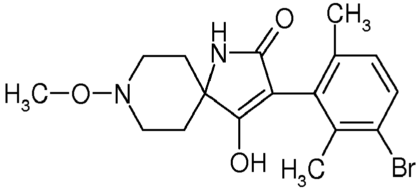

¹H NMR (600 MHz, d₆-DMSO): δ = 1.44-1.48 (cm, 2H), 2.04, 2.15 (2s, je 3H, ArCH₃), 2.08-2.13 (cm, 2H), 2.67-2.70 (cm, 2H), 3.26-3.27 (m, br, 2H), 3.44 (s, 3H, OCH₃), 6.99-7.01 (d, 1H, ArH), 7.43-7.44 (d, 1H, ArH), 8.31 (br, 1H, NH), 10.97 (br, 1H, OH) ppm.

### Beipiel II'-1

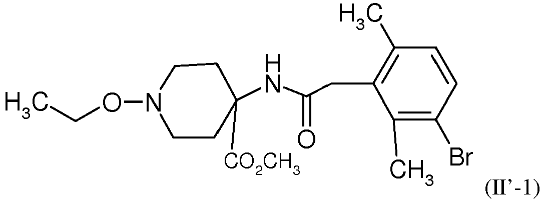

2.63 (11 mmol) 4-Amino-1-ethoxy-piperidin-4-carbonsäure-ethylester-hydrochlorid werden in 100 ml Tetrahydrofuran vorgelegt und 6.5 ml (46 mmol) Trimethylamin zugetropft. Man rührt eine Stunde bei 50°C, kühlt auf 20°C ab, versetzt mit 2.94 g (12 mmol) 3-Brom-2,6-dimethylphenyl-essigsäure, rührt 15 Min. nach, versetzt mit 4.6 ml (33 mmol) Trimethylamin und tropft sofort 0.52 ml (6.6 mmol) Phosphoroxychlorid zu und rührt anschließend 4 h bei 40°C nach. Man filtriert die Salze ab und dampft zur Trocknung ein. Der Rückstand wird auf einer Combi Flash Chromatographieanlage auf Kieselgel mit n-Hexan/Isopropanol (Gradient) getrennt. Man erhält 3.0 g (≈ 59 % d. Theorie) der Verbindung (II'-1) vom Schmelzpunkt Fp. 168°C.

¹H NMR (400 MHz, CD₃CN): δ = 1.09 (t, 3H, CH₂CH₃), 2.08-2.17 (m, br, 4H), 2.25, 2.38 (2s, je 3H, ArCH₃), 2.52 (cm, br, 2H), 3.11 (m, br, 2H), 3.58 (s, 3H, OCH₃), 3.64-3.70 (m+s, 4H, OCH₂, CH₂CO), 6.68 (br, 1H, NH), 6.93-6.95 (d, 1H, ArH), 7.36-7.38 (d, 1, ArH) ppm.

In Analogie zu Beispiel II'-1 erhält man Beispiel II'-2:

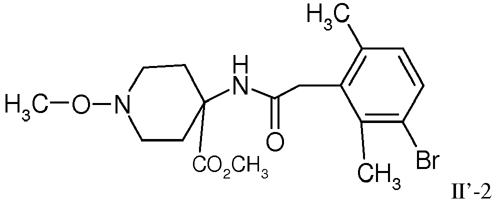

¹H NMR (400 MHz, CD₃CN): δ = 2.09-2.13 (m, 4H), 2.25, 2.38 (2s, je 3H, ArCH₃), 2.45-2.50 (cm, br, 2H), 3.16 (m, br, 2H), 3.45 (s, 3H, NOCH₃), 3.58 (s, 3H, CO₂CH₃), 3.67 (s, 2H, CH₂-CO), 6.67 (br, 1H, NH), 6.93-6.95 (d, 1H, ArH), 7.36-7.38 (d, 1H, ArH) ppm.

### Anwendungsbeispiele

### Beispiel 1

### Phaedon-Test (PHAECO Spritzbehandlung)

| | | |
|---|---|---|
| Lösungsmittel: | 78,0 | GewichtsteileAceton |
| | 1,5 | Gewichtsteile Dimethylformamid |
| | | |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Chinakohlblattscheiben (*Brassica pekinensis)* werden mit einer Wirkstoffzubereitung der gewünschten Konzentration gespritzt und nach dem Abtrocknen mit Larven des Meerrettichblattkäfers (*Phaedon cochleariae*) besetzt.

Nach 7 Tagen wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigen z. B. die folgenden Verbindungen der Herstellungsbeispiele Wirkung von
83 % bei einer Aufwandmenge von 500g/ha: Bsp Nr: I-a-1
100 % bei einer Aufwandmenge von 500 g/ha: Bsp Nr. I-a-2, I-a-3, I-a-8, I-a-9
100 % bei einer Aufwandmenge von 100 g/ha: Bsp Nr. I-a-6

### Beispiel 2

### Spodoptera frugiperda-Test (SPODFR Spritzbehandlung)

| | | |
|---|---|---|
| Lösungsmittel: | 78,0 | GewichtsteileAceton |
| | 1,5 | Gewichtsteile Dimethylformamid |
| | | |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Maisblattscheiben (*Zea mays*) werden mit einer Wirkstoffzubereitung der gewünschten Konzentration gespritzt und nach dem Abtrocknen mit Raupen des Heerwurms (*Spodoptera frugiperda*) besetzt.

Nach 7 Tagen wird die Wirkung in % bestimmt. Dabei bedeutet 100 %, dass alle Raupen abgetötet wurden; 0 % bedeutet, dass keine Raupe abgetötet wurde.

Bei diesem Test zeigen z. B. die folgenden Verbindungen der Herstellungsbeispiele Wirkung von
83 % bei einer Aufwandmenge von 500g/ha: Bsp Nr: I-a-1, I-a-3, I-a-4, I-a-12
100 % bei einer Aufwandmenge von 500 g/ha: Bsp Nr. I-a-2, I-a-5, I-a-6, I-a-14, I-a-15

### Beispiel 3

### Myzus-Test (MYZUPE Spritzbehandlung)

| | | |
|---|---|---|
| Lösungsmittel: | 78 | GewichtsteileAceton |
| | 1,5 | Gewichtsteile Dimethylformamid |
| | | |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Chinakohlblattscheiben (*Brassica pekinensis*)*,* die von allen Stadien der Grünen Pfirsichblattlaus (*Myzus persicae*) befallen sind, werden mit einer Wirkstoffzubereitung der gewünschten Konzentration gespritzt. Nach 6 Tagen wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele Wirkung von
90 % bei einer Aufwandmenge von 500g/ha: Bsp Nr: I-a-1, I-a-8, I-a-9
100 % bei einer Auwandmenge von 500 g/ha: Bsp Nr. I-a-2, I-a-3, I-a-4, I-a-5, I-a-6, I-a-7, I-a-10, I-a-11, I-a-12, I-a-13, I-a-14, I-a-15,

### Beispiel 4

### Tetranychus-Test; OP-resistent (TETRUR Spritzbehandlung)

| | | |
|---|---|---|
| Lösungsmittel: | 78,0 | GewichtsteileAceton |
| | 1,5 | Gewichtsteile Dimethylformamid |
| | | |
| Emulgator : | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Bohnenblattscheiben (*Phaseolus vulgaris*), die von allen Stadien der Gemeinen Spinnmilbe (*Tetranychus urticae*) befallen sind, werden mit einer Wirkstoffzubereitung der gewünschten Konzentration gespritzt.

Nach 6 Tagen wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigen z. B. die folgenden Verbindungen der Herstellungsbeispiele eine Wirkung von 90 % bei einer Aufwandmenge von 100g/ha: Bsp Nr: I-a-3, I-a-5, I-a-6, I-a-7, I-a-8, I-a-9, I-a-10, I-a-14, von 100 % bei einer Aufwandmenge von 100 g/ha: Bsp Nr. I-a-2, I-a-4, I-a-11, I-a-12, I-a-13, I-a-15

### Beispiel 5

### Boophilus microplus -Test (BOOPMI Injektion)

### Lösungsmittel: Dimethylsulfoxid

Zur Herstellung einer zweckmäßigen Wirkstoffzubereitung vermischt man 10 mg Wirkstoff mit 0,5 ml Lösungsmittel und verdünnt das Konzentrat mit Lösungsmittel auf die gewünschte Konzentration. Die Wirkstofflösung wird in das Abdomen *(Boophilus microplus)* injiziert, die Tiere werden in Schalen überführt und in einem klimatisierten Raum aufbewahrt. Die Wirkungskontrolle erfolgt auf Ablage fertiler Eier.

Nach 7 Tagen wird die Wirkung in % bestimmt. Dabei bedeutet 100%, dass keine Zecke fertile Eier gelegt hat.

Bei diesem Test zeigen z.B. die folgenden Verbindungen der Herstellungsbeispiele eine Wirkung von 90% bei einer Aufwandmenge von 20µg / Tier : I-a-2

Bei diesem Test zeigen z.B. die folgenden Verbindungen der Herstellungsbeispiele eine Wirkung von 95% bei einer Aufwandmenge von 20µg / Tier : I-a-1

### Beispiel 6

### Lucilia cuprina-Test (LUCICU)

### Lösungsmittel: Dimethylsulfoxid

Zur Herstellung einer zweckmäßigen Wirkstoffzubereitung vermischt man 10 mg Wirkstoff mit 0,5 ml Dimethylsulfoxid und verdünnt das Konzentrat mit Wasser auf die gewünschte Konzentration. Gefäße, die Pferdefleisch enthalten, das mit der Wirkstoffzubereitung der gewünschten Konzentration behandelt wurde, werden mit ca 20 *Lucilia cuprina* Larven besetzt.

Nach 2 Tagen wird die Abtötung in % bestimmt. Dabei bedeutet 100 %, dass alle Larven abgetötet wurden; 0 % bedeutet, dass keine Larven abgetötet wurden.

Bei diesem Test zeigen z.B. die folgenden Verbindungen der Herstellungsbeispiele eine Wirkung von 80% bei einer Aufwandmenge von 100 ppm: I-c-2

Bei diesem Test zeigen z.B. die folgenden Verbindungen der Herstellungsbeispiele eine Wirkung von 90% bei einer Aufwandmenge von 100 ppm : I-a-1, I-a-2

### Beispiel 7

### 1. Herbizide Wirkung im Vorauflauf

Samen von mono- bzw. dikotylen Unkraut- bzw. Kulturpflanzen werden in Holzfasertöpfen in sandiger Lehmerde ausgelegt und mit Erde abgedeckt. Die in Form von benetzbaren Pulvern (WP) formulierten Testverbindungen werden dann als wässrige Suspension mit einer Wasseraufwandmenge von umgerechnet 600 l/ha unter Zusatz von 0,2% Netzmittel in unterschiedlichen Dosierungen auf die Oberfläche der Abdeckerde appliziert.

Nach der Behandlung werden die Töpfe im Gewächshaus aufgestellt und unter guten Wachstumsbedingungen für die Testpflanzen gehalten. Die visuelle Bonitur der Auflaufschäden an den Versuchspflanzen erfolgt nach einer Versuchszeit von ca. 3 Wochen im Vergleich zu unbehandelten Kontrollen (herbizide Wirkung in Prozent: 100% Wirkung = Pflanzen sind abgestorben, 0 % Wirkung = wie Kontrollpflanzen).

Folgende Verbindungen zeigen neben den zuvor genannten Verbindungen im Vorauflauf mit 320 g/ha a.i. gegen Lolium multiflorum und Setaria viridis eine Wirkung von 90 %: I-1-a-1

Folgende Verbindungen zeigen neben den zuvor genannten Verbindungen in Vorauflauf mit 320 g/ha a.i. gegen Echinocloa crus-galli eine Wirkung von 100 %: I-a-2,

### 2. Herbizide Wirkung im Nachauflauf

Samen von mono- bzw. dikotylen Unkraut- bzw. Kulturpflanzen werden in Holzfasertöpfen in sandigem Lehmboden ausgelegt, mit Erde abgedeckt und im Gewächshaus unter guten Wachstumsbedingungen angezogen. 2-3 Wochen nach der Aussaat werden die Versuchspflanzen im Einblattstadium behandelt. Die als Spritzpulver (WP) formulierten Testverbindungen werden in verschiedenen Dosierungen mit einer Wasseraufwandmenge von umgerechnet 600 l/ha unter Zusatz von 0,2% Netzmittel auf die grünen Pflanzenteile gesprüht. Nach ca. 3 Wochen Standzeit der Versuchspflanzen im Gewächshaus unter optimalen Wachstumsbedingungen wird die Wirkung der Präparate visuell im Vergleich zu unbehandelten Kontrollen bonitiert (herbizide Wirkung in Prozent: 100% Wirkung = Pflanzen sind abgestorben, 0 % Wirkung = wie Kontrollpflanzen).

Neben den zuvor genannten Verbindungen zeigen folgende Verbindungen im Nachauflauf mit 80 g/ha gegen Echinocloa crus-galli und Lolium multiflorum eine Wirkung von 90%: I-a-3, I-a-4, I-a-5.

### Beispiel 8: Steigerung der Penetration in die Pflanze durch Ammonium- oder Phosphoniumsalze und synergistische Steigerung der Penetration in die Pflanze durch Ammoniumsalze in Kombination mit Penetrationsförderern

In diesem Test wurde die Penetration von Wirkstoffen durch enzymatisch isolierte Kutikeln von Apfelbaumblättern gemessen.

Verwendet wurden Blätter, die in voll entwickeltem Zustand von Apfelbäumen der Sorte Golden Delicious abgeschnitten wurden. Die Isolierung der Kutikeln erfolgte in der Weise, dass
- zunächst auf der Unterseite mit Farbstoff markierte und ausgestanzte Blattscheiben mittels Vakuuminfiltration mit einer auf einen pH Wert zwischen 3 und 4 gepufferten Pectinase-Lösung (0,2 bis 2 %ig) gefüllt wurden,
- dann Natriumazid hinzugefügt wurde und
- die so behandelten Blattscheiben bis zur Auflösung der ursprünglichen Blattstruktur und zur Ablösung der nicht zellulären Kutikula stehen gelassen wurden.

Danach wurden nur die von Spaltöffnungen und Haaren freien Kutikeln der Blattoberseiten weiter verwendet. Sie wurden mehrfach abwechselnd mit Wasser und einer Pufferlösung vom pH Wert 7 gewaschen. Die erhaltenen sauberen Kutikel wurden schließlich auf Teflonplättchen aufgezogen und mit einem schwachen Luftstrahl geglättet und getrocknet.

Im nächsten Schritt wurden die so gewonnenen Kutikelmembranen für Membran-Transport-Untersuchungen in Diffusionszellen (= Transportkammern) aus Edelstahl eingelegt. Dazu wurden die Kutikeln mit einer Pinzette mittig auf die mit Silikonfett bestrichenen Ränder der Diffusionszellen plaziert und mit einem ebenfalls gefetteten Ring verschlossen. Die Anordnung war so gewählt worden, dass die morphologische Außenseite der Kutikeln nach außen, also zur Luft, gerichtet war, während die ursprüngliche Innenseite dem Inneren der Diffusionszelle zugewandt war.

Die Diffusionszellen waren mit einer 30%igen Ethylenglykol/Wasser-Lösung befüllt. Zur Bestimmung der Penetration wurden jeweils 10 µl der Spritzbrühe der nachstehenden Zusammensetzung auf die Außenseite der Kutikula appliziert. Das Ansetzen der Spritzbrühe erfolgt mit lokalem Leitungswasser mittlerer Wasserhärte.

Nach dem Auftragen der Spritzbrühen ließ man das Wasser verdunsten, drehte die Kammern um und stellte sie in thermostatisierte Wannen, in denen die Temperatur und Luftfeuchte über der Kutikula durch einen leichten Luftstrom auf die Kutikula mit dem Spritzbelag einstellbar war (20°C, 60 % rh). In regelmäßigen Abständen wurden von einem Autosampler Aliquots entnommen und der Wirkstoffgehalt mit HPLC bestimmt.

Die Versuchsergebnisse gehen aus der folgenden Tabelle hervor. Bei den angegebenen Zahlen handelt es sich um Durchschnittswerte aus 8 bis 10 Messungen. Deutlich ist zu sehen, dass bereits Ammoniumsulfat alleine die Penetration deutlich verbessert und zusammen mit RME ein überadditiver (synergistischer) Effekt auftritt.

**Tabelle**

| Wirkstoff | Penetration nach 24h / % | | | |
|---|---|---|---|---|
| | EC | EC + AS (1 g/l) | EC + RME (1 g/l) | EC + RME (1 g/l) + AS (1 g/l) |
| Beispiel I-a-1 300 ppm in DMF / Emulgator W 7:1 (w/w) | < 0,2 | 3,8 | 0,8 | 19 |
| | EC | EC+DAHP (1 g/l) | EC+RME (1 g/l) | EC+RME (1 g/l) + DAHP (1 g/l) |
| | < 0,2 | 2,3 | 0,8 | 15 |

| | | | | |
|---|---|---|---|---|
| RME = Rapsölmethylester (Einsatz formuliert als 500 EW, Konzentrationsangabe in g Wirkstoff /1) | | | | |

### AS = Ammoniumsulfat

### DAHP = Diammonium-hydrogenphosphat

### EC = Emulgierbares Konzentrat

**Tabelle**

| Wirkstoff | Penetration nach 24h / % | | | |
|---|---|---|---|---|
| | EC | EC + AS (1 g/l) | EC + RME (1 g/l) | EC + RME (1 g/l) + AS (1 g/l) |
| Beispiel I-1-a-2 300 ppm in DMF / Emulgator W 7:1 (w/w) | 1,4 | 3,8 | 1,7 | 17 |
| | EC | EC+DAHP (1 g/l) | EC+RME (1 g/l) | EC+RME (1 g/l) + DAHP (1 g/l) |
| | 1,4 | 3,9 | 1,7 | 21 |

### Beispiel 9: Wirkungssteigerung durch Ammoniumsalze in Kombination mit Penetrationsförderern

### Myzus persicae -Test

| | | |
|---|---|---|
| Lösungsmittel: | 7 | Gewichtsteile Dimethylformamid |
| Emulgator: | 2 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration. Für die Anwendung mit Ammoniumsalzen und Penetrationsförderer (Rapsölmethylester 500 EW) werden diese jeweils in einer Konzentration von 1000 ppm a.i. der Spritzbrühe hinzugegeben.

Paprikapflanzen (*Capsicum annuum*), die stark von der Grünen Pfirsichblattlaus (*Myzus persicae*) befallen sind, werden durch Tropfnassspritzung mit der Wirkstoffzubereitung in der gewünschten Konzentration behandelt. Nach der gewünschten Zeit wird die Abtötung in % bestimmt. Dabei bedeutet 100 %, dass alle Tiere abgetötet wurden; 0 % bedeutet, dass keine Tiere abgetötet wurden.

**Tabelle**

| Wirkstoff | Konzentration / ppm | Abtötungsgrad / % nach 6 Tagen | | | |
|---|---|---|---|---|---|
| | | | + AS (1000 ppm) | + RME (1000 ppm) | + RME + AS (je 1000 ppm) |
| | | | | | |
| I-a-1 | 20 | 60 | 90 | 95 | 100 |
| | 4 | 0 | 30 | 55 | 100 |
| I-a-2 | 4 | 0 | 20 | 95 | 95 |
| | 0.8 | 0 | 0 | 0 | 40 |

### Beispiel 10

### Aphis gossypii -Test

| | |
|---|---|
| Lösungsmittel: | 7 Gewichtsteile Dimethylformamid |
| Emulgator: | 2 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Für die Anwendung mit Ammoniumsalzen und Penetrationsförderern (Rapsölmethylestern 500 EW) werden diese jeweils in einer Konzentration von 1000 ppm a.i. der Spritzbrühe hinzugegeben.

Baumwollpflanzen (*Gossypium hirsutum*), die stark von der Baumwollblattlaus (*Aphis gossypii)* befallen sind, werden durch Tropfnassspritzung mit der Wirkstoffzubereitung der gewünschten Konzentration behandelt.

Nach der gewünschten Zeit wird die Abtötung in % bestimmt. Dabei bedeutet 100 %, dass alle Blattläuse abgetötet wurden; 0 % bedeutet, dass keine Blattläuse abgetötet wurden.

**Tabelle**

| Wirkstoff | Konzentration / ppm | Abtötungsgrad / % nach 6 Tagen | | | |
|---|---|---|---|---|---|
| | | | + AS (1000 ppm) | + RME (1000 ppm) | + RME + AS (je 1000 ppm) |
| I-a-1 | 20 | 0 | 40 | 55 | 100 |
| I-a-2 | 20 | 0 | 25 | 20 | 90 |
| | 4 | 0 | 0 | 0 | 45 |

## Patentansprüche

1. Verbindungen der Formel (I-a) 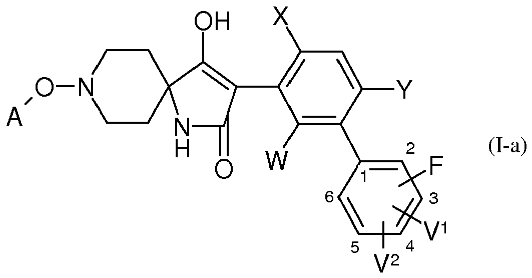 in welcher
A, W, X, Y, V und V² die folgenden Bedeutungen haben
**Tabelle 1**
| **A** | **W** | **X** | **Y** | **F** | **V¹** | **V²** |
|---|---|---|---|---|---|---|
| CH₃ | CH₃ | CH₃ | H | 2 | H | H |
| CH₃ | CH₃ | CH₃ | H | 3 | H | H |
| CH₃ | CH₃ | CH₃ | H | 4 | H | H |
| CH₃ | CH₃ | CH₃ | H | 2 | 4-F | H |
| CH₃ | CH₃ | CH₃ | H | 2 | 4-Cl | H |
| CH₃ | CH₃ | CH₃ | H | 2 | 4-CH₃ | H |
| CH₃ | CH₃ | CH₃ | H | 2 | 4-OCH₃ | H |
| CH₃ | CH₃ | CH₃ | H | 3 | 4-F | H |
| CH₃ | CH₃ | CH₃ | H | 3 | 4-Cl | H |
| CH₃ | CH₃ | CH₃ | H | 3 | 4-CH₃ | H |
| CH₃ | CH₃ | CH₃ | H | 3 | 4-OCH₃ | H |
| CH₃ | CH₃ | CH₃ | H | 4 | 3-Cl | H |
| CH₃ | CH₃ | CH₃ | H | 4 | 3-CH₃ | H |
| CH₃ | CH₃ | CH₃ | H | 4 | 3-OCH₃ | H |
| CH₃ | CH₃ | CH₃ | H | 2 | 4-F | 5-F |
| CH₃ | CH₃ | CH₃ | H | 2 | 4-F | 6-F |
| CH₃ | CH₃ | CH₃ | H | 2 | 4-Cl | 5-F |
| CH₃ | CH₃ | CH₃ | H | 2 | 5-Cl | 4-F |
| CH₃ | CH₃ | CH₃ | H | 3 | 4-F | 5-F |
| CH₃ | CH₃ | CH₃ | CH₃ | 2 | H | H |
| CH₃ | CH₃ | CH₃ | CH₃ | 3 | H | H |
| CH₃ | CH₃ | CH₃ | CH₃ | 4 | H | H |
| CH₃ | CH₃ | CH₃ | CH₃ | 2 | 4-F | H |
| CH₃ | CH₃ | CH₃ | CH₃ | 2 | 4-Cl | H |
| CH₃ | CH₃ | CH₃ | CH₃ | 2 | 4-CH₃ | H |
| CH₃ | CH₃ | CH₃ | CH₃ | 2 | 4-OCH₃ | H |
| CH₃ | CH₃ | CH₃ | CH₃ | 3 | 4-F | H |
| CH₃ | CH₃ | CH₃ | CH₃ | 3 | 4-Cl | H |
| CH₃ | CH₃ | CH₃ | CH₃ | 3 | 4-CH₃ | H |
| CH₃ | CH₃ | CH₃ | CH₃ | 3 | 4-OCH₃ | H |
| CH₃ | CH₃ | CH₃ | CH₃ | 4 | 3-Cl | H |
| CH₃ | CH₃ | CH₃ | CH₃ | 4 | 3-CH₃ | H |
| CH₃ | CH₃ | CH₃ | CH₃ | 4 | 3-OCH₃ | H |
| CH₃ | CH₃ | CH₃ | CH₃ | 2 | 4-F | 5-F |
| CH₃ | CH₃ | CH₃ | CH₃ | 2 | 4-F | 6-F |
| CH₃ | CH₃ | CH₃ | CH₃ | 2 | 4-Cl | 5-F |
| CH₃ | CH₃ | CH₃ | CH₃ | 2 | 5-Cl | 4-F |
| CH₃ | CH₃ | CH₃ | CH₃ | 3 | 4-F | 5-F |
Tabelle 2 W, X, Y, F, V¹ und V² wie in Tabelle 1 angegeben A = C₂H₅
**Tabelle 3 W, X, Y, F, V¹ und V² wie in Tabelle 1 angegeben A = C₃H₇**
| **W** | **X** | **Y** | **F** | **V¹** | **V²** | **A** |
|---|---|---|---|---|---|---|
| Cl | OCH₃ | H | 4 | H | H | C₂H₅ |
| CH₃ | C₂H₅ | H | 4 | H | H | CH₃ |
| CH₃ | C₂H₅ | H | 4 | H | H | C₂H₅ |
| Cl | OCH₃ | H | 4 | H | H | CH₃ |
| CH₃ | CH₃ | H | 4 | 3-F | H | C₂H₅ |
| CH₃ | CH₃ | H | 4 | 3-F | H | CH₃ |

2. Verfahren zur Herstellung von Verbindungen der Formel (I-a) gemäß Anspruch 1, **dadurch gekennzeichnet, dass** man zum Erhalt von
(A) Verbindungen der Formel (I-a) 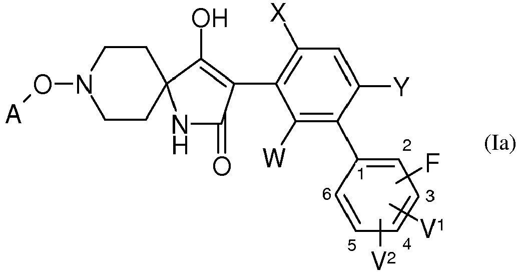 in welcher
A, W, X, Y, V¹ und V² die oben angegebenen Bedeutungen haben,
Verbindungen der Formel (II) 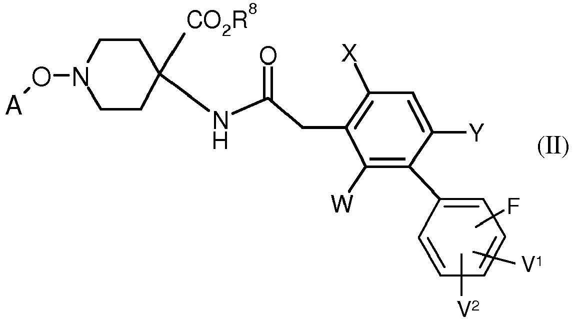 in welcher
A, W, X, Y , V¹ und V² die oben angegebenen Bedeutungen haben,
und R⁸ für Alkyl steht,
in Gegenwart eines Verdünnungsmittels und in Gegenwart einer Base intramolekular kondensiert,
(I) Verbindungen der oben gezeigten Formel (I-a) , in welcher A, W, X, Y , V¹ und V² die oben angegebene Bedeutung haben, Verbindungen der Formel (I-a') , in welcher A, W, X und Y die oben genannte Bedeutung haben und Z' bevorzugt für Brom oder Iod steht 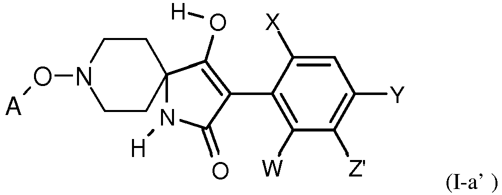
mit kupplungsfähigen (Het)-arylderivaten, z.B. Phenylboronsäuren der Formeln (XIII) 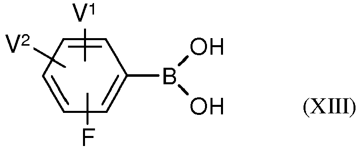 in welcher V ¹ und V² die oben angegebenen Bedeutungen hat
oder deren Ester in Gegenwart eines Lösungsmittels, in Gegenwart eines Katalysators und in Gegenwart einer Base kuppelt.

3. Schädlingsbekämpfungsmittel und/oder Herbizide und/oder Fungizide, **gekennzeichnet durch** einen Gehalt an mindestens einer Verbindung der Formel (I-a) gemäß Anspruch 1.

4. Verfahren zur Bekämpfung von tierischen Schädlingen und/oder unerwünschtem Pflanzenbewuchs und/oder Pilzen, **dadurch gekennzeichnet, dass** man Verbindungen der Formel (I-a) gemäß Anspruch 1 auf Schädlinge und/oder ihren Lebensraum einwirken lässt, ausgenommen sind Verfahren zur chirurgischen und therapeutischen Behandlung des menschlichen und tierischen Körpers

5. Verwendung von Verbindungen der Formel (I-a) gemäß Anspruch 1 zur Bekämpfung von tierischen Schädlingen und/oder unerwünschtem Pflanzenbewuchs und/oder Pilzen, ausgenommen sind Verfahren zur chirurgischen und therapeutischen Behandlung des menschlichen und tierischen Körpers.

6. Verfahren zur Herstellung von Schädlingsbekämpfungsmitteln und/oder Herbiziden und/oder Fungiziden, **dadurch gekennzeichnet, dass** man Verbindungen der Formel (I-a) gemäß Anspruch 1 mit Streckmitteln und/oder oberflächenaktiven Stoffen vermischt.

7. Verwendung von Verbindungen der Formel (I-a) gemäß Anspruch 1 zur Herstellung von Schädlingsbekämpfungsmitteln und/oder Herbiziden und/oder Fungiziden.

8. Mittel enthaltend einen wirksamen Gehalt an einer Wirkstoffkombination umfassend als Komponenten.
a') mindestens eine Verbindung der Formel (I-a), in welcher A, W, X, Y , V¹ und V² die oben angegebene Bedeutung haben und
(b') zumindest eine die Kulturpflanzen-Verträglichkeit verbessernde Verbindung aus der folgenden Gruppe von Verbindungen:
S1) Verbindungen der Formel (S1), 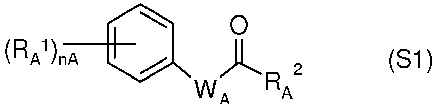 wobei die Symbole und Indizes folgende Bedeutungen haben:
n_{A} ist eine natürliche Zahl von 0 bis 5,
R_{A}¹ ist Halogen, (C₁-C₄)Alkyl, (C₁-C₄)Alkoxy, Nitro oder (C₁-C₄)Haloalkyl;
W_{A} ist ein unsubstituierter oder substituierter divalenter heterocyclischer Rest aus der Gruppe der teilungesättigten oder aromatischen Fünfring-Heterocyclen mit 1 bis 3 Heteroringatomen aus der Gruppe N und O, wobei mindestens ein N-Atom und höchstens ein O-Atom im Ring enthalten ist,
R_{A}² ist OR_{A}³, SR_{A}³ oder NR_{A}³R_{A}⁴ oder ein gesättigter oder ungesättigter 3- bis 7-gliedriger Heterocyclus mit mindestens einem N-Atom und bis zu 3 Heteroatomen, vorzugsweise aus der Gruppe O und S, der über das N-Atom mit der Carbonylgruppe in (S1) verbunden ist und unsubstituiert oder durch Reste aus der Gruppe (C₁-C₄)Alkyl, (C₁-C₄)Alkoxy oder gegebenenfalls substituiertes Phenyl substituiert ist, vorzugsweise ein Rest der Formel OR_{A}³, NHR_{A}⁴ oder N(CH₃)₂, insbesondere der Formel OR_{A}³;
R_{A}³ ist Wasserstoff oder ein unsubstituierter oder substituierter aliphatischer Kohlenwasserstoffrest, vorzugsweise mit insgesamt 1 bis 18 C-Atomen;
R_{A}⁴ ist Wasserstoff, (C₁-C₆)Alkyl, (C₁-C₆)Alkoxy oder substituiertes oder unsubstituiertes Phenyl;
S2) Chinolinderivate der Formel (S2), 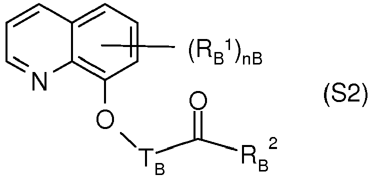 wobei die Symbole und Indizes folgende Bedeutungen haben:
R_{B}¹ ist Halogen, (C₁-C₄)Alkyl, (C₁-C₄)Alkoxy, Nitro oder (C₁-C₄)Haloalkyl;
n_{B} ist eine natürliche Zahl von 0 bis 5, vorzugsweise 0 bis 3;
R_{B}² ist OR_{B}³, SR_{B}³ oder NR_{B}³R_{B}⁴ oder ein gesättigter oder ungesättigter 3- bis 7-gliedriger Heterocyclus mit mindestens einem N-Atom und bis zu 3 Heteroatomen, vorzugsweise aus der Gruppe O und S, der über das N-Atom mit der Carbonylgruppe in (S2) verbunden ist und unsubstituiert oder durch Reste aus der Gruppe (C₁-C₄)Alkyl, (C₁-C₄)Alkoxy oder gegebenenfalls substituiertes Phenyl substituiert ist, vorzugsweise ein Rest der Formel OR_{B}³, NHR_{B}⁴ oder N(CH₃)₂, insbesondere der Formel OR_{B}³;
R_{B}³ ist Wasserstoff oder ein unsubstituierter oder substituierter aliphatischer Kohlenwasserstoffrest, vorzugsweise mit insgesamt 1 bis 18 C-Atomen;
R_{B}⁴ ist Wasserstoff, (C₁-C₆)Alkyl, (C₁-C₆)Alkoxy oder substituiertes oder unsubstituiertes Phenyl;
T_{B} ist eine (C₁ oder C₂)-Alkandiylkette, die unsubstituiert oder mit einem oder zwei (C₁-C₄)Alkylresten oder mit [(C₁-C₃)-Alkoxy]-carbonyl substituiert ist;
S3) Verbindungen der Formel (S3) 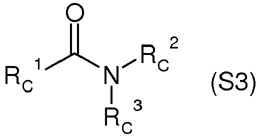 wobei die Symbole und Indizes folgende Bedeutungen haben:
R_{C}¹ ist (C₁-C₄)Alkyl, (C₁-C₄)Haloalkyl, (C₂-C₄)Alkenyl, (C₂-C₄)Haloalkenyl, (C₃-C₇)Cycloalkyl;
R_{C}², R_{C}³ sind gleich oder verschieden Wasserstoff, (C₁-C₄)Alkyl, (C₂-C₄)Alkenyl, (C₂-C₄)Alkinyl, (C₁-C₄)Haloalkyl, (C₂-C₄)Haloalkenyl, (C₁-C₄)Alkylcarbamoyl-(C₁-C₄)alkyl, (C₂-C₄)Alkenylcarbamoyl-(C₁-C₄)alkyl, (C₁-C₄)Alkoxy-(C₁-C₄)alkyl, Dioxolanyl-(C₁-C₄)alkyl, Thiazolyl, Furyl, Furylalkyl, Thienyl, Piperidyl, substituiertes oder unsubstituiertes Phenyl, oder R_{C}² und R_{C}³ bilden zusammen einen substituierten oder unsubstituierten heterocyclischen Ring, vorzugsweise einen Oxazolidin-, Thiazolidin-, Piperidin-, Morpholin-, Hexahydropyrimidin- oder Benzoxazinring;
S4) N-Acylsulfonamide der Formel (S4) und ihre Salze, 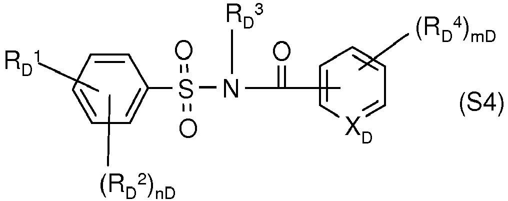 worin die Symbole und Indizes folgende Bedeutungen haben:
X_{D} ist CH oder N;
R_{D}¹ ist CO-NR_{D}⁵R_{D}⁶ oder NHCO-R_{D}⁷;
R_{D}² ist Halogen, (C₁-C₄)Haloalkyl, (C₁-C₄)Haloalkoxy, Nitro, (C₁-C₄)Alkyl, (C₁-C₄)Alkoxy, (C₁-C₄)Alkylsulfonyl, (C₁-C₄)Alkoxycarbonyl oder (C₁-C₄)Alkylcarbonyl;
R_{D}³ ist Wasserstoff, (C₁-C₄)Alkyl, (C₂-C₄)Alkenyl oder (C₂-C₄)Alkinyl;
R_{D}⁴ ist Halogen, Nitro, (C₁-C₄)Alkyl, (C₁-C₄)Haloalkyl, (C₁-C₄)Haloalkoxy, (C₃-C₆)Cycloalkyl, Phenyl, (C₁-C₄)Alkoxy, Cyano, (C₁-C₄)Alkylthio, (C₁-C₄)Alkylsulfinyl, (C₁-C₄)Alkylsulfonyl, (C₁-C₄)Alkoxycarbonyl oder (C₁-C₄)Alkylcarbonyl;
R_{D}⁵ ist Wasserstoff, (C₁-C₆)Alkyl, (C₃-C₆)Cycloalkyl, (C₂-C₆)Alkenyl, (C₂-C₆)Alkinyl, (C₅-C₆)Cycloalkenyl, Phenyl oder 3- bis 6-gliedriges Heterocyclyl enthaltend v_{D} Heteroatome aus der Gruppe Stickstoff, Sauerstoff und Schwefel, wobei die sieben letztgenannten Reste durch v_{D} Substituenten aus der Gruppe Halogen, (C₁-C₆)Alkoxy, (C₁-C₆)Haloalkoxy, (C₁-C₂)Alkylsulfinyl, (C₁-C₂)Alkylsulfonyl, (C₃-C₆)Cycloalkyl, (C₁-C₄)Alkoxycarbonyl, (C₁-C₄)Alkylcarbonyl und Phenyl und im Falle cyclischer Reste auch (C₁-C₄) Alkyl und (C₁-C₄)Haloalkyl substituiert sind;
R_{D}⁶ ist Wasserstoff, (C₁-C₆)Alkyl, (C₂-C₆)Alkenyl oder (C₂-C₆)Alkinyl, wobei die drei letztgenannten Reste durch v_{D} Reste aus der Gruppe Halogen, Hydroxy, (C₁-C₄)Alkyl, (C₁-C₄)Alkoxy und (C₁-C₄)Alkylthio substituiert sind, oder
R_{D}⁵ und R_{D}⁶ gemeinsam mit dem dem sie tragenden Stickstoffatom einen Pyrrolidinyl- oder Piperidinyl-Rest bilden;
R_{D}⁷ ist Wasserstoff, (C₁-C₄)Alkylamino, Di-(C₁-C₄)alkylamino, (C₁-C₆)Alkyl, (C₃-C₆)Cycloalkyl, wobei die 2 letztgenannten Reste durch v_{D} Substituenten aus der Gruppe Halogen, (C₁-C₄)Alkoxy, (C₁-C₆)Haloalkoxy und (C₁-C₄)Alkylthio und im Falle cyclischer Reste auch (C₁-C₄)Alkyl und (C₁-C₄)Haloalkyl substituiert sind;
n_{D} ist 0, 1 oder 2;
m_{D} ist 1 oder 2;
v_{D} ist 0, 1, 2 oder 3;
S5) Wirkstoffe aus der Klasse der Hydroxyaromaten und der aromatisch-aliphatischen Carbonsäurederivate (S5), z.B.
3,4,5-Triacetoxybenzoesäurecthylester, 3,5-Dimethoxy-4-hydroxybenzoesäure,
3,5-Dihydroxybenzoesäure, 4-Hydroxysalicylsäure, 4-Fluorsalicyclsäure, 2-Hydroxyzimtsäure, 1,2-Dihydro-2-oxo-6-trifluoromethylpyridin-3-carboxamid, 2,4-Dichlorzimtsäure.
S6) Wirkstoffe aus der Klasse der 1,2-Dihydrochinoxalin-2-one (S6), z.B. 1-Methyl-3-(2-thienyl)-1,2-dihydrochinoxalin-2-on, 1-Methyl-3-(2-thienyl)-1,2-dihydrochinoxalin-2-thion, 1-(2-Aminoethyl)-3-(2-thienyl)-1,2-dihydro-chinoxalin-2-on-hydrochlorid, 1-[2-(Diethylamino)ethyl]-6,7-dimethyl-3-thiophen-2-ylchinoxalin-2(1H)-on, 1-(2-Methylsulfonylaminoethyl)-3-(2-thienyl)-1,2-dihydro-chinoxalin-2-on.
S7) Verbindungen der Formel (S7), 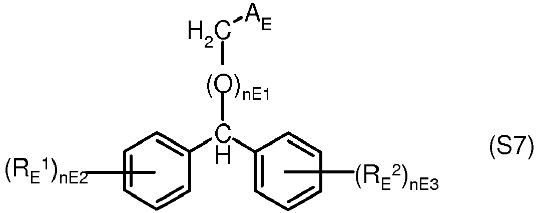 worin die Symbole und Indizes folgende Bedeutungen haben:
R_{E}¹, R_{E}² sind unabhängig voneinander Halogen, (C₁-C₄)Alkyl, (C₁-C₄)Alkoxy, (C₁-C₄)Haloalkyl, (C₁-C₄)Alkylamino, Di-(C₁-C₄)Alkylamino, Nitro;
A_{E} ist COOR_{E}³ oder COSR_{E}⁴
R_{E}³, R_{E}⁴ sind unabhängig voneinander Wasserstoff, (C₁-C₄)Alkyl, (C₂-C₆)Alkenyl, (C₂-C₄)Alkinyl, Cyanoalkyl, (C₁-C₄)Haloalkyl, Phenyl, Nitrophenyl, Benzyl, Halobenzyl, Pyridinylalkyl und Alkylammonium,
n_{E}¹ ist 0 oder 1
n_{E}², n_{E}³ sind unabhängig voneinander 0, 1 oder 2,
S8) Verbindungen der Formel (S8), 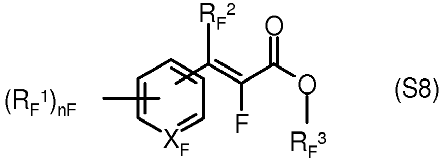 worin
X_{F} CH oder N,
n_{F} für den Fall, dass X_{F}=N ist, eine ganze Zahl von 0 bis 4 und
für den Fall, dass X_{F}=CH ist, eine ganze Zahl von 0 bis 5 ,
R_{F}¹ Halogen, (C₁-C₄)Alkyl, (C₁-C₄)Haloalkyl, (C₁-C₄)Alkoxy, (C₁-C₄)Haloalkoxy, Nitro, (C₁-C₄)Alkylthio, (C₁-C₄)-Alkylsulfonyl, (C₁-C₄)Alkoxycarbonyl, ggf. substituiertes. Phenyl, ggf. substituiertes Phenoxy,
R_{F}² Wasserstoff oder (C₁-C₄)Alkyl
R_{F}³ Wasserstoff, (C₁-C₈)Alkyl, (C₂-C₄)Alkenyl, (C₂-C₄)Alkinyl, oder Aryl, wobei jeder der vorgenannten C-haltigen Reste unsubstituiert oder durch einen oder mehrere, vorzugsweise bis zu drei gleiche oder verschiedene Reste aus der Gruppe, bestehend aus Halogen und Alkoxy substituiert ist;bedeuten, oder deren Salze,
S9) Wirkstoffe aus der Klasse der 3-(5-Tetrazolylcarbonyl)-2-chinolone (S9), z.B. 1,2-Dihydro-4-hydroxy-1-ethyl-3-(5-tetrazolylcarbonyl)-2-chinolon (CAS-Reg.Nr. 219479-18-2), 1,2-Dihydro-4-hydroxy-1-methyl-3-(5-tetrazolyl-carbonyl)-2-chinolon (CAS-Reg.Nr. 95855-00-8).
S10) Verbindungen der Formeln (S10^{a}) oder (S10^{b}) 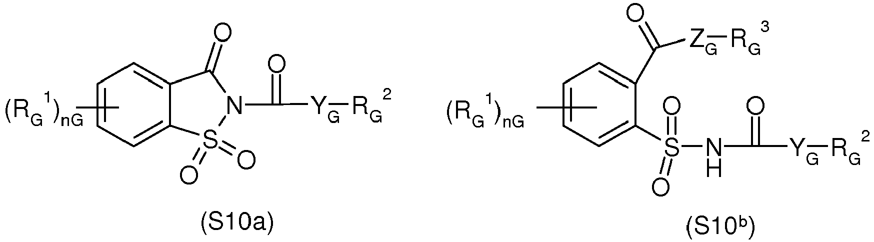 worin
R_{G}¹ Halogen, (C₁-C₄)Alkyl, Methoxy, Nitro, Cyano, CF₃, OCF₃
Y_{G}, Z_{G} unabhängig voneinander O oder S,
n_{G} eine ganze Zahl von 0 bis 4,
R_{G}² (C₁-C₁₆)Alkyl, (C₂-C₆)Alkenyl, (C₃-C₆)Cycloalkyl, Aryl; Benzyl, Halogenbenzyl,
R_{G}³ Wasserstoff oder (C₁-C₆)Alkyl bedeutet.
S11) Wirkstoffe vom Typ der Oxyimino-Verbindungen (S11), die als Saatbeizmittel bekannt sind, wie z. B.
"Oxabetrinil" ((Z)-1,3-Dioxolan-2-ylmethoxyimino(phenyl)acetonitril) (S11-1), das als Saatbeiz-Safener für Hirse gegen Schäden von Metolachlor bekannt ist,
"Fluxofenim" (1-(4-Chlorphenyl)-2,2,2-trifluor-1-ethanon-O-(1,3-dioxolan-2-ylmethyl)-oxim) (S11-2), das als Saatbeiz-Safener für Hirse gegen Schäden von Metolachlor bekannt ist, und
"Cyometrinil" oder "CGA-43089" ((Z)-Cyanomethoxyimino(phenyl)acetonitril) (S11-3), das als Saatbeiz-Safener für Hirse gegen Schäden von Metolachlor bekannt ist.
S12) Wirkstoffe aus der Klasse der Isothiochromanone (S12), wie z.B. Methyl-[(3-oxo-1H-2-benzothiopyran-4(3H)-yliden)methoxy]acetate (CAS-Reg.Nr. 205121-04-6) (S12-1) und verwandte Verbindungen aus WO-A-1998/13361.
S13) Eine oder mehrere Verbindungen aus Gruppe (S13):
"Naphthalic anhydrid" (1,8-Naphthalindicarbonsäureanhydrid) (S13-1), das als Saatbeiz-Safener für Mais gegen Schäden von Thiocarbamatherbiziden bekannt ist,
"Fenclorim" (4,6-Dichlor-2-phenylpyrimidin) (S13-2), das als Safener für Pretilachlor in gesätem Reis bekannt ist,
"Flurazole" (Benzyl-2-chlor-4-trifluormethyl-1,3-thiazol-5-carboxylat) (S13-3), das als Saatbeiz-Safener für Hirse gegen Schäden von Alachlor und Metolachlor bekannt ist,
"CL 304415" (CAS-Reg.Nr. 31541-57-8) (4-Carboxy-3,4-dihydro-2H-1-benzopyran-4-essigsäure) (S13-4) der Firma American Cyanamid, das als Safener für Mais gegen Schäden von Imidazolinonen bekannt ist,
"MG 191" (CAS-Reg.Nr. 96420-72-3) (2-Dichlormethyl-2-methyl-1,3-dioxolan) (S13-5) der Firma Nitrokemia, das als Safener für Mais bekannt ist,
"MG-838" (CAS-Reg.Nr. 133993-74-5) (2-propenyl 1-oxa-4-azaspiro[4.5]decane-4-carbodithioate) (S13-6) der Firma Nitrokemia,
"Disulfoton" (O,O-Diethyl S-2-ethylthioethyl phosphordithioat) (S13-7),
"Dietholate" (O,O-Diethyl-O-phenylphosphorotioat) (S13-8),
"Mephenate" (4-Chlorphenyl-methylcarbamat) (S13-9).
S14) Wirkstoffe, die neben einer herbiziden Wirkung gegen Schadpflanzen auch Safenerwirkung an Kulturpflanzen wie Reis aufweisen, wie z. B. "Dimepiperate" oder "MY-93" (*S*-1-Methyl-1-phenylethyl-piperidin-1-carbothioat), das als Safener für Reis gegen Schäden des Herbizids Molinate bekannt ist,
"Daimuron" oder "SK 23" (1-(1-Methyl-1-phenylethyl)-3-p-tolyl-harnstoff), das als Safener für Reis gegen Schäden des Herbizids Imazosulfuron bekannt ist,
"Cumyluron" = "JC-940" (3-(2-Chlorphenylmethyl)-1-(1-methyl-1-phenyl-ethyl)harnstoff, siehe JP-A-60087254), das als Safener für Reis gegen Schäden einiger Herbizide bekannt ist,
"Methoxyphenon" oder "NK 049" (3,3'-Dimethyl-4-methoxy-benzophenon), das als Safener für Reis gegen Schäden einiger Herbizide bekannt ist,
"CSB" (1-Brom-4-(chlormethylsulfonyl)benzol) von Kumiai, (CAS-Reg.Nr. 54091-06-4), das als Safener gegen Schäden einiger Herbizide in Reis bekannt ist.
S15) Verbindungen der Formel (S15) oder deren Tautomere 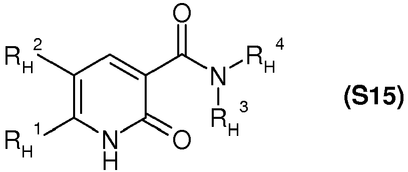 worin
R_{H}¹ einen (C₁-C₆)Haloalkylrest bedeutet und
R_{H}² Wasserstoff oder Halogen bedeutet und
R_{H}³, R_{H}⁴ unabhängig voneinander Wasserstoff, (C₁-C₁₆)Alkyl, (C₂-C₁₆)Alkenyl oder (C₂-C₁₆)Alkinyl,
wobei jeder der letztgenannten 3 Reste unsubstituiert oder durch einen oder mehrere Reste aus der Gruppe Halogen, Hydroxy, Cyano, (C₁-C₄)Alkoxy, (C₁-C₄)Haloalkoxy, (C₁-C₄)Alkylthio, (C₁-C₄)Alkylamino, Di[(C₁-C₄)alkyl]-amino, [(C₁-C₄)Alkoxy]-carbonyl, [(C₁-C₄)Haloalkoxy]-carbonyl, (C₃-C₆)Cycloalkyl, das unsubstituiert oder substituiert ist, Phenyl, das unsubstituiert oder substituiert ist, und Heterocyclyl, das unsubstituiert oder substituiert ist, substituiert ist,
oder (C₃-C₆)Cycloalkyl, (C₄-C₆)Cycloalkenyl, (C₃-C₆)Cycloalkyl, das an einer Seite des Rings mit einem 4 bis 6-gliedrigen gesättigten oder ungesättigten carbocyclischen Ring kondensiert ist, oder (C₄-C₆)Cycloalkenyl, das an einer Seite des Rings mit einem 4 bis 6-gliedrigen gesättigten oder ungesättigten carbocyclischen Ring kondensiert ist,
wobei jeder der letztgenannten 4 Reste unsubstituiert oder durch einen oder mehrere Reste aus der Gruppe Halogen, Hydroxy, Cyano, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Alkylamino, Di[(C₁-C₄)alkyl]-amino, [(C₁-C₄)Alkoxy]-carbonyl, [(C₁-C₄)Haloalkoxy]-carbonyl, (C₃-C₆)Cycloalkyl, das unsubstituiert oder substituiert ist, Phenyl, das unsubstituiert oder substituiert ist, und Heterocyclyl, das unsubstituiert oder substituiert ist, substituiert ist,
bedeutet oder
R_{H}³ (C₁-C₄)-Alkoxy, (C₂-C₄)Alkenyloxy, (C₂-C₆)Alkinyloxy oder (C₂-C₄)Haloalkoxy bedeutet und
R_{H}⁴ Wasserstoff oder (C₁-C₄)-Alkyl bedeutet oder
R_{H}³ und R_{H}⁴ zusammen mit dem direkt gebundenen N-Atom einen vier- bis achtgliedrigen heterocyclischen Ring, der neben dem N-Atom auch weitere Heteroringatome, vorzugsweise bis zu zwei weitere Heteroringatome aus der Gruppe N, O und S enthalten kann und der unsubstituiert oder durch einen oder mehrere Reste aus der Gruppe Halogen, Cyano, Nitro, (C₁-C₄)Alkyl, (C₁-C₄)Haloalkyl, (C₁-C₄)Alkoxy, (C₁-C₄)Haloalkoxy und (C₁-C₄)Alkylthio substituiert ist, bedeutet.
S16) Wirkstoffe, die vorrangig als Herbizide eingesetzt werden, jedoch auch Safenerwirkung auf Kulturpflanzen aufweisen, z.B.
(2,4-Dichlorphenoxy)essigsäure (2,4-D),
(4-Chlorphenoxy)essigsäure,
(R,S)-2-(4-Chlor-o-tolyloxy)propionsäure (Mecoprop),
4-(2,4-Dichlorphenoxy)buttersäure (2,4-DB),
(4-Chlor-o-tolyloxy)essigsäure (MCPA),
4-(4-Chlor-o-tolyloxy)buttersäure,
4-(4-Chlorphenoxy)buttersäure,
3,6-Dichlor-2-methoxybenzoesäure (Dicamba),
1-(Ethoxycarbonyl)ethyl-3,6-dichlor-2-methoxybenzoat (Lactidichlor-ethyl).

9. Verfahren zum Bekämpfen von unerwünschtem Pflanzenwuchs, **dadurch gekennzeichnet, dass** man ein Mittel gemäß Anspruch 8 auf die Pflanzen oder ihre Umgebung einwirken lässt.

10. Verwendung eines Mittels gemäß Anspruch 8 zum Bekämpfen von unerwünschten Pflanzenwuchs.

11. Verfahren zum Bekämpfen von unerwünschtem Pflanzenwuchs, **dadurch gekennzeichnet, dass** man eine Verbindung der Formel (I-a) gemäß Anspruch 1 und die die Kulturpflanzenverträglichkeit verbessernde Verbindung gemäß Anspruch 8 getrennt auf die Pflanzen oder ihre Umgebung einwirken lässt.

12. Zusammensetzung umfassend
- mindestens eine Verbindung der Formel (I-a) gemäß Anspruch 1 oder ein Mittel gemäß Anspruch 8 und
- mindestens ein Salz der Formel (III') 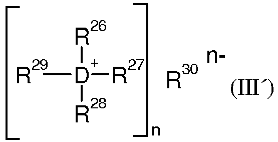 in welcher
D für Stickstoff oder Phosphor steht,
R²⁶, R²⁷, R²⁸ und R²⁹ unabhängig voneinander für Wasserstoff oder jeweils gegebenenfalls substituiertes C₁-C₈-Alkyl oder einfach oder mehrfach, ungesättigtes, gegebenenfalls substituiertes C₁-C₈-Alkylen stehen, wobei die Substituenten aus Halogen, Nitro und Cyano ausgewählt sein können,
n für 1, 2, 3 oder 4 steht,
R³⁰ für ein anorganisches oder organisches Anion steht.

13. Zusammensetzung gemäß Anspruch 12, **dadurch gekennzeichnet, dass** sie mindestens einen Penetrationsförderer enthält.

14. Verfahren zur Steigerung der Wirkung von Schädlingsbekämpfungsmitteln und/oder Herbiziden und/oder Fungiziden enthaltend einen Wirkstoff der Formel (I-a) gemäß Anspruch 1 oder ein Mittel gemäß Anspruch 8, **dadurch gekennzeichnet, dass** das anwendungsfertige Mittel (Spritzbrühe) unter Einsatz eines Salzes der Formel (III') gemäß Anspruch 12 zubereitet wird.

15. Verfahren gemäß Anspruch 14, **dadurch gekennzeichnet, dass** die Spritzbrühe unter Einsatz eines Penetrationsförderers zubereitet wird.

16. Verbindungen der Formel (II) 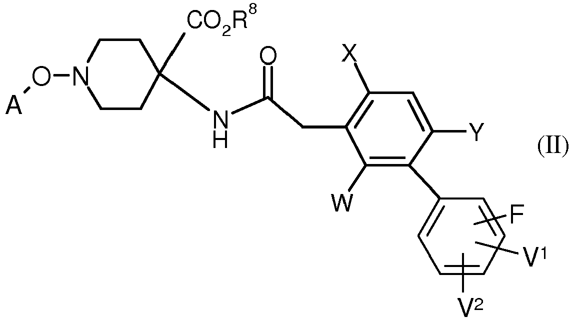 in welcher
A, W, X, Y , V¹ und V² die in Anspruch 1 angegebenen Bedeutungen haben, und
R⁸ für Alkyl steht.

17. Verbindungen der Formel (XVI) 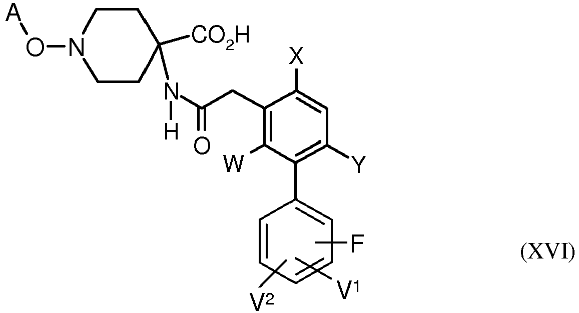 in welcher
A, W, X, Y V¹ und V²die in Anspruch 1 angegebenen Bedeutungen haben.

18. Verbindungen der Formel (XX) 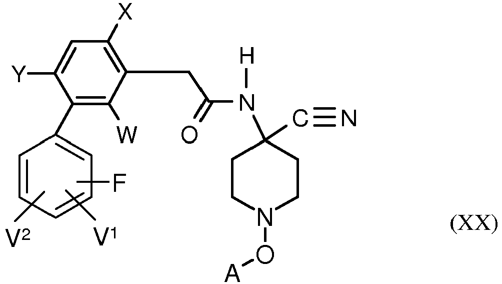 in welcher
A, W, X, Y V¹ und V² die in Anspruch 1 angegebenen Bedeutungen haben.

19. Verbindungen der Formeln (II'-1) und (II'-2)

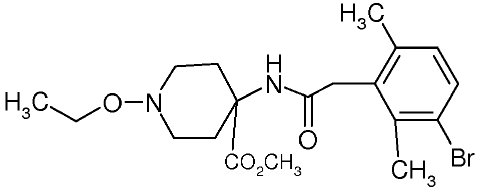

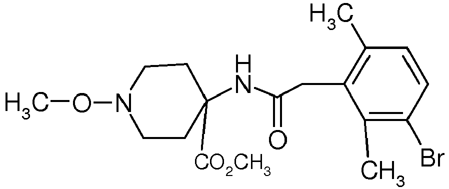

## Claims

1. Compounds of the formula (I-a) 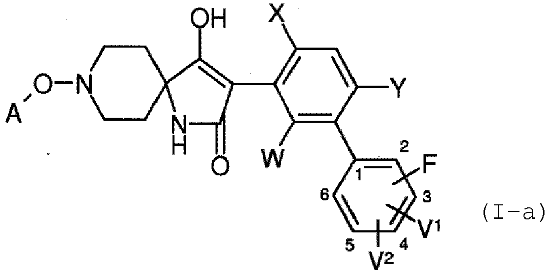 in which
A, W, X, Y, V¹ and V² have the following meanings
**Table 1**
| **A** | **W** | **X** | **Y** | **F** | **V¹** | **V²** |
|---|---|---|---|---|---|---|
| CH₃ | CH₃ | CH₃ | H | 3 | H | H |
| CH₃ | CH₃ | CH₃ | H | 4 | H | H |
| CH₃ | CH₃ | CH₃ | H | 2 | 4-F | H |
| CH₃ | CH₃ | CH₃ | H | 2 | 4-Cl | H |
| CH₃ | CH₃ | CH₃ | H | 2 | 4-CH₃ | H |
| CH₃ | CH₃ | CH₃ | H | 2 | 4-OCH₃ | H |
| CH₃ | CH₃ | CH₃ | H | 3 | 4-F | H |
| CH₃ | CH₃ | CH₃ | H | 3 | 4-Cl | H |
| CH₃ | CH₃ | CH₃ | H | 3 | 4-CH₃ | H |
| CH₃ | CH₃ | CH₃ | H | 3 | 4-OCH₃ | H |
| CH₃ | CH₃ | CH₃ | H | 4 | 3-Cl | H |
| CH₃ | CH₃ | CH₃ | H | 4 | 3-CH₃ | H |
| CH₃ | CH₃ | CH₃ | H | 4 | 3-OCH₃ | H |
| CH₃ | CH₃ | CH₃ | H | 2 | 4-F | 5-F |
| CH₃ | CH₃ | CH₃ | H | 2 | 4-F | 6-F |
| CH₃ | CH₃ | CH₃ | H | 2 | 4-Cl | 5-F |
| CH₃ | CH₃ | CH₃ | H | 2 | 5-Cl | 4-F |
| CH₃ | CH₃ | CH₃ | H | 3 | 4-F | 5-F |
| CH₃ | CH₃ | CH₃ | CH₃ | 2 | H | H |
| CH₃ | CH₃ | CH₃ | CH₃ | 3 | H | H |
| CH₃ | CH₃ | CH₃ | CH₃ | 4 | H | H |
| CH₃ | CH₃ | CH₃ | CH₃ | 2 | 4-F | H |
| CH₃ | CH₃ | CH₃ | CH₃ | 2 | 4-Cl | H |
| CH₃ | CH₃ | CH₃ | CH₃ | 2 | 4-CH₃ | H |
| CH₃ | CH₃ | CH₃ | CH₃ | 2 | 4-OCH₃ | H |
| CH₃ | CH₃ | CH₃ | CH₃ | 3 | 4-F | H |
| CH₃ | CH₃ | CH₃ | CH₃ | 3 | 4-Cl | H |
| CH₃ | CH₃ | CH₃ | CH₃ | 3 | 4-CH₃ | H |
| CH₃ | CH₃ | CH₃ | CH₃ | 3 | 4-OCH₃ | H |
| CH₃ | CH₃ | CH₃ | CH₃ | 4 | 3-Cl | H |
| CH₃ | CH₃ | CH₃ | CH₃ | 4 | 3-CH₃ | H |
| CH₃ | CH₃ | CH₃ | CH₃ | 4 | 3-OCH₃ | H |
| CH₃ | CH₃ | CH₃ | CH₃ | 2 | 4-F | 5-F |
| CH₃ | CH₃ | CH₃ | CH₃ | 2 | 4-F | 6-F |
| CH₃ | CH₃ | CH₃ | CH₃ | 2 | 4-Cl | 5-F |
| CH₃ | CH₃ | CH₃ | CH₃ | 2 | 5-Cl | 4-F |
| CH₃ | CH₃ | CH₃ | CH₃ | 3 | 4-F | 5-F |
Table 2 W, X, Y, F, V¹ and V² as indicated in Table 1 A = C₂H₅
**Table 3 W, X, Y, F, V¹ and V² as indicated in Table 1 A = C₃H₇**
| **W** | **X** | **Y** | **F** | **V¹** | **V²** | **A** |
|---|---|---|---|---|---|---|
| Cl | OCH₃ | H | 4 | H | H | C₂H₅ |
| CH₃ | C₂H₅ | H | 4 | H | H | CH₃ |
| CH₃ | C₂H₅ | H | 4 | H | H | C₂H₅ |
| Cl | OCH₃ | H | 4 | H | H | CH₃ |
| CH₃ | CH₃ | H | 4 | 3-F | H | C₂H₅ |
| CH₃ | CH₃ | H | 4 | 3-F | H | CH₃ |

2. Process for preparing compounds of the formula (I-a) according to Claim 1, **characterized in that**, to obtain
(A) compounds of the formula (I-a) 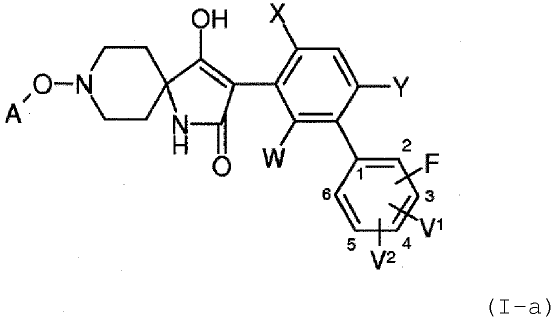 in which
A, W, X, Y, V¹ and V² have the meanings given above,
compounds of the formula (II) 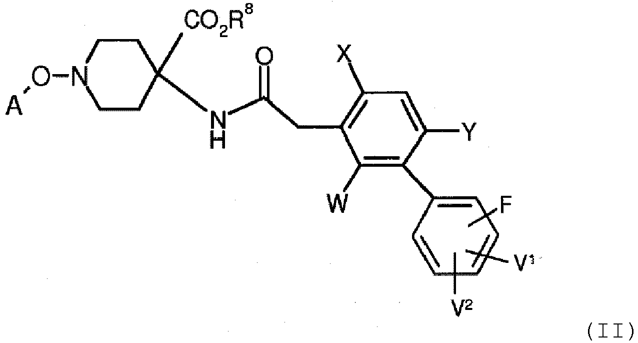 in which
A, W, X, Y, V¹ and V² have the meanings given above and R⁸ represents alkyl
are condensed intramolecularly in the presence of a diluent and in the presence of a base,
(I) compounds of the formula (I-a) shown above in which A, W, X, Y, V¹ and V² have the meaning given above, compounds of the formula (I-a') in which A, W, X and Y have the meaning given above and Z' preferably represents bromine or iodine 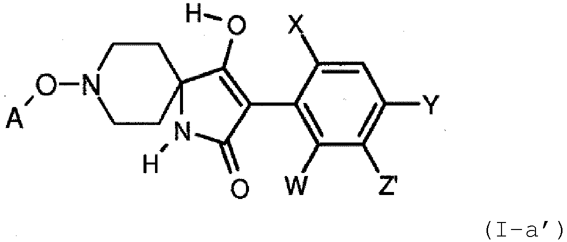
are coupled with (het)aryl derivatives capable of coupling, for example phenylboronic acids of the formula (XIII) 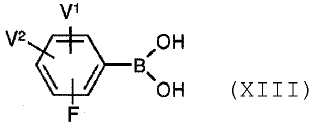 in which V¹ and V² have the meanings given above or esters thereof in the presence of a solvent, in the presence of a catalyst and in the presence of a base.

3. Pesticides and/or herbicides and/or fungicides, **characterized in that** they comprise at least one compound of the formula (I-a) according to Claim 1.

4. Method for controlling animal pests and/or unwanted vegetation and/or fungi, **characterized in that** compounds of the formula (I-a) according to Claim 1 are allowed to act on pests and/or their habitat, with the exception of methods for surgical and theraputic treatment of the human and animal body.

5. Use of compounds of the formula (I-a) according to Claim 1 for controlling animal pests and/or unwanted vegetation and/or fungi, with the exception of methods for surgical and theraputic treatment of the human and animal body.

6. Process for preparing pesticides and/or herbicides and/or fungicides, **characterized in that** compounds of the formula (I-a) according to Claim 1 are mixed with extenders and/or surfactants.

7. Use of compounds of the formula (I-a) according to Claim 1 for preparing pesticides and/or herbicides and/or fungicides.

8. Compositions, comprising an effective amount of an active compound combination comprising, as components,
a') at least one compound of the formula (I-a) in which A, W, X, Y, V¹ and V² have the meaning given above and
(b') at least one crop plant compatibility-improving compound from the following group of compounds:
S1) Compounds of the formula (S1) 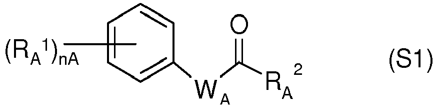 where the symbols and indices have the following meanings:
n_{A} is a natural number from 0 to 5,
R_{A}¹ is halogen, (C₁-C₄)-alkyl, (C₁-C₄)-alkoxy, nitro or (C₁-C₄)-haloalkyl;
W_{A} is an unsubstituted or substituted divalent heterocyclic radical from the group consisting of partially unsaturated or aromatic five-membered heterocycles having 1 to 3 hetero ring atoms from the group consisting of N and O, where at least one nitrogen atom and at most one oxygen atom is present in the ring,
R_{A}² is OR_{A}³, SR_{A}³ or NR_{A}³R_{A}⁴ or a saturated or unsaturated 3- to 7-membered heterocycle having at least one nitrogen atom and up to 3 heteroatoms, preferably from the group consisting of 0 and S, which is attached via the nitrogen atom to the carbonyl group in (S1) and which is unsubstituted or substituted by radicals from the group consisting of (C₁-C₄)-alkyl, (C₁-C₄)-alkoxy and optionally substituted phenyl, preferably a radical of the formula OR_{A}³, NHR_{A}⁴ or N(CH₃)₂, in particular of the formula OR_{A}³;
R_{A}³ is hydrogen or an unsubstituted or substituted aliphatic hydrocarbon radical having preferably a total of 1 to 18 carbon atoms;
R_{A}⁴ is hydrogen, (C₁-C₆)-alkyl, (C₁-C₆)-alkoxy or substituted or unsubstituted phenyl;
S2) Quinoline derivatives of the formula (S2)
where the symbols and indices have the following 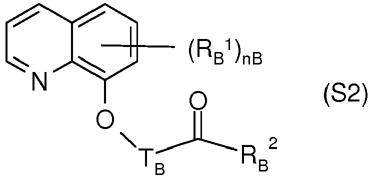 meanings:
R_{B}¹ is halogen, (C₁-C₄)-alkyl, (C₁-C₄)-alkoxy, nitro or (C₁-C₄)-haloalkyl;
n_{B} is a natural number from 0 to 5, preferably from 0 to 3;
R_{B}² is OR_{B}³, SR_{B}³ or NR_{B}³R_{B}⁴ or a saturated or unsaturated 3- to 7-membered heterocycle having at least one nitrogen atom and up to 3 heteroatoms, preferably from the group consisting of O and S, which is attached via the nitrogen atom to the carbonyl group in (S2) and which is unsubstituted or substituted by radicals from the group consisting of (C₁-C₄)-alkyl, (C₁-C₄)-alkoxy and optionally substituted phenyl, preferably a radical of the formula OR_{B}³, NHR_{B}⁴ or N(CH₃)₂, in particular of the formula OR_{B}³;
R_{B}³ is hydrogen or an unsubstituted or substituted aliphatic hydrocarbon radical having preferably a total of 1 to 18 carbon atoms;
R_{B}⁴ is hydrogen, (C₁-C₆)-alkyl, (C₁-C₆)-alkoxy or substituted or unsubstituted phenyl;
T_{B} is a (C₁- or C₂)-alkanediyl chain which is unsubstituted or substituted by one or two (C₁-C₄)-alkyl radicals or by [(C₁-C₃)-alkoxy]carbonyl;
S3) Compounds of the formula (S3) 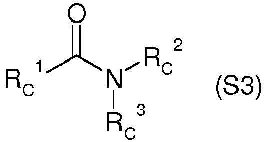 where the symbols and indices have the following meanings:
R_{C}¹ is (C₁-C₄)-alkyl, (C₁-C₄)-haloalkyl, (C₂-C₄)-alkenyl, (C₂-C₄)-haloalkenyl, (C₃-C₇)-cycloalkyl;
R_{C}², R_{C}³ are identical or different and are hydrogen, (C₁-C₄)-alkyl, (C₂-C₄)-alkenyl, (C₂-C₄)-alkynyl, (C₁-C₄)-haloalkyl, (C₂-C₄)-haloalkenyl, (C₁-C₄)-alkylcarbamoyl- (C₁-C₄) -alkyl, (C₂-C₄)-alkenylcarbamoyl-(C₁-C₄)-alkyl, (C₁-C₄)-alkoxy-(C₁-C₄)-alkyl, dioxolanyl-(C₁-C₄)-alkyl, thiazolyl, furyl, furylalkyl, thienyl, piperidyl, substituted or unsubstituted phenyl, or R_{C}² and R_{C}³ together form a substituted or unsubstituted heterocyclic ring, preferably an oxazolidine, thiazolidine, piperidine, morpholine, hexahydropyrimidine or benzoxazine ring;
S4) N-Acylsulphonamides of the formula (S4) and their salts 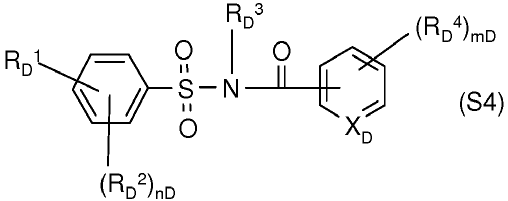 where the symbols and indices have the following meanings:
X_{D} is CH or N;
R_{D}¹ is CO-NR_{D}⁵R_{D}⁶ or NHCO-R_{D}⁷;
R_{D}² is halogen, (C₁-C₄) -haloalkyl, (C₁-C₄) -haloalkoxy, nitro, (C₁-C₄)-alkyl, (C₁-C₄)-alkoxy, (C₁-C₄)-alkylsulphonyl, (C₁-C₄)-alkoxycarbonyl or (C₁-C₄)-alkylcarbonyl;
R_{D}³ is hydrogen, (C₁-C₄)-alkyl, (C₂-C₄) -alkenyl or (C₂-C₄)-alkynyl;
R_{D}⁴ is halogen, nitro, (C₁-C₄)-alkyl, (C₁-C₄)-haloalkyl, (C₁-C₄) -haloalkoxy, (C₃-C₆)-cycloalkyl, phenyl, (C₁-C₄)-alkoxy, cyano, (C₁-C₄) -alkylthio, (C₁-C₄) -alkyl-sulphinyl, (C₁-C₄) -alkylsulphonyl, (C₁-C₄)-alkoxycarbonyl or (C₁-C₄)-alkylcarbonyl;
R_{D}⁵ is hydrogen, (C₁-C₆)-alkyl, (C₃-C₆)-cycloalkyl, (C₂-C₆)-alkenyl, (C₂-C₆) -alkynyl, (C₅-C₆) -cycloalkenyl, phenyl or 3- to 6-membered heterocyclyl which contains v_{D} heteroatoms from the group consisting of nitrogen, oxygen and sulphur, where the seven last-mentioned radicals are substituted by v_{D} substituents from the group consisting of halogen, (C₁-C₆)-alkoxy, (C₁-C₆)-haloalkoxy, (C₁-C₂)-alkylsulphinyl, (C₁-C₂) -alkylsulphonyl, (C₃-C₆)-cycloalkyl, (C₁-C₄)-alkoxycarbonyl, (C₁-C₄)-alkylcarbonyl and phenyl and, in the case of cyclic radicals, also (C₁-C₄)-alkyl and (C₁-C₄)-haloalkyl;
R_{D}⁶ is hydrogen, (C₁-C₆)-alkyl, (C₂-C₆) -alkenyl or (C₂-C₆)-alkynyl, where the three last-mentioned radicals are substituted by v_{D} radicals from the group consisting of halogen, hydroxy, (C₁-C₄)-alkyl, (C₁-C₄)-alkoxy and (C₁-C₄)-alkylthio, or
R_{D}⁵ and R_{D}⁶ together with the nitrogen atom carrying them form a pyrrolidinyl or piperidinyl radical;
R_{D}⁷ is hydrogen, (C₁-C₄)-alkylamino, di-(C₁-C₄)-alkylamino, (C₁-C₆)-alkyl, (C₃-C₆)-cycloalkyl, where the 2 last-mentioned radicals are substituted by v_{D} substituents from the group consisting of halogen, (C₁-C₄)-alkoxy, (C₁-C₆) -haloalkoxy and (C₁-C₄)-alkylthio and, in the case of cyclic radicals, also (C₁-C₄)-alkyl and (C₁-C₄)-haloalkyl;
n_{D} is 0, 1 or 2;
m_{D} is 1 or 2;
v_{D} is 0, 1, 2 or 3;
S5) Active compounds from the class of the hydroxyaromatics and aromatic-aliphatic carboxylic acid derivatives (S5), for example ethyl 3,4,5-triacetoxybenzoate, 3,5-dimethoxy-4-hydroxybenzoic acid, 3,5-dihydroxybenzoic acid, 4-hydroxysalicylic acid, 4-fluorosalicyclic acid, 2-hydroxycinnamic acid, 1,2-dihydro-2-oxo-6-trifluoromethylpyridine-3-carboxamide, 2,4-dichlorocinnamic acid.
S6) Active compounds from the class of the 1,2-dihydroquinoxalin-2-ones (S6), for example 1-methyl-3-(2-thienyl)-1,2-dihydroquinoxalin-2-one, 1-methyl-3-(2-thienyl)-1,2-dihydroquinoxaline-2-thione, 1-(2-aminoethyl)-3-(2-thienyl)-1,2-dihydroquinoxalin-2-one hydrochloride, 1-[2-(diethylamino)ethyl]-6,7-dimethyl-3-thiophen-2-ylquinoxalin-2(1H)-one, 1-(2-methylsulphonylaminoethyl)-3-(2-thienyl)-1,2-dihydroquinoxalin-2-one.
S7) Compounds of the formula (S7), 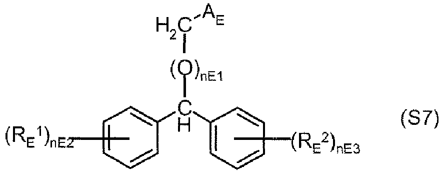 where the symbols and indices have the following meanings:
R_{E}¹, R_{E}² independently of one another are halogen, (C₁-C₄)-alkyl, (C₁-C₄)-alkoxy, (C₁-C₄) -haloalkyl, (C₁-C₄)-alkylamino, di-(C₁-C₄)-alkylamino, nitro;
A_{E} is COOR_{E}³ or COSR_{E}⁴
R_{E}³, R_{E}⁴ independently of one another are hydrogen, (C₁-C₄)-alkyl, (C₂-C₆)-alkenyl, (C₂-C₄)-alkynyl, cyanoalkyl, (C₁-C₄)-haloalkyl, phenyl, nitrophenyl, benzyl, halobenzyl, pyridinylalkyl or alkylammonium,
n_{E}¹ is 0 or 1
n_{E}², n_{E}³ independently of one another are 0, 1 or 2,
S8) Compounds of the formula (S8), 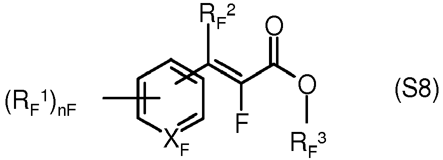 in which
X_{F} is CH or N,
n_{F} is, if X_{F}=N, an integer from 0 to 4 and
is, if X_{F}=CH, an integer from 0 to 5,
R_{F}¹ is halogen, (C₁-C₄)-alkyl, (C₁-C₄) -haloalkyl, (C₁-C₄)-alkoxy, (C₁-C₄) -haloalkoxy, nitro, (C₁-C₄)-alkylthio, (C₁-C₄) -alkylsulphonyl, (C₁-C₄)-alkoxycarbonyl, optionally substituted phenyl, optionally substituted phenoxy,
R_{F}² is hydrogen or (C₁-C₄)-alkyl,
R_{F}³ is hydrogen, (C₁-C₈)-alkyl, (C₂-C₄)-alkenyl, (C₂-C₄)-alkynyl or aryl, where each of the carbon-containing radicals mentioned above is unsubstituted or substituted by one or more, preferably by up to three, identical or different radicals from the group consisting of halogen and alkoxy; or salts thereof,
S9) Active compounds from the class of the 3-(5-tetrazolylcarbonyl)-2-quinolones (S9), for example 1,2-dihydro-4-hydroxy-1-ethyl-3-(5-tetrazolylcarbonyl)-2-quinolone (CAS Reg. No.219479-18-2), 1,2-dihydro-4-hydroxy-1-methyl-3-(5-tetrazolylcarbonyl)-2-quinolone (CAS Reg. No.95855-00-8).
S10) Compounds of the formula (S10^{a}) or (S10^{b}) 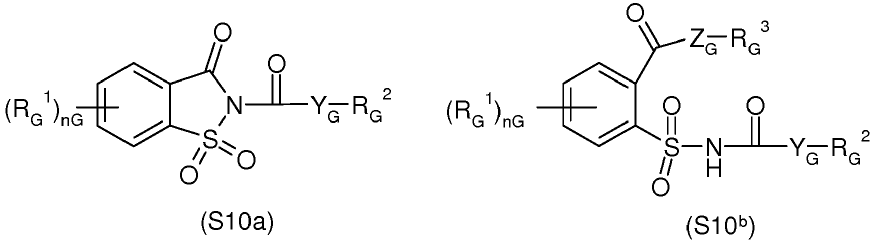 in which
R_{G}¹ is halogen, (C₁-C₄)-alkyl, methoxy, nitro, cyano, CF₃, OCF₃,
Y_{G}, Z_{G} independently of one another are O or S,
n_{G} is an integer from 0 to 4,
R_{G}² is (C₁-C₁₆)-alkyl, (C₂-C₆) -alkenyl, (C₃-C₆)-cycloalkyl, aryl; benzyl, halobenzyl,
R_{G}³ is hydrogen or (C₁-C₆)-alkyl.
S11) Active compounds of the type of the oxyimino compounds (S11), which are known as seed dressings, such as, for example,
"oxabetrinil" ((Z)-1,3-dioxolan-2-ylmethoxyimino-(phenyl)acetonitrile) (S11-1), which is known as seed dressing safener for millet against metolachlor damage,
"fluxofenim" (1-(4-chlorophenyl)-2,2,2-trifluoro-1-ethanone O-(1,3-dioxolan-2-ylmethyl)oxime) (S11-2), which is known as seed dressing safener for millet against metolachlor damage, and
"cyometrinil" or "CGA-43089" ((Z)-cyanomethoxyimino(phenyl)acetonitrile) (S11-3), which is known as seed dressing safener for millet against metolachlor damage.
S12) Active compounds from the class of the isothiochromanones (S12), such as, for example, methyl [(3-oxo-1H-2-benzothiopyran-4(3H)ylidene)methoxy]acetate (CAS Reg. No. 205121-04-6) (S12-1) and related compounds from WO-A-1998/13361.
S13) One or more compounds from group (S13):
"naphthalic anhydride" (1,8-naphthalenedicarboxylic anhydride) (S13-1), which is known as seed dressing safener for corn against thiocarbamate herbicide damage,
"fenclorim" (4,6-dichloro-2-phenylpyrimidine) (S13-2), which is known as safener for pretilachlor in sown rice,
"flurazole" (benzyl 2-chloro-4-trifluoromethyl-1,3-thiazole-5-carboxylate) (S13-3), which is known as seed dressing safener for millet against alachlor and metolachlor damage,
"CL 304415" (CAS Reg. No. 31541-57-8) (4-carboxy-3,4-dihydro-2H-1-benzopyran-4-acetic acid) (S13-4) from American Cyanamid, which is known as safener for corn against imidazolinone damage,
"MG 191" (CAS Reg. No. 96420-72-3) (2-dichloromethyl-2-methyl-1,3-dioxolane) (S13-5) from Nitrokemia, which is known as safener for corn,
"MG-838" (CAS Reg. No. 133993-74-5) (2-propenyl 1-oxa-4-azaspiro[4.5]decane-4-carbodithioate) (S13-6) from Nitrokemia,
"disulfoton" (0,0-diethyl S-2-ethylthioethyl phosphorodithioate) (S13-7),
"dietholate" (O,O-diethyl 0-phenyl phosphorothioate) (S13-8),
"mephenate" (4-chlorophenyl methylcarbamate) (S13-9).
S14) Active compounds which, besides a herbicidal effect against harmful plants, also have a safener effect on crop plants such as rice, such as, for example, "dimepiperate" or "MY-93" (*S*-1-methyl-1-phenylethyl piperidine-1-carbothioate), which is known as safener for rice against molinate herbicide damage,
"daimuron" or "SK 23" (1-(1-methyl-1-phenylethyl)-3-p-tolylurea), which is known as safener for rice against imazosulfuron herbicide damage,
"cumyluron" = "JC-940" (3-(2-chlorophenylmethyl)-1-(1-methyl-1-phenylethyl)urea, see JP-A-60087254), which is known as safener for rice against some herbicide damage,
"methoxyphenone" or "NK 049" (3,3'-dimethyl-4-methoxybenzophenone), which is known as safener for rice against some herbicide damage,
"CSB" (1-bromo-4-(chloromethylsulphonyl)benzene) from Kumiai, (CAS Reg. No. 54091-06-4), which is known as safener against some herbicide damage in rice.
S15) Compounds of the formula (S15) or tautomers thereof 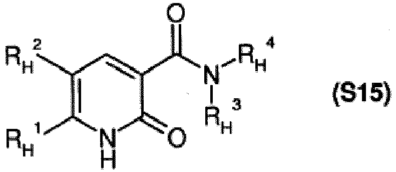 in which
R_{H}¹ represents a (C₁-C₆) -haloalkyl radical and
R_{H}² represents hydrogen or halogen and
R_{H}³, R_{H}⁴ independently of one another represent hydrogen, (C₁-C₁₆)-alkyl, (C₂-C₁₆)-alkenyl or (C₂-C₁₆)-alkynyl,
where each of the 3 last mentioned radicals is unsubstituted or substituted by one or more radicals from the group consisting of halogen, hydroxy, cyano, (C₁-C₄)-alkoxy, (C₁-C₄)-haloalkoxy, (C₁-C₄)-alkylthio, (C₁-C₄)-alkylamino, di [(C₁-C₄)-alkyl]amino, [(C₁-C₄)-alkoxy]carbonyl, [(C₁-C₄)-haloalkoxy]carbonyl, (C₃-C₆)-cycloalkyl, which is unsubstituted or substituted, phenyl, which is unsubstituted or substituted, and heterocyclyl, which is unsubstituted or substituted,
or (C₃-C₆)-cycloalkyl, (C₄-C₆)-cycloalkenyl, (C₃-C₆)-cycloalkyl which is condensed on one side of the ring to a 4- to 6-membered saturated or unsaturated carbocyclic ring, or (C₄-C₆)-cycloalkenyl which is condensed on one side of the ring to a 4- to 6-membered saturated or unsaturated carbocyclic ring,
where each of the 4 last mentioned radicals is unsubstituted or substituted by one or more radicals from the group consisting of halogen, hydroxy, cyano, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylamino, di [(C₁-C₄)-alkyl]amino, [(C₁-C₄)-alkoxy]carbonyl, [(C₁-C₄)-haloalkoxy]carbonyl, (C₃-C₆)-cycloalkyl, which is unsubstituted or substituted, phenyl, which is unsubstituted or substituted, and heterocyclyl, which is unsubstituted or substituted,
or
R_{H}³ represents (C₁-C₄)-alkoxy, (C₂-C₄)-alkenyloxy, (C₂-C₆)-alkynyloxy or (C₂-C₄)-haloalkoxy and
R_{H}⁴ represents hydrogen or (C₁-C₄)-alkyl or
R_{H}³ and R_{H}⁴ together with the directly attached nitrogen atom represent a four- to 8-membered heterocyclic ring which, in addition to the nitrogen atom, may also contain further hetero ring atoms, preferably up to two further hetero ring atoms from the group consisting of N, O and S, and which is unsubstituted or substituted by one or more radicals from the group consisting of halogen, cyano, nitro, (C₁-C₄)-alkyl, (C₁-C₄)-haloalkyl, (C₁-C₄)-alkoxy, (C₁-C₄)-haloalkoxy and (C₁-C₄)-alkylthio.
S16) Active compounds which are primarily used as herbicides, but also have safener effect on crop plants, for example
(2,4-dichlorophenoxy)acetic acid (2,4-D), (4-chlorophenoxy)acetic acid,
(R,S)-2-(4-chloro-o-tolyloxy)propionic acid (mecoprop),
4-(2,4-dichlorophenoxy)butyric acid (2,4-DB),
(4-chloro-o-tolyloxy)acetic acid (MCPA),
4-(4-chloro-o-tolyloxy)butyric acid,
4-(4-chlorophenoxy)butyric acid,
3,6-dichloro-2-methoxybenzoic acid (dicamba),
1-(ethoxycarbonyl)ethyl 3,6-dichloro-2-methoxybenzoate (lactidichlor-ethyl).

9. Method for controlling unwanted vegetation, **characterized in that** a composition according to Claim 8 is allowed to act on the plants or their environment.

10. Use of a composition according to Claim 8 for controlling unwanted vegetation.

11. Method for controlling unwanted vegetation, **characterized in that** a compound of the formula (I-a) according to Claim 1 and the crop plant compatibility-improving compound according to Claim 8 are allowed to act separately on the plants or their environment.

12. Composition, comprising
- at least one compound of the formula (I-a) according to Claim 1 or a composition according to Claim 8 and
- at least one salt of the formula (III') 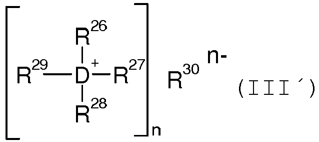 in which
D represents nitrogen or phosphorus,
R²⁶, R²⁷, R²⁸ and R²⁹ independently of one another represent hydrogen or in each case optionally substituted C₁-C₈-alkyl or mono- or polyunsaturated, optionally substituted C₁-C₈-alkylene, where the substituents may be selected from the group consisting of halogen, nitro and cyano,
n represents 1, 2, 3 or 4,
R³⁰ represents an inorganic or organic anion.

13. Composition according to Claim 12, **characterized in that** it comprises at least one penetrant.

14. Method for enhancing the action of pesticides and/or herbicides and/or fungicides comprising an active compound of the formula (I-a) according to Claim 1 or a composition according to Claim 8, **characterized in that** the ready-to-use composition (spray liquor) is prepared using a salt of the formula (III') according to Claim 12.

15. Method according to Claim 14, **characterized in that** the spray liquor is prepared using a penetrant.

16. Compounds of the formula (II) 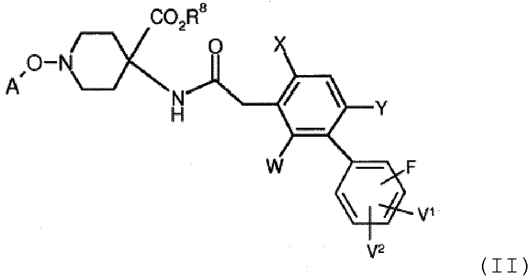 in which
A, W, X, Y, V¹ and V² have the meanings given in Claim 1 and
R⁸ represents alkyl.

17. Compounds of the formula (XVI) 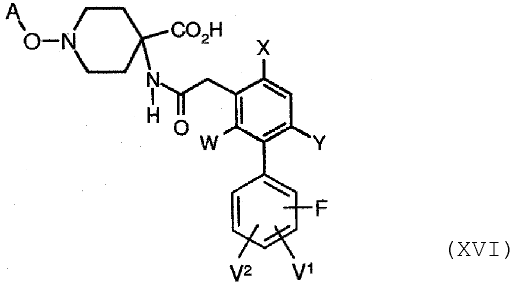 in which
A, W, X, Y, V¹ and V² have the meanings given in Claim 1.

18. Compounds of the formula (XX) 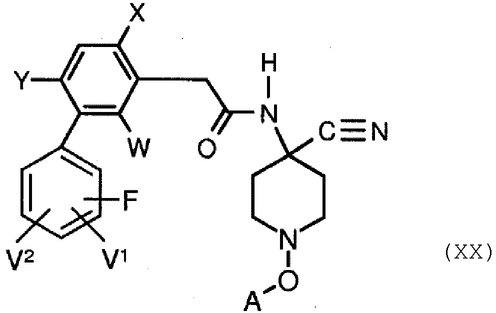 in which
A, W, X, Y, V¹ and V² have the meanings given in Claim 1.

19. Compounds of the formulae (II'-1) and (II'-2)

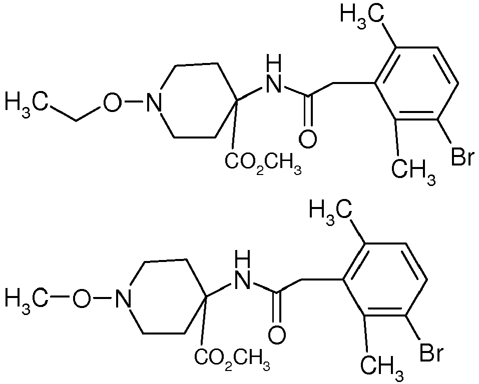

## Revendications

1. Composés de formule (I-a) 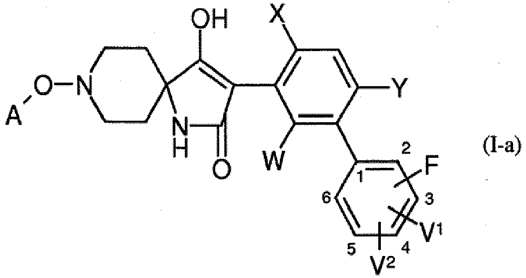 dans laquelle
A, W, X, Y, V¹ et V² ont les significations suivantes :
**Tableau 1**
| A | W | X | Y | F | V¹ | V² |
|---|---|---|---|---|---|---|
| CH₃ | CH₃ | CH₃ | H | 2 | H | H |
| CH₃ | CH₃ | CH₃ | H | 3 | H | H |
| CH₃ | CH₃ | CH₃ | H | 4 | H | H |
| CH₃ | CH₃ | CH₃ | H | 2 | 4-F | H |
| CH₃ | CH₃ | CH₃ | H | 2 | 4-Cl | H |
| CH₃ | CH₃ | CH₃ | H | 2 | 4-CH₃ | H |
| CH₃ | CH₃ | CH₃ | H | 2 | 4-OCH₃ | H |
| CH₃ | CH₃ | CH₃ | H | 3 | 4-F | H |
| CH₃ | CH₃ | CH₃ | H | 3 | 4-Cl | H |
| CH₃ | CH₃ | CH₃ | H | 3 | 4-CH₃ | H |
| CH₃ | CH₃ | CH₃ | H | 3 | 4-OCH₃ | H |
| CH₃ | CH₃ | CH₃ | H | 4 | 3-Cl | H |
| CH₃ | CH₃ | CH₃ | H | 4 | 3-CH₃ | H |
| CH₃ | CH₃ | CH₃ | H | 4 | 3-OCH₃ | H |
| CH₃ | CH₃ | CH₃ | H | 2 | 4-F | 5-F |
| CH₃ | CH₃ | CH₃ | H | 2 | 4-F | 6-F |
| CH₃ | CH₃ | CH₃ | H | 2 | 4-Cl | 5-F |
| CH₃ | CH₃ | CH₃ | H | 2 | 5-Cl | 4-F |
| CH₃ | CH₃ | CH₃ | H | 3 | 4-F | 5-F |
| CH₃ | CH₃ | CH₃ | CH₃ | 2 | H | H |
| CH₃ | CH₃ | CH₃ | CH₃ | 3 | H | H |
| CH₃ | CH₃ | CH₃ | CH₃ | 4 | H | H |
| CH₃ | CH₃ | CH₃ | CH₃ | 2 | 4-F | H |
| CH₃ | CH₃ | CH₃ | CH₃ | 2 | 4-Cl | H |
| CH₃ | CH₃ | CH₃ | CH₃ | 2 | 4-CH₃ | H |
| CH₃ | CH₃ | CH₃ | CH₃ | 2 | 4-OCH₃ | H |
| CH₃ | CH₃ | CH₃ | CH₃ | 3 | 4-F | H |
| CH₃ | CH₃ | CH₃ | CH₃ | 3 | 4-Cl | H |
| CH₃ | CH₃ | CH₃ | CH₃ | 3 | 4-CH₃ | H |
| CH₃ | CH₃ | CH₃ | CH₃ | 3 | 4-OCH₃ | H |
| CH₃ | CH₃ | CH₃ | CH₃ | 4 | 3-Cl | H |
| CH₃ | CH₃ | CH₃ | CH₃ | 4 | 3-CH₃ | H |
| CH₃ | CH₃ | CH₃ | CH₃ | 4 | 3-OCH₃ | H |
| CH₃ | CH₃ | CH₃ | CH₃ | 2 | 4-F | 5-F |
| CH₃ | CH₃ | CH₃ | CH₃ | 2 | 4-F | 6-F |
| CH₃ | CH₃ | CH₃ | CH₃ | 2 | 4-Cl | 5-F |
| CH₃ | CH₃ | CH₃ | CH₃ | 2 | 5-Cl | 4-F |
| CH₃ | CH₃ | CH₃ | CH₃ | 3 | 4-F | 5-F |
Tableau 2 W, X, Y, F, V¹ et V² tels qu'indiqués dans le Tableau 1 A = C₂H₅
**Tableau 3 W, X, Y, F, V¹ et V² tels qu'indiqués dans le Tableau 1 A = C₃H₇**
| W | X | Y | F | V¹ | V² | A |
|---|---|---|---|---|---|---|
| Cl | OCH₃ | H | 4 | H | H | C₂H₅ |
| CH₃ | C₂H₅ | H | 4 | H | H | CH₃ |
| CH₃ | C₂H₅ | H | 4 | H | H | C₂H₅ |
| Cl | OCH₃ | H | 4 | H | H | CH₃ |
| CH₃ | CH₃ | H | 4 | 3-F | H | C₂H₅ |
| CH₃ | CH₃ | H | 4 | 3-F | H | CH₃ |

2. Procédé de fabrication de composés de formule (I-a) selon la revendication 1, **caractérisé en ce que**, pour l'obtention de
(A) composés de formule (I-a) 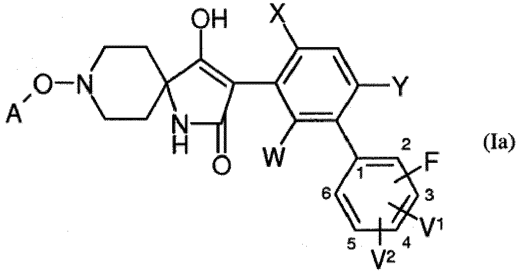 dans laquelle
A, W, X, Y, V¹ et V² ont les significations indiquées précédemment,
des composés de formule (II) 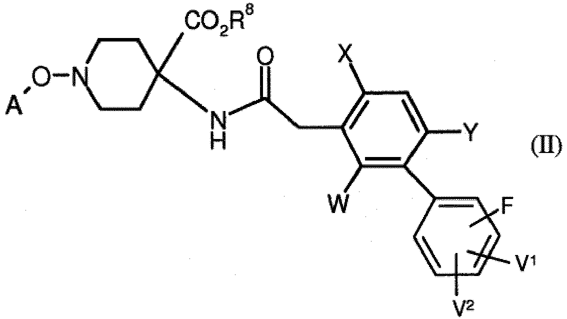 dans laquelle
A, W, X, Y, V¹ et V² ont les significations indiquées précédemment,
et R⁸ représente alkyle
sont condensés intramoléculairement en présence d'un diluant et en présence d'une base,
(I) composés de la formule (I-a) représentée précédemment, dans laquelle A, W, X, Y, V¹ et V² ont la signification indiquée précédemment, des composés de formule (I-a'), dans laquelle A, W, X et Y ont la signification indiquée précédemment et Z' représente de préférence brome ou iode 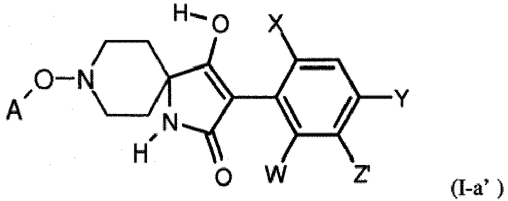 sont couplés avec des dérivés d'(Het)-aryle aptes au couplage, p. ex. des acides phénylboroniques de formule (XIII) 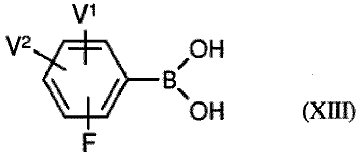 dans laquelle V¹ et V² ont les significations indiquées précédemment,
ou leurs esters, en présence d'un solvant, en présence d'un catalyseur et en présence d'une base.

3. Agents de lutte contre les animaux nuisibles et/ou herbicides et/ou fongicides, **caractérisés par** une teneur en au moins un composé de formule (I-a) selon la revendication 1.

4. Procédé de lutte contre les animaux nuisibles et/ou la végétation et/ou les champignons indésirables, **caractérisé en ce que** des composés de formule (I-a) selon la revendication 1 sont laissés agir sur les animaux nuisibles et/ou leur habitat, à l'exception des procédés de traitement chirurgical et thérapeutique du corps humain et animal.

5. Utilisation de composés de formule (I-a) selon la revendication 1 pour lutter contre les animaux nuisibles et/ou la végétation et/ou les champignons indésirables, à l'exception des procédés de traitement chirurgical et thérapeutique du corps humain et animal.

6. Procédé de fabrication d'agents de lutte contre les animaux nuisibles et/ou d'herbicides et/ou de fongicides, **caractérisé en ce que** des composés de formule (I-a) selon la revendication 1 sont mélangés avec des extendeurs et/ou des substances tensioactives.

7. Utilisation de composés de formule (I-a) selon la revendication 1 pour la fabrication d'agents de lutte contre les animaux nuisibles et/ou d'herbicides et/ou de fongicides.

8. Agent contenant une teneur efficace en une combinaison d'agents actifs comprenant en tant que composants :
(a') au moins un composé de formule (I-a) dans laquelle A, W, X, Y, V¹ et V² ont la signification indiquée précédemment, et
(b') au moins un composé améliorant la compatibilité avec les plantes cultivées choisi dans le groupe suivant de composés :
S1) les composés de formule (S1) 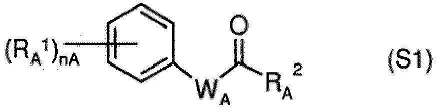 dans laquelle les symboles et les indices ont les significations suivantes :
n_{A} représente un nombre naturel de 0 à 5,
R_{A}¹ représente halogène, alkyle en (C₁-C₄), alcoxy en (C₁-C₄), nitro ou haloalkyle en (C₁-C₄);
W_{A} représente un radical hétérocyclique bivalent non substitué ou substitué du groupe constitué par les hétérocycles à cinq éléments partiellement insaturés ou aromatiques contenant 1 à 3 hétéroatomes de cycle du groupe constitué par N et O, au moins un atome N et au plus un atome O étant contenus dans le cycle,
R_{A}² représente OR_{A}³, SR_{A}³ ou NR_{A}³R_{A}⁴ ou un hétérocycle de 3 à 7 éléments, saturé ou insaturé, contenant au moins un atome N et jusqu'à 3 hétéroatomes, de préférence choisis dans le groupe constitué par O et S, qui est relié par l'atome N avec le groupe carbonyle dans (S1) et qui est non substitué ou substitué par des radicaux du groupe constitué par alkyle en (C₁-C₄), alcoxy en (C₁-C₄)ou phényle éventuellement substitué, de préférence un radical de formule OR_{A}³, NHR_{A}⁴ ou N(CH₃)₂, notamment de formule OR_{A}³;
R_{A}³ représente l'hydrogène ou un radical hydrocarboné aliphatique non substitué ou substitué, de préférence contenant au total 1 à 18 atomes C ;
R_{A}⁴ représente hydrogène, alkyle en (C₁-C₆), alcoxy en (C₁-C₆) ou phényle substitué ou non substitué ;
S2) les dérivés de quinoline de formule (S2) 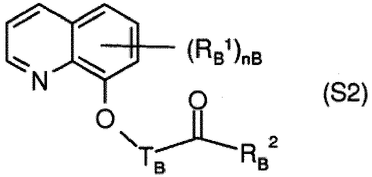 dans laquelle les symboles et les indices ont les significations suivantes :
R_{B}¹ représente halogène, alkyle en (C₁-C₄), alcoxy en (C₁-C₄), nitro ou haloalkyle en (C₁-C₄);
n_{B} représente un nombre naturel de 0 à 5, de préférence de 0 à 3 ;
R_{B}² représente OR_{B}², SR_{B}³ ou NR_{B}³R_{B}⁴ ou un hétérocycle de 3 à 7 éléments, saturé ou insaturé, contenant au moins un atome N et jusqu'à 3 hétéroatomes, de préférence choisis dans le groupe constitué par O et S, qui est relié par l'atome N avec le groupe carbonyle dans (S2) et qui est non substitué ou substitué par des radicaux du groupe constitué par alkyle en (C₁-C₄), alcoxy en (C₁-C₄)ou phényle éventuellement substitué, de préférence un radical de formule OR_{B}³, NHR_{B}⁴ ou N(CH₃)₂, notamment de formule OR_{B}³;
R_{B}³ représente l'hydrogène ou un radical hydrocarboné aliphatique non substitué ou substitué, de préférence contenant au total 1 à 18 atomes C ;
R_{B}⁴ représente hydrogène, alkyle en (C₁-C₆), alcoxy en (C₁-C₆) ou phényle substitué ou non substitué ;
T_{B} représente une chaîne alcanediyle en (C₁ ou C₂), qui est non substituée ou substituée avec un ou deux radicaux alkyle en (C₁-C₄)ou avec [alcoxy en (C₁-C₃)]-carbonyle;
S3) les composés de formule (S3) 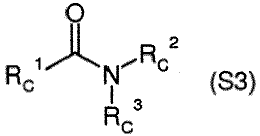 dans laquelle les symboles et les indices ont les significations suivantes :
R_{C}¹ représente alkyle en (C₁-C₄), haloalkyle en (C₁-C₄), alcényle en (C₂-C₄), haloalcényle en (C₂-C₄), cycloalkyle en (C₃-C₇) ;
R_{C}², R_{C}³ sont identiques ou différents et représentent hydrogène, alkyle en (C₁-C₄), alcényle en (C₂-C₄), alcynyle en (C₂-C₄), haloalkyle en (C₁-C₄), haloalcényle en (C₂-C₄), alkylcarbamoyle en (C₁-C₄)-alkyle en (C₁-C₄), alcénylcarbamoyle en (C₂-C₄)-alkyle en (C₁-C₄), alcoxy en (C₁-C₄)-alkyle en (C₁-C₄), dioxolanyl-alkyle en (C₁-C₄), thiazolyle, furyle, furylalkyle, thiényle, pipéridyle, phényle substitué ou non substitué, ou R_{C}² et R_{C}³ forment ensemble un cycle hétérocyclique substitué ou non substitué, de préférence un cycle oxazolidine, thiazolidine, pipéridine, morpholine, hexahydropyrimidine ou benzoxazine ;
S4) les N-acylsulfonamides de formule (S4) et leurs sels 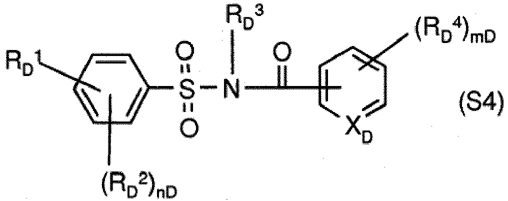 dans laquelle les symboles et les indices ont les significations suivantes :
X_{D} représente CH ou N ;
R_{D}¹ représente CO-NR_{D}⁵R_{D}⁶ ou NHCO-R_{D}⁷;
R_{D}² représente halogène, haloalkyle en (C₁-C₄), haloalcoxy en (C₁-C₄), nitro, alkyle en (C₁-C₄), alcoxy en (C₁-C₄), alkylsulfonyle en (C₁-C₄), alcoxycarbonyle en (C₁-C₄)ou alkylcarbonyle en (C₁-C₄);
R_{D}³ représente hydrogène, alkyle en (C₁-C₄), alcényle en (C₂-C₄) ou alcynyle en (C₂-C₄) ;
R_{D}⁴ représente halogène, nitro, alkyle en (C₁-C₄), haloalkyle en (C₁-C₄), haloalcoxy en (C₁-C₄), cycloalkyle en (C₃-C₆), phényle, alcoxy en (C₁-C₄), cyano, alkylthio en (C₁-C₄), alkylsulfinyle en (C₁-C₄), alkylsulfonyle en (C₁-C₄), alcoxycarbonyle en (C₁-C₄) ou alkylcarbonyle en (C₁-C₄);
R_{D}⁵ représente hydrogène, alkyle en (C₁-C₆), cycloalkyle en (C₃-C₆), alcényle en (C₂-C₆), alcynyle en (C₂-C₆), cycloalcényle en (C₅-C₆), phényle ou hétérocyclyle de 3 à 6 éléments, contenant v_{D} hétéroatomes choisis dans le groupe constitué par azote, oxygène et soufre, les sept derniers radicaux cités étant substitués par v_{D} substituants choisis dans le groupe constitué par halogène, alcoxy en (C₁-C₆), haloalcoxy en (C₁-C₆), alkylsulfinyle en (C₁-C₂), alkylsulfonyle en (C₁-C₂), cycloalkyle en (C₃-C₆), alcoxycarbonyle en (C₁-C₄), alkylcarbonyle en (C₁-C₄) et phényle et, dans le cas de radicaux cycliques, également alkyle en (C₁-C₄)et haloalkyle en (C₁-C₄);
R_{D}⁶ représente hydrogène, alkyle en (C₁-C₆), alcényle en (C₂-C₆) ou alcynyle en (C₂-C₆), les trois derniers radicaux cités étant substitués par v_{D} radicaux choisis dans le groupe constitué par halogène, hydroxy, alkyle en (C₁-C₄), alcoxy en (C₁-C₄) et alkylthio en (C₁-C₄), ou R_{D}⁵ et R_{D}⁶ forment ensemble avec l'atome d'azote qui les porte un radical pyrrolidinyle ou pipéridinyle ;
R_{D}⁷ représente hydrogène, alkylamino en (C₁-C₄), di-alkylamino en (C₁-C₄), alkyle en (C₁-C₆), cycloalkyle en (C₃-C₆), les 2 derniers radicaux cités étant substitués par v_{D} substituants choisis dans le groupe constitué par halogène, alcoxy en (C₁-C₄), haloalcoxy en (C₁-C₆) et alkylthio en (C₁-C₄) et, dans le cas de radicaux cycliques, également alkyle en (C₁-C₄) et haloalkyle en (C₁-C₄);
n_{D} représente 0, 1 ou 2 ;
m_{D} représente 1 ou 2 ;
v_{D} représente 0, 1, 2 ou 3 ;
S5) les agents actifs de la classe des composés hydroxyaromatiques et des dérivés d'acides carboxyliques aromatiques-aliphatiques (S5), p. ex. l'ester éthylique de l'acide 3,4,5-triacétoxybenzoïque, l'acide 3,5-diméthoxy-4-hydroxybenzoïque, l'acide 3,5-dihydroxybenzoïque, l'acide 4-hydroxysalicylique, l'acide 4-fluorosalicyclique, l'acide 2-hydroxycinnamique, le 1,2-dihydro-2-oxo-6-trifluorométhylpyridine-3-carboxamide, l'acide 2,4-dichlorocinnamique ;
S6) les agents actifs de la classe des 1,2-dihydroquinoxalin-2-ones (S6), p. ex.
la 1-méthyl-3-(2-thiényl)-1,2-dihydroquinoxalin-2-one, la 1-méthyl-3-(2-thiényl)-1,2-dihydroquinoxalin-2-thione, le chlorhydrate de 1-(2-aminoéthyl)-3-(2-thiényl)-1,2-dihydro-quinoxalin-2-one, la 1-[2-(diéthylamino)éthyl]-6,7-diméthyl-3-thiophén-2-ylquinoxalin-2(1H)-one, la 1-(2-méthylsulfonylaminoéthyl)-3-(2-thiényl)-1,2-dihydro-quinoxalin-2-one ;
S7) les composés de formule (S7) 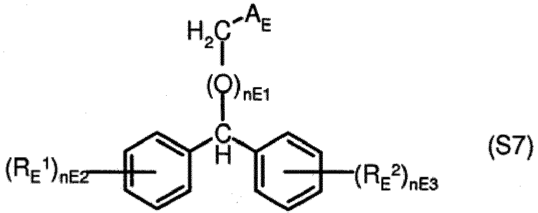 dans laquelle les symboles et les indices ont les significations suivantes :
R_{E}¹, R_{E}² représentent indépendamment l'un de l'autre halogène, alkyle en (C₁-C₄), alcoxy en (C₁-C₄), haloalkyle en (C₁-C₄), alkylamino en (C₁-C₄), di-alkylamino en (C₁-C₄), nitro;
A_{E} représente COOR_{E}³ ou COSR_{E}⁴ ;
R_{E}³, R_{E}⁴ représentent indépendamment l'un de l'autre hydrogène, alkyle en (C₁-C₄), alcényle en (C₂-C₆), alcynyle en (C₂-C₄), cyanoalkyle, haloalkyle en (C₁-C₄), phényle, nitrophényle, benzyle, halobenzyle, pyridinylalkyle et alkylammonium,
n_{E}¹ représente 0 ou 1,
n_{E}², n_{E}³ représentent indépendamment l'un de l'autre 0, 1 ou 2 ;
S8) les composés de formule (S8) 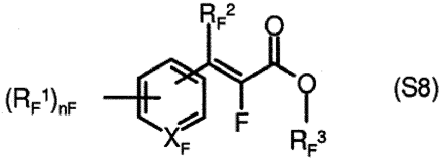 dans laquelle
X_{F} représente CH ou N,
n_{F} représente un nombre entier de 0 à 4 lorsque X_{F} = N, et
représente un nombre entier de 0 à 5 lorsque X_{F} = CH,
R_{F}¹ représente halogène, alkyle en (C₁-C₄), haloalkyle en (C₁-C₄), alcoxy en (C₁-C₄), haloalcoxy en (C₁-C₄), nitro, alkylthio en (C₁-C₄), alkylsulfonyle en (C₁-C₄), alcoxycarbonyle en (C₁-C₄), phényle éventuellement substitué, phénoxy éventuellement substitué,
R_{F}² représente hydrogène ou alkyle en (C₁-C₄),
R_{F}³ représente hydrogène, alkyle en (C₁-C₈), alcényle en (C₂-C₄), alcynyle en (C₂-C₄) ou aryle, chacun des radicaux contenant C susmentionnés étant non substitué ou substitué par un ou plusieurs, de préférence jusqu'à trois, radicaux identiques ou différents, choisis dans le groupe constitué par halogène et alcoxy ; ou leurs sels ;
S9) les agents actifs de la classe des 3-(5-tétrazolylcarbonyl)-2-quinolones (S9), p. ex. la 1,2-dihydro-4-hydroxy-1-éthyl-3-(5-tétrazolylcarbonyl)-2-quinolone (n° CAS 219479-18-2), la 1,2-dihydro-4-hydroxy-1-méthyl-3-(5-tétrazolyl-carbonyl)-2-quinolone (n° CAS 95855-00-8) ;
S10) les composés de formule (S10^{a}) ou (S10^{b}) 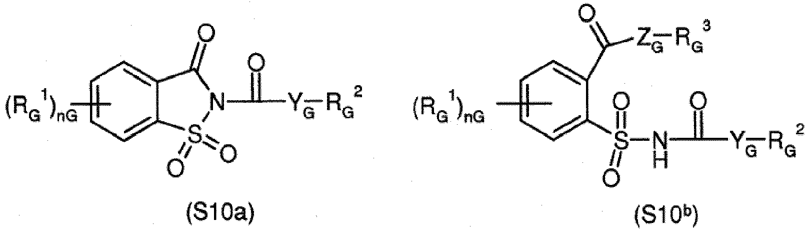 dans lesquelles
R_{G}¹ représente halogène, alkyle en (C₁-C₄), méthoxy, nitro, cyano, CF₃, OCF₃,
Y_{G}, Z_{G} représentent indépendamment l'un de l'autre O ou S,
n_{G} représente un nombre entier de 0 à 4,
R_{G}² représente alkyle en (C₁-C₁₆), alcényle en (C₂-C₆), cycloalkyle en (C₃-C₆), aryle, benzyle, halogénobenzyle,
R_{G}³ représente hydrogène ou alkyle en (C₁-C₆);
S11) les agents actifs du type des composés oxyimino (S11), qui sont connus en tant qu'agents de traitement de graines, tels que p. ex.
« l'oxabétrinile » ((Z)-1,3-dioxolan-2-ylméthoxyimino(phényl)acétonitrile) (S11-1), qui est connu en tant qu'agent protecteur de graines pour le millet contre les dégâts causés par le métolachlore,
« le fluxofénim » (1-(4-chlorophényl)-2,2,2-trifluoro-1-éthanone-O-(1,3-dioxolan-2-ylméthyl)-oxime) (S11-2), qui est connu en tant qu'agent protecteur de graines pour le millet contre les dégâts causés par le métolachlore, et « le cyométrinil » ou « CGA-43089 » ((Z)-cyanométhoxyimino(phényl)acétonitrile) (S11-3), qui est connu en tant qu'agent protecteur de graines pour le millet contre les dégâts causés par le métolachlore ;
S12) les agents actifs de la classe des isothiochromanones (S12), tels que p. ex. le [(3-oxo-1H-2-benzothiopyran-4(3H)-ylidène)méthoxy]acétate de méthyle (n° CAS 205121-04-6) (S12-1) et les composés apparentés de WO-A-1998/13361 ;
S13) un ou plusieurs composés du groupe (S13):
« l'anhydride naphtalique » (anhydride de l'acide 1,8-naphtalinedicarboxylique) (S13-1), qui est connu en tant qu'agent protecteur de graines pour le maïs contre les dégâts causés par les herbicides thiocarbamate,
« le fenclorim » (4,6-dichloro-2-phénylpyrimidine) (S13-2), qui est connu en tant qu'agent protecteur pour le prétilachlore dans les semences de riz,
« le flurazole » (2-chloro-4-trifluorométhyl-1,3-thiazol-5-carboxylate de benzyle) (S13-3), qui est connu en tant qu'agent protecteur de graines pour le millet contre les dégâts causés par l'alachlore et le métolachlore,
« CL 304415 » (n° CAS 31541-57-8) (acide 4-carboxy-3,4-dihydro-2H-1-benzopyrane-4-acétique) (S13-4) de la société American Cyanamid, qui est connu en tant qu'agent protecteur pour le maïs contre les dégâts causés par les imidazolinones,
« MG 191 » (n° CAS 96420-72-3) (2-dichlorométhyl-2-méthyl-1,3-dioxolane) (S13-5) de la société Nitrokemia, qui est connu en tant qu'agent protecteur pour le maïs,
« MG-838 » (n° CAS 133993-74-5) (1-oxa-4-azaspiro[4.5]décane-4-carbodithioate de 2-propényle) (S13-6) de la société Nitrokemia,
« le disulfoton » (phosphorodithioate de O,O-diéthyl-S-2-éthylthioéthyle) (S13-7),
« le diétholate » (phosphorotioate de O,O-diéthyl-O-phényle) (S13-8),
« le méphénate » (carbamate de 4-chlorophényl-méthyle) (S13-9) ;
S14) les agents actifs qui, en plus d'une action herbicide contre les plantes nocives, présentent également un effet protecteur sur les plantes cultivées, telles que le riz, tels que p. ex. « le dimépipérate » ou « MY-93 » (1-carbothioate de S-1-méthyl-1-phényléthyl-pipéridine), qui est connu en tant qu'agent protecteur pour le riz contre les dégâts de l'herbicide molinate,
« le daimuron » ou « SK 23 » (1-(1-méthyl-1-phényléthyl)-3-p-tolyl-urée), qui est connu en tant qu'agent protecteur pour le riz contre les dégâts de l'herbicide imazosulfurone,
« le cumyluron » = « JC-940 » (3-(2-chlorophénylméthyl)-1-(1-méthyl-1-phényl-éthyl)-urée, voir JP-A-60087254), qui est connu en tant qu'agent protecteur pour le riz contre les dégâts de plusieurs herbicides,
« la méthoxyphénone » ou « NK 049 » (3,3'-diméthyl-4-méthoxy-benzophénone), qui est connue en tant qu'agent protecteur pour le riz contre les dégâts de plusieurs herbicides,
« CSB » (1-bromo-4-(chlorométhylsulfonyl)benzène) de Kumiai, (n° CAS 54091-06-4), qui est connu en tant qu'agent protecteur pour le riz contre les dégâts de plusieurs herbicides ;
S15) les composés de formule (S15) ou leurs tautomères 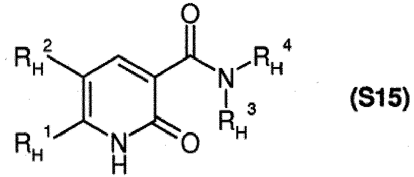 dans laquelle
R_{H}¹ signifie un radical haloalkyle en (C₁-C₆), et
R_{H}² signifie hydrogène ou halogène, et
R_{H}³, R_{H}⁴ signifient indépendamment l'un de l'autre hydrogène, alkyle en (C₁-C₁₆), alcényle en (C₂-C₁₆) ou alcynyle en (C₂-C₁₆),
chacun des 3 derniers radicaux cités étant non substitué ou substitué par un ou plusieurs radicaux choisis dans le groupe constitué par halogène, hydroxy, cyano, alcoxy en (C₁-C₄), haloalcoxy en (C₁-C₄), alkylthio en (C₁-C₄), alkylamino en (C₁-C₄), di[alkyle en (C₁-C₄)]amino, [alcoxy en (C₁-C₄)]-carbonyle, [haloalcoxy en (C₁-C₄)]-carbonyle, cycloalkyle en (C₃-C₆), qui est non substitué ou substitué, phényle, qui est non substitué ou substitué, et hétérocyclyle, qui est non substitué ou substitué,
ou cycloalkyle en (C₃-C₆), cycloalcényle en (C₄-C₆), cycloalkyle en (C₃-C₆) qui est condensé d'un côté du cycle avec un cycle carbocyclique saturé ou insaturé de 4 à 6 éléments, ou cycloalcényle en (C₄-C₆) qui est condensé d'un côté du cycle avec un cycle carbocyclique saturé ou insaturé de 4 à 6 éléments,
chacun des 4 derniers radicaux cités étant non substitué ou substitué par un ou plusieurs radicaux choisis dans le groupe constitué par halogène, hydroxy, cyano, alkyle en (C₁-C₄), haloalkyle en (C₁-C₄), alcoxy en (C₁-C₄), haloalcoxy en (C₁-C₄), alkylthio en (C₁-C₄), alkylamino en (C₁-C₄), di[alkyle en (C₁-C₄)]-amino, [alcoxy en (C₁-C₄)]-carbonyle, [haloalcoxy en (C₁-C₄)]-carbonyle, cycloalkyle en (C₃-C₆), qui est non substitué ou substitué, phényle, qui est non substitué ou substitué, et hétérocyclyle, qui est non substitué ou substitué, ou
R_{H}³ signifie alcoxy en (C₁-C₄), alcényloxy en (C₂-C₄), alcynyloxy en (C₂-C₆) ou haloalcoxy en (C₂-C₄), et
R_{H}⁴ signifie hydrogène ou alkyle en (C₁-C₄), ou
R_{H}³ et R_{H}⁴ forment ensemble avec l'atome N directement relié un cycle hétérocyclique de quatre à huit éléments, qui peut contenir en plus de l'atome N également d'autres hétéroatomes de cycle, de préférence jusqu'à deux hétéroatomes de cycle supplémentaires du groupe constitué par N, O et S, et qui est non substitué ou substitué par un ou plusieurs radicaux choisis dans le groupe constitué par halogène, cyano, nitro, alkyle en (C₁-C₄), haloalkyle en (C₁-C₄), alcoxy en (C₁-C₄), haloalcoxy en (C₁-C₄) et alkylthio en (C₁-C₄) ;
S16) les agents actifs qui sont utilisés en tant qu'herbicides, mais qui présentent toutefois également un effet protecteur sur les plantes cultivées, p. ex.
l'acide (2,4-dichlorophénoxy)acétique (2,4-D),
l'acide (4-chlorophénoxy)acétique,
l'acide (R,S)-2-(4-chloro-o-tolyloxy)propionique (Mecoprop),
l'acide 4-(2,4-dichlorophénoxy)butyrique (2,4-DB), l'acide (4-chloro-o-tolyloxy)acétique (MCPA),
l'acide 4-(4-chloro-o-tolyloxy)butyrique,
l'acide 4-(4-chlorophénoxy)butyrique,
l'acide 3,6-dichloro-2-méthoxybenzoïque (Dicamba),
le 3,6-dichloro-2-méthoxybenzoate de 1-(éthoxycarbonyl)éthyle (Lactidichlor-éthyl).

9. Procédé de lutte contre une végétation indésirable, **caractérisé en ce qu'**un agent selon la revendication 8 est laissé agir sur les plantes ou leur environnement.

10. Utilisation d'un agent selon la revendication 8 pour lutter contre une végétation indésirable.

11. Procédé de lutte contre une végétation indésirable, **caractérisé en ce qu'**un composé de formule (I-a) selon la revendication 1 et le composé améliorant la compatibilité avec les plantes cultivées selon la revendication 8 sont laissés agir séparément sur les plantes ou leur environnement.

12. Composition comprenant :
- au moins un composé de formule (I-a) selon la revendication 1 ou un agent selon la revendication 8 et
- au moins un sel de formule (III') 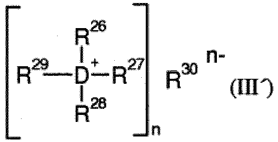 dans laquelle
D représente l'azote ou le phosphore,
R²⁶, R²⁷, R²⁸ et R²⁹ représentent indépendamment les uns des autres l'hydrogène ou alkyle en C₁-C₈ à chaque fois éventuellement substitué, ou alkylène en C₁-C₈ mono- ou polyinsaturé, éventuellement substitué, les substituants pouvant être choisis parmi halogène, nitro et cyano,
n représente 1, 2, 3 ou 4,
R³⁰ représente un anion inorganique ou organique.

13. Composition selon la revendication 12, **caractérisée en ce qu'**elle contient au moins un promoteur de pénétration.

14. Procédé d'augmentation de l'effet d'agents de lutte contre les animaux nuisibles et/ou d'herbicides et/ou de fongicides contenant un agent actif de formule (I-a) selon la revendication 1 ou un agent selon la revendication 8, **caractérisé en ce que** l'agent prêt à l'utilisation (bouillie de pulvérisation) est préparé en utilisant un sel de formule (III') selon la revendication 12.

15. Procédé selon la revendication 14, **caractérisé en ce que** la bouillie de pulvérisation est préparée en utilisant un promoteur de pénétration.

16. Composés de formule (II) 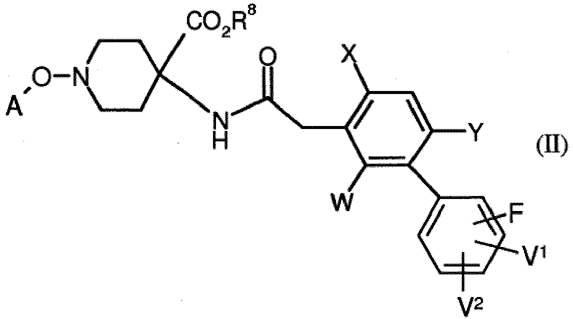 dans laquelle
A, W, X, Y, V¹ et V² ont les significations indiquées dans la revendication 1, et
R⁸ représente alkyle.

17. Composés de formule (XVI) 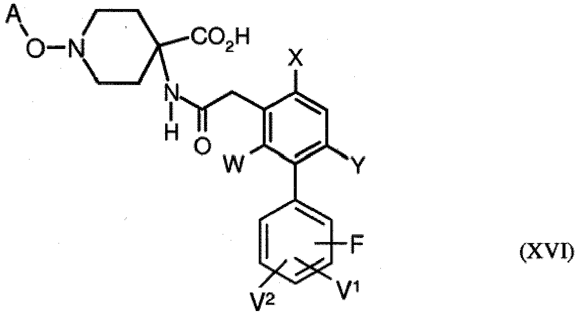 dans laquelle
A, W, X, Y, V¹ et V² ont les significations indiquées dans la revendication 1.

18. Composés de formule (XX) 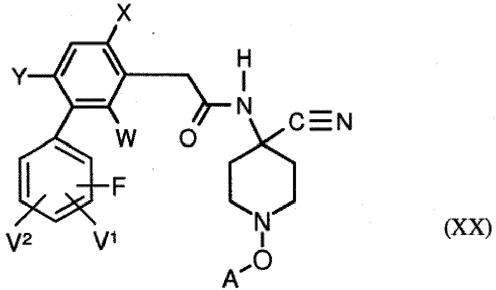 dans laquelle
A, W, X, Y, V¹ et V² ont les significations indiquées dans la revendication 1.

19. Composés de formules (II'-1) et (II'-2)
